# EUROPÄISCHE PATENTSCHRIFT

(11) **EP 2 099 788 B1**
(45) Veröffentlichungstag und Bekanntmachung des Hinweises auf die Patenterteilung: **06.03.2013**
(21) Anmeldenummer: 07846651.3
(22) Anmeldetag: 17.11.2007
(51) Int. Cl.: C07D 405/04, C07D 413/14, C07D 417/12, C07D 417/14, A61K 31/4439, A61P 9/00

(54) **CYCLISCH SUBSTITUIERTE 3,5-DICYANO-2-THIOPYRIDINE UND IHRE VERWENDUNG**
CYCLICALLY SUBSTITUTED 3,5-DICYANO-2-THIOPYRIDINES AND USE THEREOF
3,5-DICYANO-2-THIOPYRIDINES SUBSTITUÉES DE MANIÈRE CYCLIQUE ET LEUR UTILISATION

(30) Priorität: 01.12.2006 DE 102006056740
(43) Veröffentlichungstag der Anmeldung: 16.09.2009
(73) Patentinhaber: Bayer Intellectual Property GmbH, 40789 Monheim (DE)
(72) Erfinder: NELL, Peter, 42115 Wuppertal (DE); HÜBSCH, Walter, 42113 Wuppertal (DE); ALBRECHT-KÜPPER, Barbara, 42289 Wülfrath (DE); VAKALOPOULOS, Alexandros, 40721 Hilden (DE); SÜSSMEIER, Frank, 42277 Wuppertal (DE); KELDENICH, Jörg, 42113 Wuppertal (DE); TELSER, Joachim, 42115 Wuppertal (DE)
(74) Vertreter: BIP Patents
(86) Internationale Anmeldenummer: PCT/EP2007/009962
(87) Internationale Veröffentlichungsnummer: WO 2008/064788

(56) Entgegenhaltungen:
- EP-A- 1 302 463
- WO-A-01/62233
- WO-A-02/070485
- BEUKERS MARGOT W ET AL: "New, non-adenosine, high-potency agonists for the human adenosine A2B receptor with an improved selectivity profile compared to the reference agonist n-ethylcarboxamidoadenosine" JOURNAL OF MEDICINAL CHEMISTRY, AMERICAN CHEMICAL SOCIETY. WASHINGTON, US, Bd. 47, Nr. 15, 15. Juli 2004 (2004-07-15), Seiten 3707-3709, XP002390870 ISSN: 0022-2623
- DYACHENKO, V. D.: "Cyclohexanecarboxaldehyde in multicomponent syntheses of functionalized cyclohexyl-substituted acrylonitriles, 4H-chalcogenopyrans, 1,4-dihydropyridines, and pyridines" RUSSIAN JOURNAL OF GENERAL CHEMISTRY , 76(2), 282-291 CODEN: RJGCEK; ISSN: 1070-3632, Februar 2006 (2006-02), XP009096685

## Beschreibung

Die vorliegende Anmeldung betrifft neue 4-Cycloalkyl- und 4-Heterocycloalkyl-3,5-dicyano-2-thiopyidin-Derivate, Verfahren zu ihrer Herstellung, ihre Verwendung zur Behandlung und/oder Prophylaxe von Krankheiten sowie ihre Verwendung zur Herstellung von Arzneimittein zur Behandlung und/oder Prophylaxe von Krankheiten, vorzugsweise zur Behandlung und/oder Prävention von Hypertonie und anderen kardiovaskulären Erkrankungen.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf den Blutdruck, die Herzfrequenz, auf die Ausschüttung von Neurotransmittern und auf die Lymphozyten-Differenzierung. In Adipozyten ist Adenosin in der Lage, die Lipolyse zu hemmen und somit die Konzentration an freien Fettsäuren und Triglyzeriden im Blut zu senken.

Diese Wirkungen von Adenosin zielen darauf ab, das Sauerstoffangebot der betroffenen Organe zu erhöhen bzw. den Stoffwechsel dieser Organe zu drossein, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Als "Adenosinrezeptor-selektive Liganden" werden erfindungsgemäß solche Substanzen bezeichnet, die selektiv an einen oder mehrere Subtypen der Adenosin-rezeptoren binden und dabei entweder die Wirkung des Adenosin nachahmen (Adenosin-Agonisten) oder dessen Wirkung blockieren (Adenosin-Antagonisten) können.

Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die Al - oder A3-Rezeptoren über eine Hemmung der Adenylatzyklase eine Abnahme des intrazellulären cAMP-Gehalts bewirkt.

Im Herz-Kreislaufsystem sind die Hauptwirkungen der Aktivierung von Adenosin-Rezeptoren: Bradykardie, negative Inotropie und Protektion des Herzens vor Ischämie ("preconditioning") über Al-Rezeptoren, Dilation der Gefäße über A2a- und A2b-Rezeptoren sowie Inhibition der Fibroblasten und Glattmuskelzellproliferation über A2b-Rezeptoren.

Im Falle von A1-Agonisten (Kopplung bevorzugt über Gᵢ-Proteine) wird dabei eine Abnahme des intrazellulären cAMP-Gehaltes beobachtet (bevorzugt nach direkter Vorstimulation der Adenylatzyklase durch Forskolin). Entsprechend führen A2a- und A2b-Agonisten (Kopplung bevorzugt über Gₛ-Proteine) zu einer Zunahme und A2a- und A2b-Antagonisten zu einer Abnahme im cAMP-Gehalt der Zellen. Im Falle der A2-Rezeptoren ist eine direkte Vorstimulation der Adenylatzyklase durch Forskolin nicht hilfreich.

Die Aktivierung von A2b-Rezeptoren durch Adenosin oder spezifische A2b-Agonisten führt über die Erweiterung von Gefäßen zu einer Bluldrucksenkung. Die Blutdrucksenkung ist von einem reflektorischen Herzfrequenzanstieg begleitet. Der Herzfrequenzanstieg kann durch die Aktivierung von A1-Rezeptoren durch spezifische Al-Agonisten reduziert werden.

Die kombinierte Wirkung von selektiven Al/A2b-Agonisten auf das Gefäßsystem und die Herzfrequenz resultiert somit in einer systemischen Blutdrucksenkung ohne relevanten Herzfrequenzanstieg. Mit einem solchen pharmakolopischen Profil könnten duale Al/A2b-Agonisten zur Behandlung z.B. der Hypertonie beim Menschen eingesetzt werden.

In Adipozyten bewirkt die Aktivierung von Al- und A2b-Rezeptoren eine Inhibition der Lipolyse. Die kombinierte Wirkung von A1/A2b-Agonisten auf den Lipidstoffwechsel führt somit zu einer Senkung von freien Fettsäuren und Triglyzeriden. Eine Senkung der Lipide wiederum führt bei Patienten mit Metabolischem Syndrom und bei Diabetikern zur Verringerung der Insulinresistenz und zur Verbesserung der Symptomatik.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles, "Cloning, expression, and characterization of the unique bovine Al adenosine receptor. Studies on the ligand binding site by site-directed mutagenesis", J. Biol. Chem. 267 (1992), Seiten 10764-10770, deren Offenbarung hiermit im vollen Umfang durch Bezugnahme eingeschlossen ist).

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characlerization of stably transfected receptors in CHO cells", Naunyn Schmiedebergs Arch. Pharmacol. 357 (1998), Seiten 1-9, deren Offenbarung hiermit im vollen Umfang durch Bezugnahme eingeschlossen ist).

Bei den aus dem Stand der Technik bekannten, als "Adenosinrezeptor-spezifisch" geltenden Liganden handelt es sich überwiegend um Derivate auf Basis des natürlichen Adenosins [S.-A. Poulsen und R. J. Quinn, "Adenosine receptors: New opportunities for future drugs", Bioorganic and Medicinal Chemistry 6 (1998), Seiten 619-641]. Diese aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Deshalb werden sie überwiegend nur für experimentelle Zwecke verwendet. In klinischer Entwicklung befindliche Verbindungen dieser Art eignen sich bislang nur zur intravenösen Applikation.

In WO 01/25210 und WO 02/070485 werden substituierte 2-Thio-3,5-dicyano-4-aryl-6-aminopyridine als Adenosinrezeptor-Liganden für die Behandlung von Erkrankungen beschrieben. In WO 03/053441 werden spezifisch substituierte 2-Thio-3,5-dicyano-4-phenyl-6-aminopyridine als selektive Liganden des Adenosin A1-Rezeptors offenbart, und in WO 2006/027142 werden substituierte Phenylaminothiazol-Derivate als duale Adenosin A1/A2b-Agonisten für die Behandlung der Hypertonie und anderer kardiovaskulärer Erkrankungen beansprucht. Allerdings zeigte es sich, dass diese Verbindungen zum Teil hinsichtlich ihrer physikochemischen und/oder pharmakokinetischen Eigenschaften, wie beispielsweise ihrer Löslichkeit in Wasser und anderen physiologischen Medien oder ihres Absorptionsverhaltens im Körper, Nachteile aufweisen.

In WO 01/62233 werden verschiedene Pyridin- und Pyrimidin-Derivate sowie ihre Verwendung als Adenosinrezeptor-Modulatoren offenbart. Die Verwendung von 4-Cycloalkyl- und 4-Heterocycloalkyl-substituierten 3,5-Dicyanopyridinen als Calcium-abhängige Kaliumkanalöffner zur Behandlung urologischer Erkrankungen wird in EP 1 302 463-A1 beschrieben. In WO 2004/054505 wird die Verwendung von 2-Amino-3-cyanopyidin-Derivaten als MK-2-Inhibitoren zur Behandlung unterschiedlicher Erkrankungen beansprucht. Verschiedene heterocyclisch substituierte Pyridin-Derivate und ihre Verwendung zur Behandlung von Krankheiten werden in WO 99/32117, WO 2005/046603, WO 2005/007647 und WO 2006/034446 beschrieben. In WO 02/50071 werden Aminothiazol-Derivate als Tyrosin-Kinase-Inhibitoren für die Behandlung von Krebs sowie immunologischer und allergischer Erkrankungen offenbart.

In V.D. Dyachenko, "Cyclohexanecarbaldehyde in Multicomponent Synthesis of functionalized cyclohexyl-substituted acrylonitriles, 4H-Chalcogenopyrans, 1,4-Dihydrogyridines and Pyridines", Russian Journal of General Chemistry 2 (2006), Vol. 76Seiten 282-291 werden thio-substituierte Dicyanopyridines beschrieben. Allerdings warden keinerlei biologische Daten zu diesen Verbindungen offenbart..

Aufgabe der vorliegenden Erfindung ist die Bereitstellung neuer Verbindungen, die als selektive Agonisten des Adenosin A1-Rezeptors oder als selektive duale Agonisten des Adenosin Al-und A2b-Rezeptors wirken, als solche zur Behandlung und/oder Prävention insbesondere von Hypertonie und anderen kardiovaskulären Erkrankungen, des Metabolischen Syndrom, Diabetes und Dyslipidämien sowie zur Organprotektion bei Transplantationen und operativen Eingriffen geeignet sind und darüber hinaus ein verbessertes physikochemisches und/oder pharmakokinetisches Profil gegenüber den aus dem Stand der Technik bekannten Substanzen aufweisen.

Gegenstand der vorliegenden Erfindung sind Verbindungen der Formel (I)

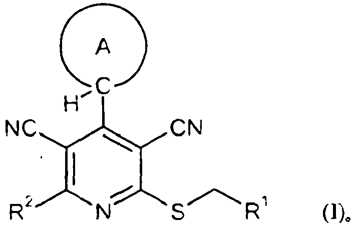

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (1) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enatiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologische unbedenkliche Salze der eifindungsgemäl3en Verbindungen bevorzugt. Umfasst sind auch Salze, die für pharmazeutische. Anwendungen selbst nicht geeignet sind, jedoch beispieisweise für die Isolierung oder Reinigung der erfindungssemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.
(C₅-C₇)-Cycloalkyl, (C₃-C₆)-Cycloalkyl und (C₃-C₅)-Cycloalkyl stehen im Rahmen der Erfindung für einen monocyclischen, gesättigten Carbocyclus mit 4 bis 7, 3 bis 6 bzw. 3 bis 5 Ring-Kohlenstoffatomen. Beispielhaft und vorzugsweise seien genannt: Cyclopropyl, Cyclobutyl, Cyclopentyl, Cyclohexyl und Cycloheptyl.
(C₄-C₇)-Cycloalkenyl steht im Rahmen der Erfindung für einen monocyclischen Carbocyclus mit 4 bis 7 Ring-Kohlenstoffatomen und einer Doppelbindung. Beispielhaft und vorzugsweise seien genannt: Cyclobut-2-en-1-yl, Cyclopent-2-en-1-yl, Cyclopent-3-en-1-yl, Cyclohex-2-en-1-yl, Cyclohex-3-en-1-yl und Cyclohept-3-en-1-yl.
(C₁-C₆)-Alkoxv und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.
(C₁-C₆)-Alkoxycarbonyl und (C₁-C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
(C₁-C₆)-Acyl und (C₁-C₄)-Acy) [(C₁-C₆)-Alkanoyl und (C₁-C₄)-Alkanoyl] stehen im Rahmen der Erfindung für einen geradkettigen oder verzweigten Alkylrest mit 1 bis 6 bzw. 1 bis 4 Kohlenstoffatomen, der in der 1-Position ein doppelt gebundenes Sauerstoffatom trägt und über die 1-Position verknüpft ist. Bevorzugt ist ein Acylrest mit 1 bis 4 Kohlenstoffatomen. Beispielhaft und vorzugsweise seien genannt: Formyl, Acetyl, Propionyl, n-Butyryl, iso-Butyryl, n-Pentanoyl, Pivaloyl und n-Hexanoyl.
(C₁-C₄)-Acyloxy steht im Rahmen der Erfindung für einen geradkettigen oder verzweigten AlkylRest mit 1 bis 4 Kohlenstoffatomen, der in der 1-Position ein doppelt gebundenes Sauerstoffatom trägt und in der 1-Position über ein weiteres Sauerstoffatom verknüpft ist. Beispielhaft und vorzugsweise seien genannt: Acetoxy, Propionoxy, n-Butyroxy und iso-Butyroxy.
Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkyl. amino-Rest mit 1 bis 4 Kohlenstoffatomen. Beispielhaft und vorzugsweise seien genannt: Methylamino, Ethylamino, n-Propylamino, Isopropylamino, n-Butylamino, tert.-Butylamino, n-Pentylamino und n-Hexylamino.
Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: N,N-Dimethylamino, N,N-Diethylamino, N-Ethyl-N-methylamino, N-Methyl-N-n-propylamino, N-Isopropyl-N-n-propylamino, N,N-Diiopropylamino, N-n-Butyl-N-methylamino, N-tert.-Butyl-N-methylamino, N-Ethyl-N-n-pentylamino und N-n-Hexyl-N-methylamino.
Mono- bzw. Di-(C₁-C₆)-alkylaminocarbonyl steht im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit jeweils 1 bis 6 Kohlenstoffatomen aufweist. Bevorzugt ist ein Mono- bzw. Dialkylaminocarbonyl-Rest mit 1 bis 4 Kohlenstoffatomen in der Alkylgruppe. Beispielhaft und vorzugsweise seien genannt: Methylaminocarbonyl, Ethylaminocarbonyl, n-Propylaminocarbonyl, Isopropylaminocarbonyl, n-Butylaminocarbonyl, tert.-Butylaminocarbonyl, N,N-Dimethylaminocarbonyl, N,N-Diethylaminocarbonyl, J, N-Ethyl-N-methylaminocarbonyl, N-Methyl-N-n-propylaminocarbonyl, N-n-Butyl-N-methylaminocarbonyl und N-tert.-Butyl-N-methylaminocarbonyl.
(C₆-C₁₀)-Aryl steht im Rahmen der Erfindung für einen aromatischen Carbocyclus mit 6 oder 10 Ring-Kohlenstoffatomen. Bevorzugte Arylreste sind Phenyl und Naphthyl.
Ein 4- bis 7-gliedriger Heterocyclus steht im Rahmen der Erfindung für einen gesättigten Heterocyclus mit insgesamt 4 bis 7 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Bevorzugt ist ein 5- oder 6-gliedriger Heterocyclus mit ein oder zwei Ring-Heteroatomen aus der Reihe N und/oder O. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl, Hexahydroazepinyl und Hexahydro-1,4-diazepinyl. Bevorzugt sind Pyrrolidinyl, Tetrahydrofuranyl, Piperidinyl, Piperazinyl, Tetrahydropyranyl und Morpholinyl.
5- bis 10-gliedriges Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind monocyclische 5- oder 6-gliedrige Heteroaryl-Reste mit bis zu drei Ring-Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl, Triazinyl.
Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei gleichen oder verschiedenen Substituenten.

Fegenstand der vorliegenden Erfindung sind Verbindungen der Formel (I), in welcher
der Ring A für Cyclopentyl, Cyclohexyl, Cyclopent-2-en-1-yl, Cyclopent-3-en-1-yl, Cyclohex-2-en-1-yl oder Cyclohex-3-en-1-yl, für einen 5- oder 6-gliedrigen, C-verknüpften, esältigten Heterocyclus, der ein Ringglied aus der Reihe N-R³ oder O enthält, oder für einen C-verknüpften, esältigten Heterocyclus der Formel 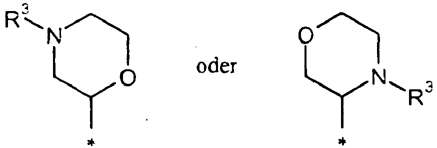 steht, worin
Cyclopentyl, Cyclohexyl, Cyclopentenyl sowie Cyclohexenyl ein- oder zweifach, gleich oder verschieden, mit einem Rest ausgewählt aus der Reihe (C₁-C₄)-Alkyl, Hydroxy, (C₁-C₄)-Alkoxy, Amino, Mono-(C₁-C₄)-alkylamino und Di-(C₁-C₄)-alkylamino substituiert sein können, wobei die genannten (C₁-C₄)-Alkyl- und (C₁-C₄)-Alkoxy-Reste ihrerseits ein- oder zweifach, gleich oder verschieden, mit Hydroxy, (C₁-C₄)-Alkoxy und/oder (C₃-C₅)-Cycloalkyl substituiert sein können,
- *: die Verknüpfungsstelle mit dem Pyridin-Ring bedeutet
und
- R³: Wasserstoff, (C₁-C₄)-Alkyl, welches ein- oder zweifach, gleich oder verschieden, mit Hydroxy, (C₁-C₄)-Alkoxy, (C₁-C₄)-Acyloxy und/oder (C₃-C₅)-Cycloalkyl substituiert sein kann, oder (C₁-C₄)-Acyl, welches mit Hydroxy oder (C₁-C₄)-Alkoxy substituiert sein kann, bedeutet,
- R¹: ür Phenyl oder 5- oder 6-gliedriges Heteroaryl mit bis zu drei Ring-Heteroatomen aus der Reihe N, O und/oder S steht, welche jeweils
(i) ein- oder zweifach, gleich oder verschieden, mit einem Rest ausgewählt aus der Reihe Fluor, Chlor, Cyano, (C₁-C₄)-Alkyl, Amino, Mono-(C₁-C₄)-alkylamino, Di-(C₁-C₄)-alkylamino, (C₁-C₄)-Alkoxycarbonyl, Carboxyl und Carbamoyl
   und/oder
(ii) mit Morpholino, N'-(C₁-C₄)-Alkylpiperazino oder einer Gruppe der Formel -L-R⁴, worin
   L eine Bindung oder NH bedeutet
   und
   R⁴ Phenyl oder 5- oder 6-gliedriges Heteroaryl mit bis zu drei Ring-Heteroatomen aus der Reihe N, O und/oder S bedeutet, welche jeweils ein- bis dreifach, gleich oder verschieden, mit einem Rest ausgewählt aus der Reihe Fluor, Chlor, Cyano, (C₁-C₄)-Alkyl, Trifluormethyl, (C₁-C₄)-Alkoxy, Trifluormethoxy, (C₁-C₄)-Alkoxycarbonyl und Carboxyl substituiert sein können,
substituiert sind,
oder
- R¹: für N-Oxidopyridyl steht,
und
- R²: für Wasserstoff oder für (C₁-C₄)-Alkoxy, das bis zu dreifach mit Fluor substituiert sein kann, steht
oder
- R²: für eine Gruppe der Formel -NR⁵R⁶ steht, worin
- R⁵: Wasserstoff oder (C₁-C₄)-Alkyl, das mit Hydroxy, (C₁-C₄)-Alkoxy, Amino, Mono-(C₁-C₄)-alkylamino, Di-(C₁-C₄)-alkylamino, Carboxyl, (C₁-C₄)-Alkoxycarbonyl oder einem 5- oder 6-gliedrigen, gesättigten Heterocyclus substituiert sein kann, bedeutet,
wobei der genannte Heterocyclus ein oder zwei Ring-Heteroatome aus der Reihe N und/oder O enthält und seinerseits ein- oder zweifach, gleich oder verschieden, mit Methyl, Ethyl, Hydroxy, Methoxy und/oder Ethoxy substituiert sein kann,
- R⁶: Wasserstoff oder Methyl bedeutet
oder
- R⁵ und R⁶: zusammen mit dem Stickstoffatom, an das sie gebunden sind, einen 5- oder 6-gliedrigen, gesättigten Heterocyclus bilden, der ein weiteres Ring-Heteroatom aus der Reihe N oder O enthalten und ein- oder zweifach, gleich oder verschieden, mit Methyl, Ethyl, Hydroxy, Methoxy und/oder Ethoxy substituiert sein kann,
sowie ihre Salze, Solvate und Solvate der Salze.

Besonders bevorzugt im Rahmen der vorliegenden Erfindung sind Verbindungen der Formel (I), in welcher
der Ring A für eine Gruppe der Formel 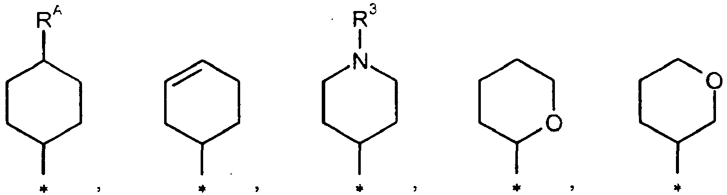 oder 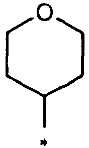 steht, worin
- *: die Verknüpfungsstelle mit dem Pyridin-Ring,
- R^{A}: Wasserstoff, Hydroxy, Methoxy, Ethoxy oder 2-Hydroxyethoxy und
- R³: Methyl, Ethyl, 2-Hydroxyethyl, 2-Acetoxyethyl, 3-Hydroxypropyl, 3-Acetoxypropyl oder Hydroxyacetyl
bedeuten,
- R¹: für Phenyl, Oxazolyl, Thiazolyl oder Pyridyl steht, welche jeweils
(i) ein- oder zweifach, gleich oder verschieden, mit einem Rest ausgewählt aus der Reihe Fluor, Chlor, Methyl, Amino, Methoxycarbonyl, Ethoxycarbonyl, Carboxyl und Carbamoyl
   oder
(*ii*) mit einer Gruppe der Formel -L-R⁴, worin
   L eine Bindung oder NH bedeutet
   und
   R⁴ Phenyl oder Pyridyl bedeutet, welche jeweils ein- oder zweifach, gleich oder verschieden, mit einem Rest ausgewählt aus der Reihe Fluor, Chlor, Cyano, Methyl, Methoxy und Carboxyl substituiert sein können, substituiert sind,
und
- R²: für Wasserstoff, Methoxy oder eine Gruppe der Formel -NR⁵R⁶ steht, worin
- R⁵: Wasserstoff oder (C₁-C₄)-Alkyl, das mit Hydroxy, Amino, Methylamine, Ethylamino, Dimethylamino, Diethylamino oder einem Helerocyclus der Formel 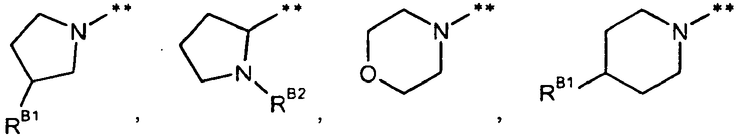 oder 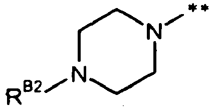 substituiert sein kann, bedeutet,
- R⁶: Wasserstoff bedeutet
oder
- R⁵ und R⁶: zusammen mit dem Stickstoffatom, an das sie gebunden sind, eine Gruppe der Formel 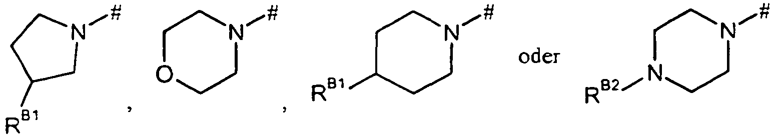 bilden, worin jeweils
** die Verknüpfungsstelle mit dem (C₁-C₄)-Alkylrest,
# die Verknüpfungsstelle mit dem Pyridin-Ring,
R^{B1} Wasserstoff oder Hydroxy
und
R^{B2} Wasserstoff oder Methyl bedeuten,
sowie ihre Salze, Solvate und Solvate der Salze.

Weiterer Gegenstand der vorliegenden Erfindung ist ein Verfahren zur Herstellung der erfindungsgemäßen Verbindungen der Formel (I), in welcher R² für NH₂ steht, dadurch gekennzeichnet, dass man eine Verbindung der Formel (II) 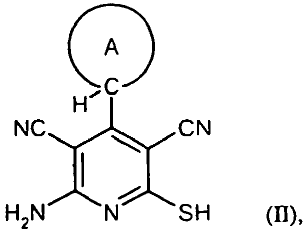 in welcher der Ring A die oben angegebene Bedeutung hat,

in einem inerten Lösungsmittel in Gegenwart einer Base mit einer Verbindung der Formel (III) 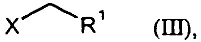 in welcher R¹ die oben angegebene Bedeutung hat und
- X: für eine geeignete Abgangsgruppe, vorzugsweise für Halogen, insbesondere Chlor, Brom oder Iod, oder für Mesylat, Tosylat oder Triflat steht,
zu einer Verbindung der Formel (I-A) 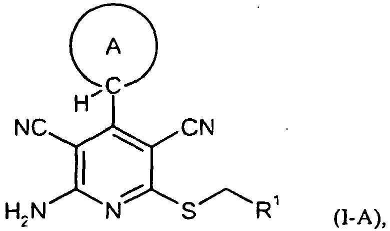 in welcher R¹ und der Ring A die oben angegebenen Bedeutungen haben,
umsetzt
und die Verbindungen der Formel (1-A) gegebenenfalls mit den entsprechenden (i) Lösungsmitteln und/oder (*ii*) Basen oder Säuren in ihre Solvate, Salze und/oder Solvate der Salze überführt.

Das zuvor beschriebene Verfahren kann durch das folgende Reaktionsschema erläutert werden:

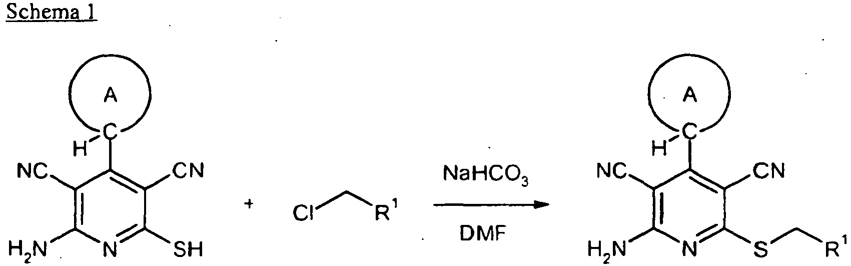

Als Lösungsmittel für das erfindungsgemäße Verfahren eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), N-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Bevorzugt wird Dimethylformamid als Lösungsmittel verwendet.

Als Base für die Umsetzung (II) + (III) → (I-A) eignen sich die üblichen anorganischen oder organischen Basen. Hierzu gehören bevorzugt Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt sind Alkalicarbonate und Alkalihydrogencarbonate.

Die Base kann hierbei in einer Menge von 1 bis 10 Mol, bevorzugt von 1 bis 5 Mol, insbesondere von 1 bis 4 Mol, bezogen auf 1 Mol der Verbindung der Formel (II), eingesetzt werden.

Die Reaktion erfolgt im Allgemeinen in einem Temperaturbereich von -78°C bis +140°C, bevorzugt im Bereich von -20°C bis +80°C, insbesondere bei 0°C bis +50°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (II) können in Analogie zu literaturbekannten Methoden beispielsweise dadurch hergestellt werden, dass man Aldehyde der Formel (IV) 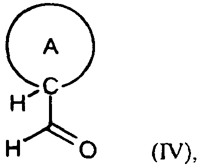 in welcher der Ring A die oben angegebene Bedeutung hat,

in Gegenwart einer Base mit zwei Äquivalenten Cyanothioacetamid umsetzt (siehe Schema 2; vgl. z.B. Dyachenko et al., Russ. J. Chem. 33 (7), 1014-1017 (1997), 34 (4), 557-563 (1998); Dyachenko et al., Chemistry of Hererocyclic Compounds 34 (2), 188-194 (1998); Qintela et al., Eur. J. Med. Chem. 33, 887-897 (1998); Kandeel et al., Z. Naturforsch. 42b, 107-111 (1987); Reddy et al., J. Med. Chem. 49, 607-615 (2006); Evdokimov et al., Org. Lett. 8, 899-902 (2006)]. 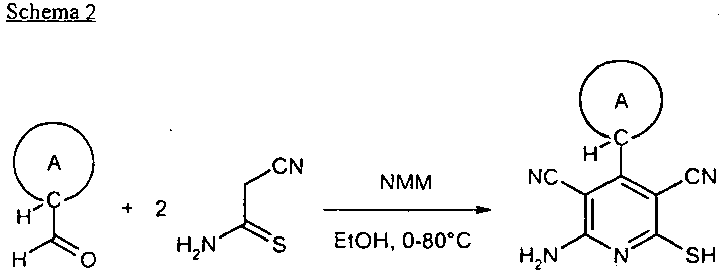 [EtOH = Ethanol, NMM = N-Methylmorpholin].

Alternativ können Verbindungen der Formel (II) auch ausgehend von Verbindungen der Formel (V) 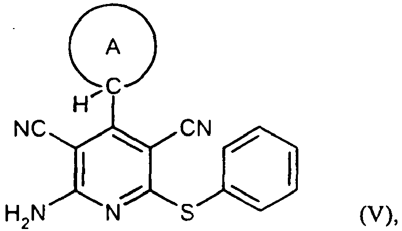 in welcher der Ring A die oben angegebene Bedeutung hat,
durch Reaktion mit einem Alkalisulfid hergestellt werden. Diese Herstellungsmethode kann durch folgendes Formelschema beispielhaft erläutert werden:

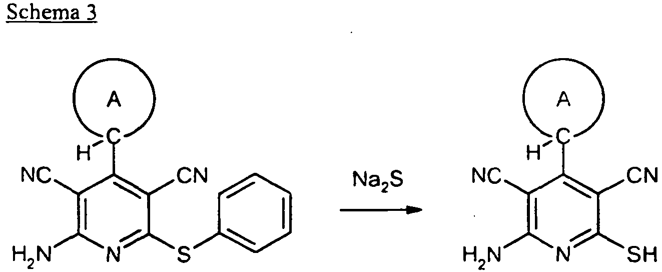

Als Alkalisulfid wird vorzugsweise Natriumsulfid in einer Menge von 1 bis 10 Mol, bevorzugt von 1 bis 5 Mol, insbesondere von 1 bis 4 Mol, bezogen auf 1 Mol der Verbindung der Formel (V), eingesetzt.

Als Lösungsmittel sind hierfür alle organischen Lösungsmittel geeignet, die unter den Reaktionsbedingungen inert sind. Hierzu gehören insbesondere Dimethylformamid, N-Methylpyrrolidinon, Pyridin und Acetonitril. Ebenso ist es möglich, Gemische der zuvor genannten Lösungsmittel einzusetzen. Bevorzugt wird Dimethylformamid verwendet.

Die Reaktion erfolgt im Allgemeinen in einem Temperaturbereich von +20°C bis +140°C, bevorzugt im Bereich von +20°C bis +120°C, insbesondere bei +60°C bis +100°C. Die Umsetzung kann bei normalem, erhöhtem oder erniedrigtem Druck durchgerührt werden (z.B. im Bereich von 0.5 bis 5 bar). Im Allgemeinen arbeitet man bei Normaldruck.

Die Verbindungen der Formel (V) können in Analogie zu literaturbeschriebenen Verfahren hergestellt werden [vgl. z.B. Kambe et al., Synthesis, 531-533 (1981); Elnagdi et al., Z. Naturforsch. 47b, 572-578 (1991); Reddy et al., J. Med. Chem. 49, 607-615 (2006); Evdokimov et al., Org. Lett. 8, 899-902 (2006)].

Die Verbindungen der Formel (III) sind kommerziell erhältlich, literaturbekannt oder nach literaturbekannten Methoden herstellbar. So können beispielsweise durch Reaktion von Amiden, Thioamiden bzw. Thioharnstoff-Derivaten mit einem 1,3-Dihalogenaceton substituierte Oxazol- und Thiazol-Derivate der Formel (ID-A), (III-B) bzw. (III-C) erhalten werden (siehe Schema 4):

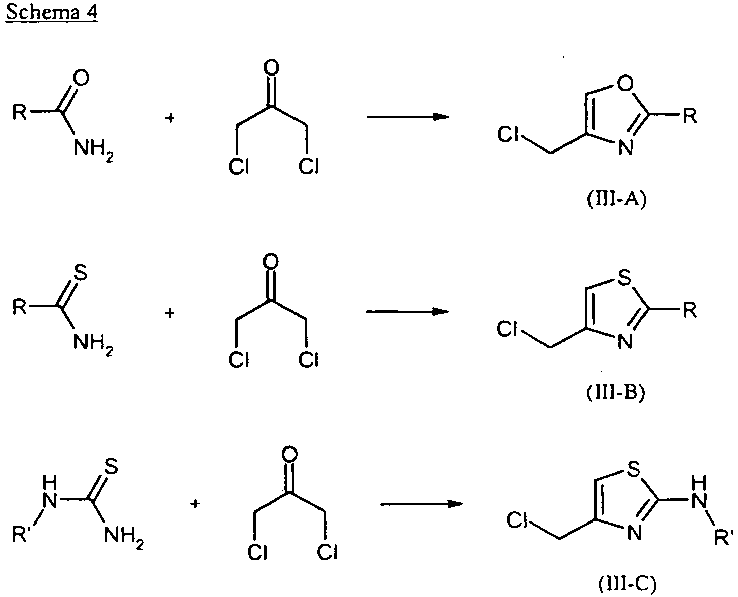

Im Falle der Verbindungen (III-C) können diese entweder analog zur Literatur hergestellt und isoliert werden [vgl. z.B. I. Simiti et al., Chem. Ber. 95, 2672-2679 (1962)], oder sie können *in situ* erzeugt und direkt weiter mit einer Verbindung der Formel (II) umgesetzt werden. Bevorzugt ist die *in situ*-Erzeugung unter Verwendung von 1,3-Dichloraceton in Dimethylformamid oder Ethanol als Lösungsmittel. Die Darstellung erfolgt im Allgemeinen in einem Temperaturbereich von 0°C bis +140°C, bevorzugt im Bereich von +20°C bis +120°C, insbesondere bei +60°C bis +100°C.

Die Aldehyde der Formel (IV) sind kommerziell erhältlich, in der Literatur beschrieben oder nach Standardmethoden aus bekannten Ausgangsverbindungen herstellbar.

Weiterer Gegenstand der vorliegenden Erfindung ist ein Verfahren zur Herstellung der erfindungsgemäßen Verbindungen der Formel (I), in welcher R² für die Gruppe -NR⁵R⁶ steht, worin mindestens einer der beiden Reste R⁵ und R⁶ nicht Wasserstoff bedeutet, dadurch gekennzeichnet, dass man Verbindungen der Formel (I-A) zunächst mit Kupfer(II)chlorid und Isoamylnitrit in einem geeigneten Lösungsmittel in Verbindungen der Formel (VI) 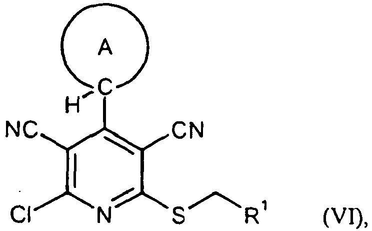 in welcher R¹ und der Ring A die zuvor angegebenen Bedeutungen haben,
überführt und diese anschließend mit einer Verbindung der Formel (VII) 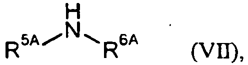 in welcher
- R^{5A}: die oben angegebene Bedeutung von R⁵ hat,
- R^{6A}: die oben angegebene Bedeutung von R⁶ hat,
jedoch mindestens einer der beiden Reste R^{5A} und R^{6A} nicht für Wasserstoff steht,
zu Verbindungen der Formel (I-B) 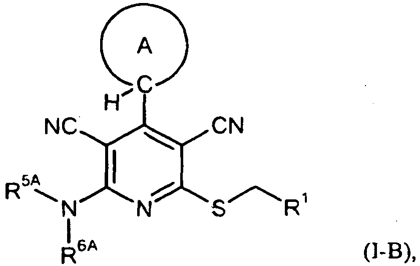 in welcher R¹, R^{5A}, R^{6A} und der Ring A jeweils die zuvor angegebenen Bedeutungen haben,
umsetzt
und die Verbindungen der Formel (I-B) gegebenenfalls mit den entsprechenden *(i)* Lösungsmitteln und/oder *(ii)* Basen oder Säuren in ihre Solvate, Salze und/oder Solvate der Salze überführt.

Das zuvor beschriebene Verfahren kann durch das folgende Reaktionsschema erläutert werden:

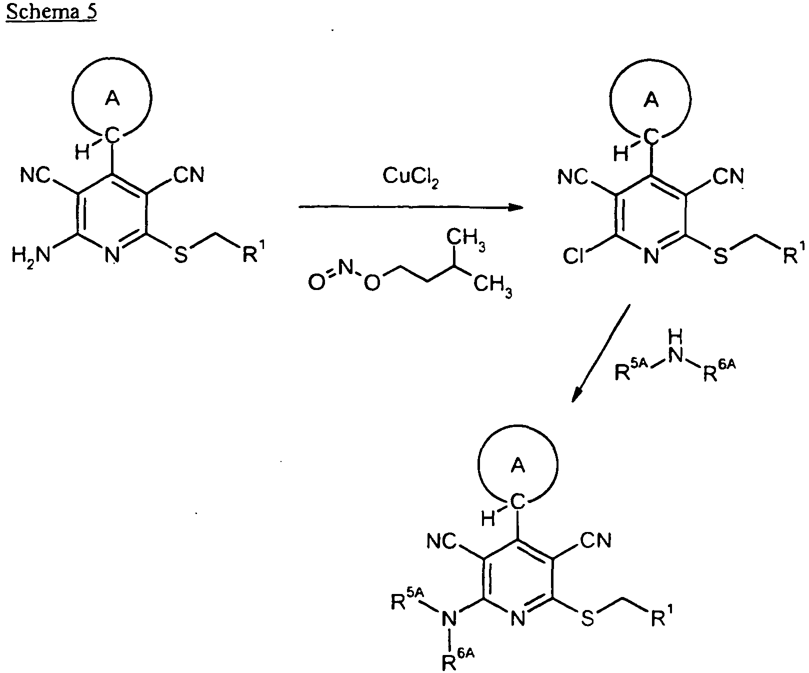

Die Umsetzung (I-A) → (VI) erfolgt im Allgemeinen in einem Molverhältnis von 2 bis 12 Mol Kupfer(II)chlorid und 2 bis 12 Mol Isoamylnitrit bezogen auf 1 Mol der Verbindung der Formel (I-A).

Als Lösungsmittel für den Verfahrensschritt (I-A) → (VI) eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril oder Pyridin. Ebenso ist es möglich, Gemische der zuvor genannten Lösungsmittel einzusetzen. Bevorzugte Lösungsmittel sind Acetonitril und Dimethylformamid.

Die Reaktion erfolgt im Allgemeinen in einem Temperaturbereich von -78°C bis +180°C= bevorzugt im Bereich von 0°C bis +100°C, insbesondere bei +20°C bis +80°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Umsetzung (VI) + (VII) → (I-B) erfolgt im Allgemeinen in einem Molverhältnis von 1 bis 8 Mol der Verbindung der Formel (von bezogen auf 1 Mol der Verbindung der Formel (VI).

Als Lösungsmittel für den Verfahrensschritt (VI) + (VII) → (I-B) eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril, Pyridin oder Dimethylsulfoxid. Wasser ist als Lösungsmittel ebenfalls geeignet. Ebenso ist es möglich, Gemische der zuvor genannten Lösungsmittel einzusetzen. Bevorzugtes Lösungsmittel ist Dimethylformamid.

Die Reaktion erfolgt im Allgemeinen in einem Temperaturbereich von 0°C bis +180°C, bevorzugt im Bereich von +20°C bis +150°C, insbesondere bei +20°C bis +100°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (VII) sind entweder kommerziell erhältlich, dem Fachmann bekannt oder nach üblichen Methoden herstellbar.

Die erfindungsgemäßen Verbindungen der Formel (I), in welcher R² für Wasserstoff steht, können hergestellt werden, indem man Verbindungen der Formel (I-A) in einem geeigneten Lösungsmittel mit Isoamylnitrit in Gegenwart einer *katalytischen* Menge Kupfer(II)chlorid umsetzt. Diese Methode kann durch das folgende Reaktionsschema erläutert werden:

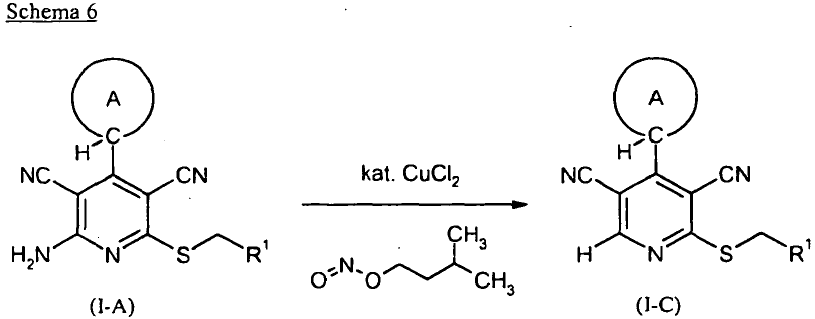

Die Umsetzung (I-A) → (I-C) erfolgt im Allgemeinen in einem Molverhältnis von 0.01 bis 0.2 Mol Kupfer(II)chlorid und 2 bis 12 Mol Isoamylnitrit bezogen auf 1 Mol der Verbindung der Formel (1-A).

Als Lösungsmittel für die Reaktion (I-A) → (I-C) eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril oder Pyridin. Ebenso ist es möglich, Gemische der genannten Lösungsmittel einzusetzen. Bevorzugte Lösungsmittel sind Tetrahydrofuran und Dimethylformamid.

Die Reaktion erfolgt im Allgemeinen in einem Temperaturbereich von -78°C bis +150°C, bevorzugt im Bereich von 0°C bis +80°C, insbesondere bei +10°C bis +40°C. Die Umsetzung kann bei normalem, erhöhtem oder emiedrigtem Druck durchgeführt werden (z.B. im Bereich von 0.5 bis 5 bar). Im Allgemeinen arbeitet man bei Normaldruck.

Die erfindungsgemäßen Verbindungen der Formel (I), in welcher R² für gegebenenfalls substituiertes (C₁-C₆)-Alkyl oder (C₁-C₆)-Alkoxy steht, sind in Analogie zu literaturbeschriebenen Methoden ausgehend von Verbindungen der Formel (VI) herstellbar [vgl. z.B. D. Mabire et al., *J*. *Med. Chem.* 48, 2134-2153 (2005)]. Alternativ können die Verbindungen der Formel (I), in welcher R² für gegebenenfalls substituiertes (C₁-C₆)-Alkoxy steht, auch durch Alkylierung von Verbindungen der Formel (VIII) erhalten werden (siehe Schema 7):

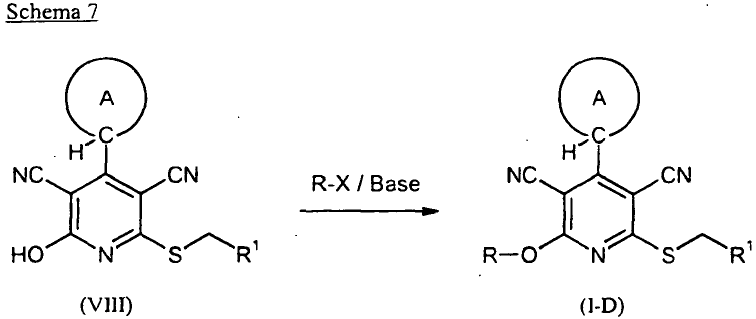

Die Verbindungen der Formel (VIII) ihrerseits sind nach literaturbekannten Methoden aus Verbindungen der Formel (VI) oder (I-A) zugänglich [vgl. z.B. G. Lavecchia et al., Tetrahedron Lett. 45, 6633-6636 (2004)].

Weitere erfindungsgemäße Verbindungen können gegebenenfalls auch hergestellt werden durch Umwandlungen von funktionellen Gruppen einzelner Reste und Substituenten, insbesondere den unter R¹, R² und zum Ring A aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile oder elektrophile Substitution, Oxidation, Reduktion, Hydrierung, Alkylierung, Acylierung, Aminierung, Bildung von Carbonsäureamiden und -estern, Esterhydrolyse, Veretherung, Etherspaltung sowie die Einführung und Entfernung temporärer Schutzgruppen.

Überraschenderweise zeigen die erfindungsgemäßen Verbindungen ein nicht vorhersehbares, wertvolles pharmakologisches Wirkspektrum und sind daher insbesondere zur Prophylaxe und/oder Behandlung von Erkrankungen geeignet. Darüber hinaus weisen die erfindungsgemäßen Substanzen gegenüber den Verbindungen aus dem Stand der Technik ein verbessertes Absorptionsverhalten im Körper und/oder eine verbesserte Löslichkeit in Wasser und anderen physiologischen Medien auf, was beispielsweise für ihre galenische Formullerbarkeit und/oder die parenterale Anwendung von Vorteil ist.

Die pharmazeutische Wirksamkeit der erfindungsgemäßen Verbindungen lässt sich durch ihre Wirkung als potente, selektive Liganden an Adenosin A1- und/oder A2b-Rezeptoren erklären. Sie wirken hierbei als selektive A1- oder als selektive duale A1/A2b-Agonisten.

Als "selektive Liganden an Adenosin A1- und/oder A2b-Rezeptoren" werden im Rahmen der vorliegenden Erfindung solche Adenosin-Rezeptorliganden bezeichnet, bei denen einerseits eine deutliche Wirkung an A1- und/oder A2b-Adenosinrezeptor-Subtypen und andererseits keine oder eine deutliche schwächere Wirkung (Faktor 10 oder höher) an A2a- und A3-Adenosinrezeptor-Subtypen zu beobachten ist, wobei bezüglich der Testmethoden für die Wirk-Selektivität Bezug genommen wird auf die im Abschnitt B-1. beschriebenen Tests.

Die Verbindungen der Formel (I) sind allein oder in Kombination mit einem oder mehreren anderen Wirkstoffen zur Prophylaxe und/oder Behandlung verschiedener Erkrankungen geeignet, so beispielsweise insbesondere bei Hypertonie und anderen Erkrankungen des Herzkreislauf-Systems (kardiovaskulären Erkrankungen) sowie zur Kardioprotektion.

Im Sinne der vorliegenden Erfindung sind unter Erkrankungen des Herzkreislauf-Systems bzw. kardiovaskulären Erkrankungen neben der Hypertonie beispielsweise insbesondere die folgenden Erkrankungen zu verstehen: periphere und kardiale Gefäßerkrankungen, koronare Herzerkrankung, koronare Restenose wie z.B. Restenose nach Ballondilatation von peripheren Blutgefäßen, akutes Koronarsyndrom, stabile und instabile Angina pectoris, Herzinsuffizienz, Tachykardien, Arrhythmien, Vorhof- und Kammerflimmern sowie periphere Durchblutungsstörungen.

Weiterhin eignen sich die erfindungsgemäßen Verbindungen insbesondere auch zur Reduktion des von einem Infarkt betroffenen Myokardbereichs sowie zur Prophylaxe von Sekundärinfarkten.

Des weiteren sind die erfindungsgemäßen Verbindungen insbesondere zur Prophylaxe und/oder Behandlung von thromboembolischen Erkrankungen und Ischämien wie Myokardinfarkt, Himschlag und transitorischen ischämischen Attacken sowie zur Protektion von Organen bei Transplantationen und operativen Eingriffen, beispielsweise am Herzen, geeignet.

Weitere Indikationsgebiete, für die die erfindungsgemäßen Verbindungen verwendet werden können, sind beispielsweise insbesondere die Prophylaxe und/oder Behandlung von Erkrankungen des Urogenitalbereiches, wie z.B. Reizblase, erektile Dysfunktion und weibliche sexuelle Dysfunktion, daneben aber auch die Prophylaxe und/oder Behandlung von inflammatorischen Erkrankungen, wie z.B. Asthma und entzündliche Dermatosen, von neuroinflammatorischen Erkrankungen des Zentralnervensystems, wie beispielsweise Zustände nach Hirninfarkt, der Alzheimer-Erkrankung, weiterhin auch von neurodegenerativen Erkrankungen sowie von Schmerzzuständen, Krebs und Übelkeit und Erbrechen in Verbindung mit Krebstherapien.

Ein weiteres Indikationsgebiet sind beispielsweise insbesondere die Prophylaxe und/oder Behandlung von Erkrankungen der Atemwege wie beispielsweise Asthma, chronische Bronchitis, Lungenemphysem, Bronchiektasien, zystische Fibrose (Mukoviszidose) und pulmonale Hypertonie.

Schließlich kommen die erfindungsgemäßen Verbindungen beispielsweise insbesondere auch für die Prophylaxe und/oder Behandlung von Diabetes, insbesondere Diabetes mellitus, diabetischen Folgeerkrankungen wie z.B. Nephropathie und Neuropathie, des Metabolischen Syndroms sowie von Dyslipidämien in Betracht.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen.

Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/ oder antithrombotisch wirkende Mittel, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten) sowie Analgetika wie beispielsweise Aspirin.

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylharnstoffe, Biguanide, Meglitinid-Derivate, Glukosidase-Inhibitoren, Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/ oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin AII-Antagonisten, ACE-Hemmer, Renin-Inhibitoren, beta-Rezeptoren-Blocker, alpha-Rezeptoren-Blocker, Diuretika, Phosphodiesterase-Inhibitoren, sGC-Stimulatoren, Verstärker der cGMP-Spiegel, Aldosteron-Antagonisten, Mineralocorticoid-Rezeptor-Antagonisten, ECE-Inhibitoren sowie der Vasopeptidase-Inhibitoren; und/oder
- antithrombotisch wirkenden Mitteln, beispielhaft und vorzugsweise aus der Gruppe der Thrombozytenaggregationshemmer oder der Antikoagulantien
kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantien/Radikalfänger sowie der Cannabinoid-Rezeptor 1-Antagonisten verstanden.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Torcetrapib, JTT-705, BAY 60-5521, BAY 78-7499 oder CETP vaccine (Avant), verabreicht.

Bei einer bevorzugten Ausführungsform der Erfindung werden die erfindungsgemäßen Verbindungen in Kombination mit einem PPAR-γ-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylhamstoffe, Biguanide, Meglitinid-Derivate, Glukosidase-Inhibitoren und PPAR-γ-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenc1amid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem PPAR-γ-Agonisten beispielsweise aus der Klasse der Thiazolidindione, wie beispielhaft und vorzugsweise Pioglitazone oder Rosiglitazone, verabreicht.

Unter den Blutdruck senkenden Mitteln werden vorzugsweise Verbindungen aus der Gruppe der Calcium-Antagonisten, Angiotensin AII-Antagonisten, ACE-Hemmer, beta-Rezeptoren-Blocker, alpha-Rezeptoren-Blocker und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losanan, Valsartan, Candesarian, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, mit Kaliumkanal-Agonisten wie Minoxidil, Diazoxid, Dihydralazin oder Hydralazin, oder mit Stickoxid freisetzenden Stoffen wie Glycerinnitrat oder Nitroprussidnatrium verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

| | |
|---|---|
| Bsp. | Beispiel |
| c | Konzentration |
| DC | Dünnschichtchromatographie |
| DCI | direkte chemische Ionisation (bei MS) |
| DMF | N,N-Dimethylformamid |
| DMSO | Dimethylsulfoxid |
| d. Th. | der Theorie (bei Ausbeute) |
| ee | Enantiomerenüberschuss |
| EI | Elekironenstoß-Ionisation (bei MS) |
| ent | Enantiomer / enantiomerenrein |
| ESI | Elektrospray-Ionisation (bei MS) |
| Et | Ethyl |
| Fp. | Schmelzpunkt |
| GC-MS | Gaschromatographie-gekoppelte Massenspektrometrie |
| ges. | gesättigt |
| h | Stunde(n) |
| HPLC | Hochdruck-, Hochleistungsflüssigchromatographie |
| kat. | katalytisch |
| konz. | konzentriert |
| LC-MS | Flüssigchromatographie-gekoppelte Massenspektrometrie |
| Lit. | Literatur(stelle) |
| min | Minute(n) |
| M S | Massenspektrometrie |
| NMR | Kernresonanzspektrometrie |
| rac. | racemisch |
| RP-HPLC | reverse phase HPLC |
| RT | Raumtemperatur |
| R, | Retentionszeit (bei HPLC) |
| TBME | tert.-Butylmethylether |
| TFA | Trifluoressigsäure |
| THF | Tetrahydrofuran |
| verd. | verdünnt |
| wässr. | wässrig |

### HPLC-, LC-MS- und GC-MS-Methoden:

### Methode 1 (HPLC):

Instrument: Hewlett Packard Series 1050; Säule: Symmetry TM C18 3.9 x 150 mm; Fluss: 1.5 ml/min; Eluent A: Wasser, Eluent B: Acetonitril; Gradient: → 0.6 min 10% B → 3.8 min 100% B → 5.0 min 100% B → 5.5 min 10% B; Stopzeit: 6.0 min; Injektionsvolumen: 10 µl; Diodenarraydetektor-Signal: 214 und 254 nm.

### Methode 2 (LC-MS):

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / 1, Eluent B: Acetonitril + 500 µl 50%-ige Ameisensäure / 1; Gradient: 0.0 min 10% B → 7.0 min 95% B → 9.0 min 95% B; Ofen: 35°C; Fluss: 0.0 min 1.0 ml/min → 7.0 min 2.0 ml/min → 9.0 min 2.0 ml/min; UV-Detektion: 210 nm.

### Methode 3 (LC-MS):

Instrument: Micromass Quattro LCZ mit HPLC Agilent Serie 1100; Säule: Phenomenex Gemini 3µ 30 mm x 3.00 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 4 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (LC-MS):

Instrument: Micromass Quattro LCZ mit HPLC Agilent Serie 1100; Säule: Phenomenex Onyx Monolithic C18, 100 mm x 3 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 6 (präparative HPLC):

Gerätetyp HPLC: Abimed/Gilson Pump 305/306; Manometric Module 806; UV Knauer Variable Wavelength Monitor; Säule: Gromsil C18, 10 nm, 250 mm x 30 mm; Eluent A: 1 1 Wasser + 0.5 ml 99% TFA, Eluent B: 1 1 Acetonitril; Gradient: 0.0 min 2% B - 10 min 2% B → 50 min 90% B; Fluss: 20 ml/min; Volumen: 628 ml A und 372 ml B.

### Methode 7 (LC-MS):

Gerätetyp MS: Waters ZQ; Gerätetyp HPLC: Waters Alliance 2795; Säule: Merck Chromolith RP18e, 100 mm x 3 mm; Eluent A: 1 1 Wasser + 0.5 ml 50%-ige Ameisensäure, Eluent B: 11 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 8 (GC-MS):

Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 9 (GC-MS):

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 10 (chirale HPLC):

Gerätetyp HPLC: HP 1100 mit DAD-Detektion; Säule: Daicel Chiralpak IA, 5 µm, 250 mm x 4.6 mm; Eluent: 50% iso-Hexan, 5% Methanol, 45% *tert*.-Butylmethylether; Fluss: 15 ml/min.

### Methode 11 (chirale HPLC):

Gerätetyp HPLC: HP 1100 mit DAD-Detektion; Säule: Daicel Chiralpak AD-H, 5 µm, 250 mm x 20 mm; Eluent: 50% iso-Hexan, 50% 2-Propanol; Fluss: 15 ml/min.

### Methode 12 (LC-MS]:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 13 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 mi/min → 2.5 min/3.0 min/4.5 min 2 ml/min; Ofen: 50°C; UV-Detektion: 208-400 nm.

### Methode 14 (LC-MS):

Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Fluss: 0.8 ml/min; Ofen: 50°C; UV-Detektion: 210 nm.

### Methode 15 (LC-MS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 16 (chirale HPLC):

Gerätetyp HPLC: HP 1100 mit DAD-Detektion; Säule: Daicel Chiralpak 1A-H, 5 µm, 250 mm x 20 mm; Eluent: 80% TBME, 20% Methanol; Fluss: 15 ml/min.

### Methode 17 (chirale HPLC):

Gerätetyp HPLC: HP 1100 mit DAD-Detektion; Säule: Daicel Chiralpak IA, 5 µm, 250 mm x 4.6 mm; Eluent: 80% TBME, 20% Methanol; Fluss: 1.0 ml/min.

### Methode 18 (chirale HPLC):

Gerätetyp HPLC): HP 1100 mit DAD-Detektion; Säule: Daicel Chiralpak 1A-H, 5 µm, 250 mm x 20 mm; Eluent: 50% iso-Hexan, 45% TBME, 5% Methanol; Fluss: 15 ml/min.

### Methode 19 (chirale HPLC):

Gerätetyp HPLC: HP 1100 mit DAD-Detektion; Säule: Daicel Chiralpak IA, 5 µm, 250 mm x 4.6 mm; Eluent: 40% iso-Hexan, 54% TBME, 6% Methanol; Fluss: 1.0 ml/min.

### Methode 20 (HPLC):

Gerätetyp HPLC: HP 1100 mit DAD-Detektion; Säule: Kromasil 100 C 18, 5 µm, 250 mm x 20 mm; Eluent: 25% 0.2%-ige Essigsäure, 75% Acetonitril; Fluss: 25 ml/min.

### Methode 21 (LC-MS):

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B:1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 22 (LC-MS):

Instrument: Micromass Quattro LCZ mit HPLC Agilent Serie 1100; Säule: Phenomenex Synergi 2.5µ MAX-RP 10M Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208-400 nm.

### Ausgangsverbindungen und Intermediate:

### Beispiel 1A

### 4-(2-Hydroxyethoxy)cyclohexancarbonsäure

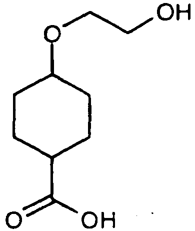

1.00 g (5.49 mmol) 4-(2-Hydroxyethoxy)benzoesäure werden in 30 ml trockenem THF und 30 ml trockenem Ethanol gelöst und mit 1.13 mg (0.55 mmol) Rhodium auf Aluminiumoxid versetzt. Das Reaktionsgemisch wird 12 h lang bei +50°C mit 50 bar Wasserstoffdruck hydriert. Anschließend wird für weitere 48 h bei +60°C mit 80 bar Wasserstoffdruck hydriert. Nach Filtration wird das Filtrat am Rotationsverdampfer eingeengt. Der Rückstand wird ohne weitere Reinigung direkt in der Folgereaktion eingesetzt.
Ausbeute: 1.00 g (84% d. Th., 87% Reinheit)
¹H-NMR (400 MHz, DMSO-d₆): δ = 12.3 (br. s, 1H), 4.52 (br. s, 1H), 3.49-3.34 (m, 4H), 3.25-3.14 (m, 1H), 2.32-2.22 (m, 1 H), 2.20-0.90 (m, 8H).
GC-MS (Methode 8): R₁ = 1.43 min; MS (ESIpos): m/z = 222 [M+H]⁺

### Beispiel 2A

### 4-(2-Hydroxyethoxy)cyclohexancarbonsäuremethylester

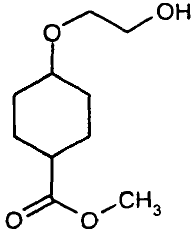

Das Rohprodukt aus Beispiel 1A wird in 40 ml Methanol gelöst und mit 200 mg Dowex 50 WX8-100-Ionenaustauscher (zuvor 7 x mit je 20 ml 2 M Salzsäure und anschließend 7 x mit je 20 ml Methanol gewaschen) versetzt. Es wird 20 h bei +64°C gerührt. Nach Abkühlen auf RT wird der Ionenaustauscher abfiltriert und das Filtrat am Rotationsverdampfer eingeengt. Das Rohprodukt wird direkt ohne weitere Reinigung in der Folgereaktion eingesetzt.
Ausbeute: 1.00 g (79% d. Th., 76% Reinheit)
GC-MS (Methode 8): R, = 5.26 min; MS (ESIpos): m/z = 183 [M+H]⁺.

### Beispiel 3A

### 4-(2- {[tert.-Butyl(diphenyl)silyl]oxy} ethoxy)cyclohexancarbonsäuremethylester

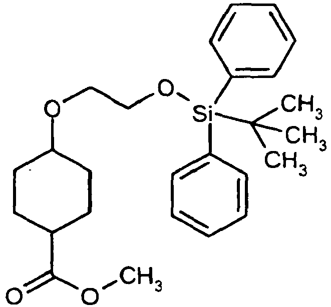

Das Rohprodukt aus Beispiel 2A wird in 20 ml Dichlormethan gelöst und mit 0.83 ml (5.93 mmol) Triethylamin sowie 1.54 ml (5.93 mmol) tert.-Butyldiphenylchlorsilan versetzt. Nach Zugabe von 24.2 mg (0.20 mmol) 4-N,N-Dimethylaminopyridin wird 20 h bei RT gerührt. Der Ansatz wird danach mit je 10 ml Ethylacetat und ges. wässriger Natriumhydrogencarbonat-Lösung versetzt. Nach Trennen der Phasen werden die vereinigten organischen Phasen über Magnesiumsulfat getrocknet. Das Lösungsmittel wird am Rotationsverdampfer entfernt und der Rückstand an Kieselgel 60 chromatographisch gereinigt (Laufmittel: Gradient Cyclohexan/Ethylacetat 100:1 → 20:1).
Ausbeute: 1.90 g (87% d. Th., *cis*/*trans*-Gemisch, 87% Reinheit)
GC-MS (Methode 8): R, = 10.36 min und 10.44 min; MS (ESIpos): m/z = 458 [M+NH₄]⁺.

### Beispiel 4A

### [4-(2-{[tert.-Butyl(diphenyl)silyl]oxy}ethoxy)cyclohexyl]methanol

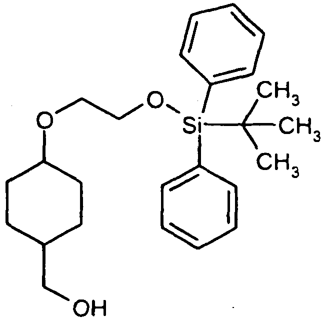

1.90 g (3.75 mmol, 87% Reinheit) der Verbindung aus Beispiel 3A werden in 15 ml Diethylether gelöst und zu einer Suspension von 170.9 mg (4.50 mmol) Lithiumaluminiumhydrid in 15 ml Diethylether getropft. Anschließend wird 20 h bei RT gerührt. Es werden weitere 136.7 mg (3.60 mmol) Lithiumaluminiumhydrid zugegeben und die Mischung erneut für 20 h bei RT gerührt. Der Ansatz wird dann mit 257 µl Wasser und 257 µl 15%-iger Kalilauge verrührt. Es werden weitere 10 ml Wasser zugegeben und die Phasen getrennt. Die organische Phase wird über Magnesiumsulfat getrocknet. Das Lösungsmittel wird am Rotationsverdampfer entfernt und der Rückstand an Kieselgel 60 chromatographisch gereinigt (Laufmittel: Gradient Cyclohexan/Ethylacetat 50:1 → 10:1).
Ausbeute: 1.20 g (72% d. Th., 93% Reinheit, *cis*/*trans*-Gemisch)
¹H-NMR (400 MHz, DMSO-d₆): δ = 7.71-7.63 (m, 4H), 7.49-7.39 (m, 6H), 4.43-4.30 (m, 1H), 3.79-3.70 (m, 2H), 3.54-3.46 (m, 2H), 3.21-3.14 (m, 2H), 2.00-1.56 (m, 3H), 1.48-0.78 (m, 6H), 0.98 (s, 9H).
LC-MS (Methode 12): Rₜ = 3.10 min und 3.16 min; MS (ESIpos): m/z = 435 [M+Na]⁺.

### Beispiel 5A

### 4-(2- {[tert.-Butyl(diphenyl)silyl]oxy} ethoxy)cyclohexancarbaldehyd

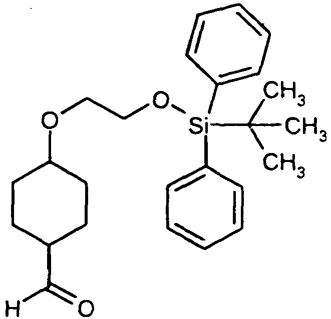

553.7 mg (4.36 mmol) Oxalsäuredichlorid werden in 25 ml Dichlormethan gelöst und auf -78°C gekühlt. Es werden langsam 681.6 mg (8.72 mmol) Dimethylsulfoxid zugegeben. Anschließend wird eine Lösung von 1.20 g (2.91 mmol) der Verbindung aus Beispiel 4A in 10 ml Dichlormethan zugetropft. Es wird 1 h bei -78°C nachgerührt. Man versetzt dann mit 2.0 ml (14.54 mmol) Triethylamin und lässt innerhalb von 1 h auf RT erwärmen. Der Ansatz wird danach auf 20 ml eines 1:1-Gemisches aus ges. wässriger Natriumhydrogencarbonat-Lösung und Ethylacetat gegeben. Nach Trennen der Phasen wird die wässrige Phase noch zweimal mit je 10 ml Ethylacetat extrahiert. Die vereinigten organischen Phasen werden am Rotationsverdampfer vom Lösungsmittel befreit. Der Rückstand wird an Kieselgel 60 chromatographisch gereinigt (Laufmittel: Gradient Cyclohexan/Ethylacetat 20:1 → 5:1).
Ausbeute: 1.00 g (84% d. Th., *cis*/*trans*-Gemisch)
¹H-NMR (400 MHz, DMSO-d₆): δ = 9.57, 9.53 (2 s, 1H, *cis*/*trans*)*,* 7.70-7.62 (m, 4H), 7.49-7.38 (m, 6H), 3.78-3.71 (m, 2H), 3.56-3.42 (m, 3H), 2.37-2.28 (m, 1H), 1.85-1.74 (m, 1H), 1.74-1.48 (m, 5H), 1.29-1.1 (m, 2H), 0.98 (s, 9H).
GC-MS (Methode 8): R, = 9.95 min und 9.98 min; MS (ESIpos): m/z = 428 [M+NH₄]⁺

### Beispiel 6A

### 2-Amino-4-[4-(2-{[tert.-butyl(diphenyl)silyl]oxy}ethoxy)cyclohexyl]-6-mercaptopyridin-3,5-di-carbonitril

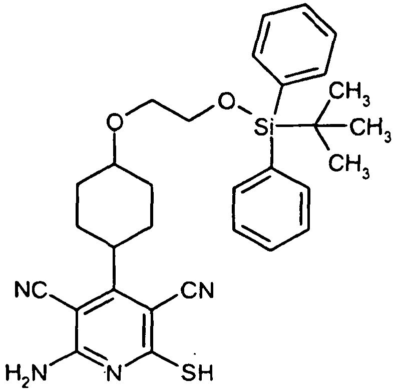

1.00 g (2.44 mmol) der Verbindung aus Beispiel 5A werden in 10 ml trockenem Ethanol gelöst und mit 512.1 mg (5.11 mmol) Cyanothioacetamid sowie 517.3 mg (5.11 mmol) 4-Methylmorpholin versetzt. Die Reaktionsmischung wird 6 h unter Rückfluss und anschließend für 20 h bei RT gerührt. Das Lösungsmittel wird danach am Rotationsverdampfer entfernt. 290 mg des erhaltenen Rohprodukts werden direkt über präparative HPLC gereinigt (Säule: YMC GEL ODS-AQ S-5 / 15 µm; Laufmittelgradient: Acetonitril/Wasser 10:90 → 95:5). Man erhält dabei die cis/trans-Isomere in reiner Form. Durch wiederholte HPLC-Trennungen kann auch der Rest des Rohprodukts gereinigt werden; es wird jedoch für die folgende Reaktion als Isomerengemisch eingesetzt.
*trans*-Isomer:
Ausbeute: 14 mg (1% d. Th.)
¹H-NMR (400 MHz, DMSO-d₆): δ = 12.89 (br. s, 1H), 8.09-7.60 (br. s, 2H), 7.71-7.62 (m, 4H), 7.50-7.39 (m, 6H), 3.78-3.71 (m, 2H), 3.61-3.53 (m, 2H), 2.93-2.78 (m, 1H), 2.08-1.96 (m, 4H), 1.86-1.70 (m, 2H), 1.27-1.10 (m, 2H), 0.99 (s, 9H).
LC-MS (Methode 5): R, = 4.55 min; MS (ESIpos): m/z = 574 [M+NH₄]⁺.
*cis*-Isomer:
Ausbeute: 33 mg (2% d. Th.)
¹H-NMR (400 MHz, DMSO-d₆): δ = 12.87 (br. s, 1H), 7.82-7.56 (br. s, 2H), 7.70-7.62 (m, 4H), 7.48-7.39 (m, 6H), 3.84-3.78 (m, 2H), 3.64-3.60 (m, 1H), 3.54-3.48 (m, 2H), 2.93-2.83 (m, 1H), 2.38-2.23 (m, 2H), 2.02-1.89 (m, 2H), 1.52-1.36 (m, 4H), 0.98 (s, 9H).
LC-MS (Methode 5): Rₜ = 4.65 min; MS (ESIneg): m/z = 555 [M-H]⁻.

### Beispiel 7A

### 2-Amino-4-[4-(2-{[tert.-butyl(diphenyl)silyl]oxy}ethoxy)cyclohexyl]-6-({[2-(4-chlorphenyl)-1,3-thiazol-4-yl)methyl}thio)pyridin-3,5-dicarbonitril

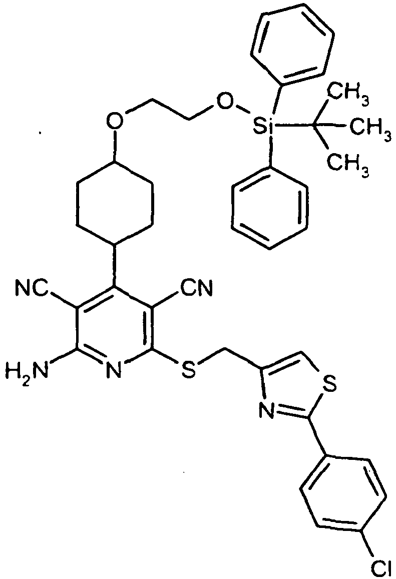

300 mg (0.32 mmol) der Verbindung aus Beispiel 6A (Isomerengemisch) werden in 2.4 ml trockenem DMF gelöst, mit 95 mg (0.39 mmol) 4-(Chlormethyl)-2-(4-chlorphenyl)-1,3-thiazol sowie 109 mg Natriumhydrogencarbonat versetzt und 8 h bei RT gerührt. Das Gemisch wird danach direkt über präparative HPLC gereinigt (Säule: YMC GEL ODS-AQ S-5 / 15 µm; Laufmittelgradient: Acetonitril/Wasser 10:90 → 95:5), wobei die *cis*/*trans-*Isomere getrennt werden.
trans-Isomer:
Ausbeute: 35 mg (14% d. Th.)
¹H-NMR (400 MHz, DMSO-d₆): δ = 8.21-7.82 (br. s, 2H), 7.95 (d, 2H), 7.89 (s, 1H), 7.70-7.62 (m, 4H), 7.56 (d, 2H), 7.50-7.40 (m, 6H), 4.58 (s, 2H), 3.73 (t, 2H), 3.56 (t, 2H), 2.89-2.78 (m, 1H), 2.13-2.05 (m, 2H), 2.05-1.92 (m, 2H), 1.80-1.70 (m, 2H), 1.23-1.11 (m, 2H), 0.99 (s, 9H).
LC-MS (Methode 4): R, = 3.76 min; MS (ESIpos): m/z = 764 [M+H]⁺.
*cis*-Isomer:
Ausbeute: 43 mg (17% d. Th.)
¹H-NMR (400 MHz, DMSO-d₆): δ = 8.16-7.22 (br. s, 2H), 7.96-7.90 (m, 2H), 7.88 (s, 1H), 7.69-7.60 (m, 4H), 7.55 (d, 2H), 7.49-7.38 (m, 6H), 4.57 (s, 2H), 3.80 (t, 2H), 3.61 (br. s, 1H), 3.50 (t, 2H), 2.94-2.82 (m, 1H), 2.37-2.20 (m, 2H), 2.01-1.91 (m, 2H), 1.48-1.38 (m, 4H), 0.99 (s, 9H).
LC-MS (Methode 4): R, = 3.80 min; MS (ESIpos): m/z = 764 [M+H]⁺.

### Beispiel 8A

### 4-Hydroxycyclohexancarbonsäuremethylester

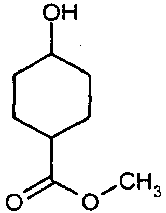

5.00 g (34.68 mmol) 4-Hydroxycyclohexancarbonsäure werden in 80 ml Methanol gelöst und langsam mit 2 ml konz. Schwefelsäure versetzt. Es wird 20 h unter Rückfluss gerührt. Nach Abkühlen auf RT wird der Ansatz vorsichtig in ein Gemisch aus 100 ml Ethylacetat und 100 ml ges. wässriger Natriumhydrogencarbonat-Lösung gegossen. Die Phasen werden getrennt, und die organische Phase wird einmal mit 20 ml ges. wässriger Ammoniumchlorid-Lösung gewaschen. Die organische Phase wird über Magnesiumsulfat getrocknet und das Lösungsmittel am Rotationsverdampfer entfernt. Das Rohprodukt wird ohne weitere Reinigung in der Folgeraktion eingesetzt.
Ausbeute: 4.5 g (74% d. Th., 90% Reinheit)
LC-MS (Methode 13): Rₜ = 1.17 min; MS (ESIpos): m/z = 1.59 [M+H]⁺.

### Beispiel 9A

### Methyl 4-{[tert.-butyl(dimethyl)silyl]oxy}cyclohexancarboxylat

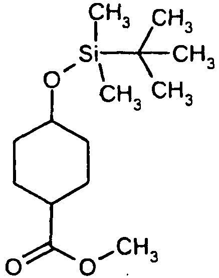

2.00 g (11.38 mmol) des Rohprodukts aus Beispiel 8A (90% Reinheit) werden in 50 ml trockenem DMF gelöst und mit 1.47 g (21.62 mmol) Imidazol sowie 2.40 g (15.93 mmol) *tert*.-Butyldimethyl-silylchlorid versetzt. Das Reaktionsgemisch wird 20 h bei RT gerührt. Der Ansatz wird danach mit je 40 ml Diethylether und ges. wässriger Natriumhydrogencarbonat-Lösung versetzt. Nach Phasentrennung wird die organische Phase über Magnesiumsulfat getrocknet und das Lösungsmittel am Rotationsverdampfer entfernt. Das Rohprodukt wird ohne weitere Reinigung in der Folgereaktion eingesetzt.
Ausbeute: 3.9 g (100% d, Th., 80% Reinheit)
GC-MS (Methode 9): Rₜ = 7.30 min; MS (ESIpos): m/z = 215 [M-C₄H₉]⁺.

### Beispiel 10A

### (4-{[tert.-Butyl(dimethyl)silyl]oxy}cyclohexyl)methanol

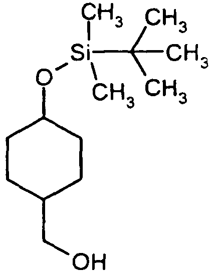

200 mg (0.59 mmol) des Rohprodukts aus Beispiel 9A (80% Reinheit) werden in 1.5 ml Diethylether gelöst. Diese Lösung wird bei RT zu einer Suspension von 13.4 mg (0.35 mmol) Lithiumaluminiumhydrid in 1.5 ml Diethylether getropft. Es wird 20 h bei RT gerührt. Der Ansatz wird danach mit 12 µl Wasser, 12 µl 15%-iger Kalilauge sowie 3 ml Diethylether versetzt und 30 min bei RT gerührt. Das Reaktionsgemisch wird anschließend über eine Kartusche, die mit 2.4 g Kieselgel und 2.4 g Extrelut beladen ist, filtriert (Laufmittel: Dichlormethan/Ethano) 10:1). Das Lösungsmittel wird am Rotationsverdampfer entfernt und der Rückstand an Kieselgel 60 chromatographisch gereinigt (Laufmittel: Gradient Cyclohexan/Ethylacetat 20:1 → 10:1).
Ausbeute: 88 mg (61% d. Th.)
¹H-NMR (400 MHz, DMSO-d₆): δ = 4.31 (t, 1H), 3.95-3.89 (m, 1H), 3.17 (t, 2H), 1.58-1.49 (m, 2H), 1.43-1.20 (m, 7H), 0.83 (s, 9H), 0.01 (s, 6H).
GC-MS (Methode 9): Rₜ = 6.94 min; MS (ESIpos): m/z = 245 [M+H]⁺.

### Beispiel 11A

### 4-{[tert.-Butyl(dimethyl)silyl]oxy}cyclohexancarbaldehyd

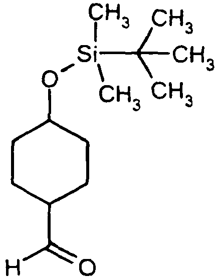

0.93 g (7.31 mmol) Oxalsäuredichlorid werden in 50 mol Dichlormethan gelöst, auf -78°C gekühlt und langsam mit 1.14 g (14.63 mmol) Dimethylsulfoxid versetzt. Anschließend wird eine Lösung von 1.49 g (4.88 mmol) der Verbindung aus Beispiel 10A (inkl. Nachsynthesen), gelöst in 10 ml Dichlormethan, zugegeben. Das Reaktionsgemisch wird 1 h bei -78°C nachgerührt. Danach wird langsam mit 3.4 ml (24.38 mmol) Triethylamin versetzt und innerhalb von 1 h auf RT erwärmt. Der Ansatz wird dann mit 50 ml Ethylacetat und 30 ml Natriumhydrogencarbonat-Lösung versetzt. Die Phasen werden getrennt, und die wässrige Phase wird zweimal mit je 20 ml Ethylacetat extrahiert. Die vereinigten organischen Phasen werden über Magnesiumsulfat getrocknet. Das Lösungsmittel wird am Rotationsverdampfer entfernt und der Rückstand an Kieselgel 60 chromatographisch gereinigt (Laufmittel: Gradient Cyclohexan/Ethylacetat 50:1 → 10:1).
Ausbeute: 690 mg (56% d. Th.)
¹H-NMR (400 MHz, DMSO-d₆): δ = 9.53 (s, 1H), 3.91-3.82 (m, 1H), 2.38-2.28 (m, 1H), 1.80-1.21 (m, 8H), 0.87 (s, 9H), 0.02 (s, 6H).
GC-MS (Methode 9): Rₜ = 6.59 min; MS (ESIpos): m/z = 185 [M-C₄H₉]⁺.

### Beispiel 12A

### 2-Amino-4-(4-{[tert.-butyl(dimethyl)silyl]oxy} cyclohexyl)-6-mercaptopyridin-3,5-dicarbonitril

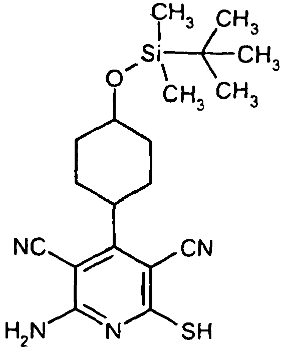

690 mg (2.85 mmol) der Verbindung aus Beispiel 11A und 598 mg (5.98 mmol) Cyanothioacetamid werden in 20 ml Ethanol gelöst und mit 604 mg (5.98 mmol) N-Methylmorpholin versetzt. Das Reaktionsgemisch wird 4 h bei +90°C gerührt. Nach Abkühlen auf RT wird für weitere 20 h bei RT nachgerührt. Der ausgefallene Niederschlag wird abgesaugt und mit 3 ml auf 0°C gekühltem Ethanol gewaschen. Das gemeinsame Filtrat wird am Rotationsverdampfer eingeengt und der Rückstand über präparative HPLC gereinigt (Säule: YMC GEL ODS-AQ S-5 / 15 µm; Laufmittelgradient: Acetonitril/Wasser 10:90 → 95:5), wobei die cis/trans-Isomere getrennt werden.
*cis*-Isomer:
Ausbeute: 197 mg (17% d. Th.)
¹H-NMR (400 MHz, DMSO-d₆): δ = 12.89 (s, 1H), 7.99-7.57 (br. s, 2H), 4.10-4.04 (m, 1H), 2.91-2.81 (m, 1H), 2.51-2.32 (m, 1H), 1.79-1.69 (m, 2H), 1.57-1.39 (m, 6H), 0.90 (s, 9H), 0.08 (s, 6H).
LC-MS (Methode 4): R, = 3.10 min; MS (ESIpos): m/z= 389 [M+H]⁺.
*trans*-Isomer:
Ausbeute: 23 mg (2% d. Th.)
¹H-NMR (400 MHz, DMSO-d₆): δ = 12.99-12.82 (br. s, 1H), 8.10-7.57 (br. s, 2H), 3.68-3.54 (m, 1H), 2.88-2.77 (m, 1H), 2.15-1.92 (m, 4H), 1.83-1.69 (m, 2H), 1.37-1.21 (m, 2H), 0.87 (s, 9H), 0.08 (s, 6H).
LC-MS (Methode 13): Rₜ = 2.88 min; MS (ESIpos): m/z = 389 [M+H]⁺.

### Beispiel 13A

### 2-Amino-4-(trans-4-{[tert.-butyl(dimethyl)silyl]oxy}cyclohexyl)-6-({[2-(4-chlorphenyl)-1,3-thiazol-4-yl]methyl}thio)pyridin-3,5-dicarbonitril

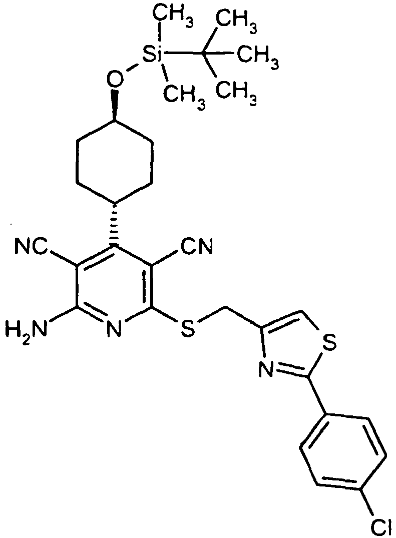

23 mg (0.06 mmol) der Verbindung aus Beispiel 12A (trans-Isomer), 17 mg (0.07 mmol) 4-(Chlormethyl)-2-(4-chlorphenyl)-1,3-thiazol und 20 mg (0.24 mmol) Natriumhydrogencarbonat werden in 2 ml trockenem DMF vorgelegt und 20 h bei RT gerührt. Der Ansatz wird danach direkt über präparative HPLC gereinigt (Säule: YMC GEL ODS-AQ S-5 / 15 µm; Laufmittelgradient: Acetonitril/Wasser 10:90 → 95:5).
Ausbeute: 25 mg (71% d. Th.)
LC-MS (Methode 5): Rₜ = 5.33 min; MS (ESIpos): m/z = 596 [M+H]⁺.

### Beispiel 14A

### 2-Amino-4-(cis-4-{[tert.-butyl(dimethyl)silyl]oxy}cyclohexyl)-6-({[2-(4-chlorphenyl)-1,3-thiazol-4-yl]methyl}thio)pyridin-3,5-dicarbonitril

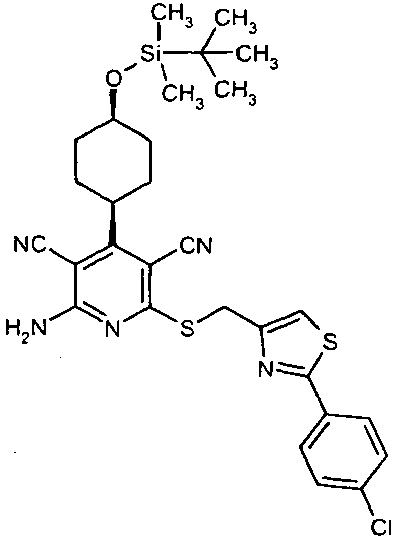

35 mg (0.08 mmol) der Verbindung aus Beispiel 12A (cis-Isomer), 23 mg (0.09 mmol) 4-(Chlormethyl)-2-(4-chlorphenyl)-1,3-thiazol und 26 mg (0.31 mmol) Natriumhydrogencarbonat werden in 2 ml trockenem DMF vorgelegt und 20 h bei RT gerührt. Der Ansatz wird danach direkt über präparative HPLC gereinigt (Säule: YMC GEL ODS-AQ S-5 / 15 5 µm; Laufmittelgradient: Acetonitril/Wasser 10:90 → 95:5).
Ausbeute: 24 mg (51% d. Th.)
LC-MS (Methode 5): Rₜ = 5.40 min; MS (ESIpos): m/z = 596 [M+H]⁺.

### Beispiel 15A

### 2-Amino-4-(cis-4-{[tert-butyl(dimethyl)silyl]oxy}cyclohexyl)-6-[(pyridin-3-ylmethyl)thio]-pyridin-3,5-dicarbonitril

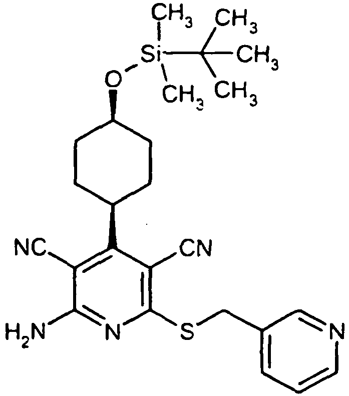

97 mg (0.23 mmol) der Verbindung aus Beispiel 12A (*cis*-Isomer), 46 mg (0.28 mmol) 3-Pyridinmethylchlorid-Hydrochlorid und 78 mg (0.93 mmol) Natriumhydrogencarbonat werden in 2 ml trockenem DMF vorgelegt und 20 h bei RT gerührt. Der Ansatz wird danach filtriert und das Filtrat direkt über präparative HPLC gereinigt (Säule: YMC GEL ODS-AQ S-5 / 15 µm; Laufmittelgradient: Acetonitril/Wasser 10:90 → 95:5).
Ausbeute: 80 mg (72% d. Th.)
¹H-NMR (500 MHz, DMSO-d₆): δ = 8.70 (s, 1H), 8.38 (d, 1H), 8.20-7.72 (br. s, 2H), 7.87 (d, 1H), 7.30-7.25 (m, 1H), 4.39 (s, 2H), 4.00 (s, 1H), 2.85-2.77 (m, 1H), 2.36 (dq, 2H), 1.71-1.62 (m, 2H), 1.52-1.43 (m, 2H), 1.41-1.34 (m, 2H), 0.83 (s, 9H), 0.01 (s, 6H).
LC-MS (Methode 12): Rₜ = 3.03 min; MS (ESIpos): m/z = 480 [M+H]⁺.

### Beispiel 16A

### 2-Amino-4-(cis-4-{[tert.-butyl(dimethyl)silyl]oxy}cyclohexyl)-6-[({2-[(4-fluorphenyl)amino]-1,3-thiazol-4-yl}methyl)thio]pyridin-3,5-dicarbonitril

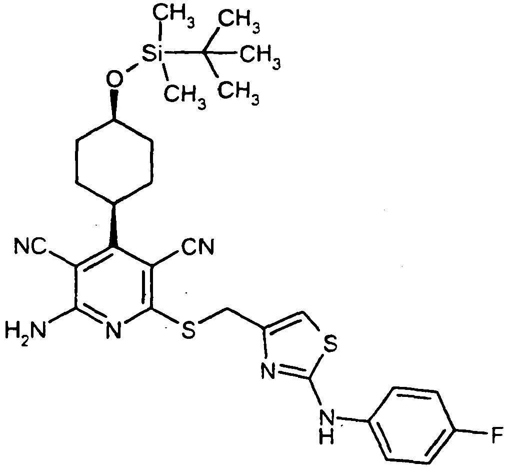

43 mg (0.26 mmol) 4-Fluorphenylthioharnstoff und 31 mg (0.24 mmol) 1,3-Dichloraceton werden in 2 ml DMF gelöst und 3 h bei +80°C gerührt. Nach Abkühlen auf RT wird das Reaktionsgemisch mit 93 mg (0.23 mmol) der Verbindung aus Beispiel 12A (*cis*-Isomer) sowie 78 mg (0.93 mmol) Natriumhydrogencarbonat versetzt und 20 h bei RT gerührt. Der Ansatz wird danach direkt über präparative HPLC gereinigt (Säule: YMC GEL ODS-AQ S-5 / 15 µm; Laufmittelgradient: Acetonitrit/Wasser 10:90 → 95:5).
Ausbeute: 66 mg (43% d. Th.)
¹H-NMR (500 MHz, DMSO-d₆): δ = 10.22 (s, 1H), 8.05-7.87 (br. s, 2H), 7.63-7.56 (m, 2H), 7.12 (t, 2H), 6.92 (s, 1H), 4.40 (s, 2H), 4.05 (br. s, 1H), 2.91-2.82 (m, 1H), 2.48-2.71 (m, 2H), 1.76-1.68 (m, 2H), 1.58-1.47 (m, 2H), 1.47-1.39 (m, 2H), 0.89 (s, 9H), 0.06 (s, 6H).
LC-MS (Methode 13): Rₜ = 3.49 min; MS (ESIpos): m/z = 595 [M+H]⁺.

### Beispiel 17A

### tert.-Butyl 4-(2-amino-3,5-dicyano-6-mercaptopyridin-4-yl)piperidin-1-carboxylat

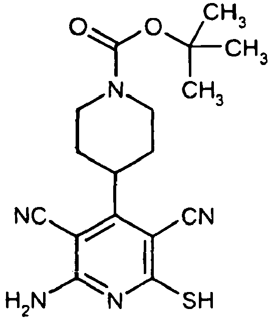

3.00 g (14.07 mmol) *tert*.-Butyl 4-formylpiperidin-1-carboxylat und 2.82 g (28.13 mmol) Cyanothioacetamid werden in 32 ml Ethanol gelöst und mit 2.85 g (28.13 mmol) 4-Methylmorpholin versetzt. Es wird 4 h unter Rückfluss und anschließend weitere 8 h bei RT gerührt. Das Reaktionsgemisch wird danach am Rotationsverdampfer eingeengt und der Rückstand an Kieselgel 60 chromatographisch aufgereinigt (Laufmittel: Gradient Dichlormethan/Ethanol 50:1 → 5:1). Das erhaltene Produkt wird ohne weitere Reinigung in den Folgereaktionen eingesetzt.
Ausbeute: 2.59 g (32% d. Th., 63% Reinheit)
LC-MS (Methode 13): R, = 2.02 min; MS (ESIneg): m/z = 358 [M-H]⁻.

### Beispiel 18A

### tert.-Butyl 4-{2-amino-3,5-dicyano-6-[({2-[(4-fluorphenyl)amino]-1,3-thiazol-4-yl}methyl)thio]-pyridin-4-yl}piperidin-1-carboxylat

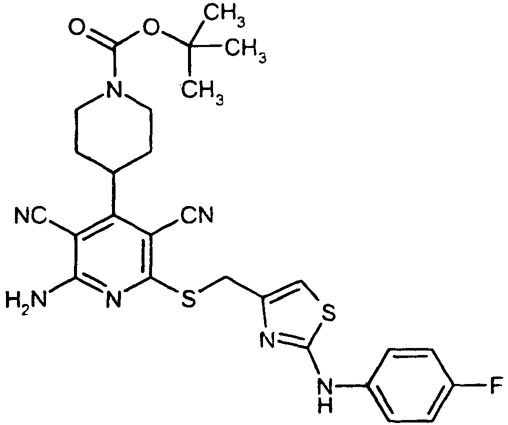

100 mg (0.20 mmol) der Verbindung aus Beispiel 17A (70% Reinheit) und 130 mg (0.24 mmol) 4-(Chlormethyl)-*N*-(4-fluorphenyl)-1,3-thiazol-2-amin (aus 4-Fluorphenylthioharnstoff und 1,3-Dichloraceton) werden in 2.5 ml trockenem DMF gelöst und mit 100 mg (0.98 mmol) Natriumhydrogencarbonat versetzt. Das Reaktionsgemisch wird 8 h bei RT gerührt. Nach Filtration wird das Filtrat im Vakuum eingeengt und der verbleibende Rückstand über präparative HPLC gereinigt (Säule: YMC GEL ODS-AQ S-5 / 15 µm; Laufmittelgradient: Acetonitril/Wasser 10:90 → 95:5).
Ausbeute: 40 mg (32% d. Th., 88% Reinheit)
LC-MS (Methode 13): R, = 2.89 min; MS (ESIpos): m/z = 566 [M+H]⁺.

### Beispiel 19A

### tert.-Butyl 4-(2-amino-6-({[2-(4-chlorphenyl)-1,3-thiazol-4-yl]methyl}thio)-3,5-dicyanopyridin-4-yl]piperidin-1-carboxylat

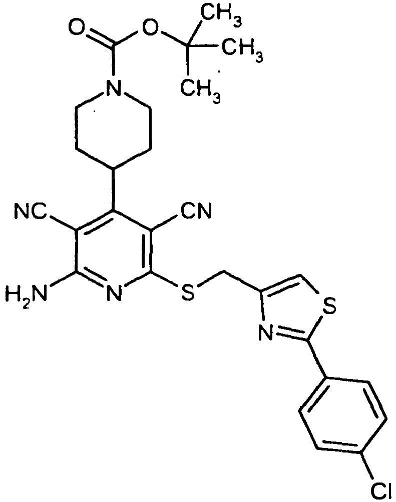

Die Titelverbindung wird analog zu Beispiel 18A ausgehend von Beispiel 17A und 4-(Chlormethyl)-2-(4-chlorphenyl)-1,3-thiazol erhalten.
Ausbeute: 41 % d. Th.
LC-MS (Methode 13): Rₜ = 3.20 min; MS (ESIpos): m/z = 567 [M+H]⁺.

### Beispiel 20A

### 4-({[tert.-Butyl(diphenyl)silyl]oxy}methyl)piperidin

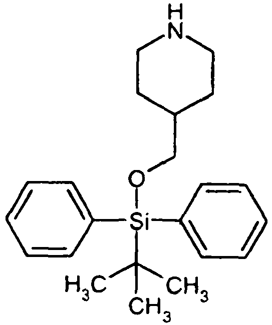

5.00 g (43.41 mmol) 4-Piperidinmethanol werden in 400 mol Dichlormethan gelöst und nacheinander mit 9.1 ml (65.11 mmol) Triethylamin, 212 mg (1.74 mmol) 4-*N,N*-Dimethylaminopyridin und 16.9 ml (65.12 mmol) *tert*.-Butyldiphenylchlorsilan versetzt. Das Reaktionsgemisch wird 8 h bei RT gerührt. Nach Zugabe von 50 ml Dichlormethan wird einmal mit 20 ml Wasser und einmal mit 20 ml ges. wässriger Natriumchlorid-Lösung gewaschen. Die organische Phase wird über Magnesiumsulfat getrocknet. Das Rohprodukt wird ohne weitere Reinigung in der Folgereaktion eingesetzt.
Ausbeute: 21.16 g (96% d. Th., 70% Reinheit)
LC-MS (Methode 4): Rₜ = 1.91 min; MS (ESIpos): m/z = 354 [M+H]⁺.

### Beispiel 21A

### 2-[4-({[tert.-Butyl(diphenyl)silyl]oxy} methyl)piperidin-1-yl]-2-oxoethyl-acetat

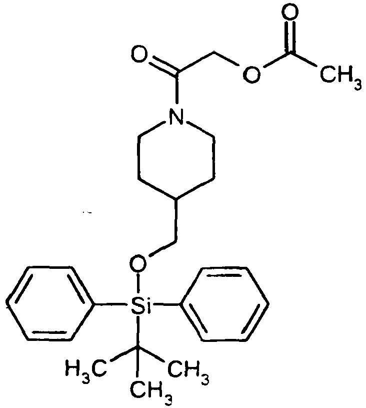

5 g des Rohprodukts aus Beispiel 20A werden in 24 ml Dichlormethan suspendiert und auf 0°C gekühlt. Es werden 1.88 g (13.79 mmol) Acetoxyessigsäurechlorid und 5.9 ml (42.42 mmol) Triethylamin zugegeben. Das Reaktionsgemisch wird 8 h bei RT gerührt. Nach Zugabe von 25 ml Dichlormethan wird einmal mit 10 ml Wasser und einmal mit 10 ml ges. wässriger Natriumchlorid-Lösung gewaschen. Die organische Phase wird über Magnesiumsulfat getrocknet. Nach Entfernten des Lösungsmittel am Rotationsverdampfer wird der Rückstand an Kieselgel 60 chromatographisch gereinigt (Laufmittel: Gradient Cyclohexan/Ethylacetat 20:1 → 1:1).
Ausbeute: 4.04 g (84% d. Th.)
LC-MS (Methode 4): Rₜ = 3.21 min; MS (ESIpos): m/z = 454 [M+H]⁺.

### Beispiel 22A

### 2-[4-(Hydroxymethyl)piperidin-1-yl]-2-oxoethyl-acetat

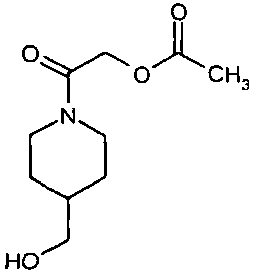

4.04 g (8.91 mmol) der Verbindung aus Beispiel 21A werden in 69 ml trockenem THF gelöst und mit 9.8 mol (9.8 mmol) einer 1 M Lösung von Tetrabutylammoniumfluorid in THF versetzt. Das Reaktionsgemisch wird 48 h bei RT gerührt. Das Lösungsmittel wird danach am Rotationsverdampfer entfernt und der Rückstand an Kieselgel 60 chromatographisch gereinigt (Laufmittel: Gradient Dichlormethan/Ethanol 200:1 → 1:1).
Ausbeute: 800 mg (42% d. Th.)
GC-MS (Methode 8): Rₜ = 6.59 min; MS (ESIpos): m/z = 216 [M+H]⁺.

### Beispiel 23A

### 2-(4-Formylpiperidin-1-yl)-2-oxoethyl-acetat

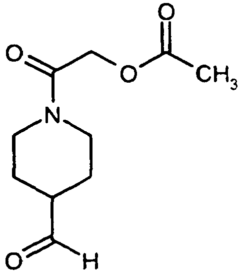

600 mg (2.79 mmol) der Verbindung aus Beispiel 22A werden in 6 ml trockenem Dichlormethan gelöst und mit 1.5 g gepulvertem Molekularsieb (4Å) und 490 mg (4.18 mmol) *N*-Methylmorpholin-N-oxid versetzt. Anschließend werden 49 mg (0.14 mmol) Tetrapropylammoniumperruthenat zugegeben und der Ansatz 1 h bei RT gerührt. Das Reaktionsgemisch wird danach direkt an Kieselgel 60 chromatographisch gereinigt (Laufmittel: Gradient Dichlormethan/Ethanol 100:1 → 20:1).
Ausbeute: 194 mg (33% d. Th.)
GC-MS (Methode 8): Rₜ = 6.78 min; MS (ESIpos): m/z = 214 [M+H]⁺.

### Beispiel 24A

### 2-[4-(2-Amino-3,5-dicyano-6-mercaptopyridin-4-yl)piperidin-1-yl]-2-oxoethyl-acetat

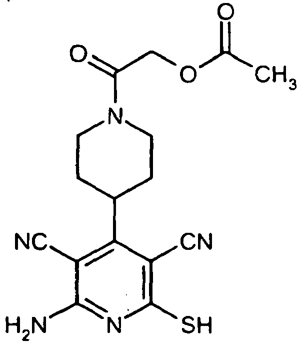

194 mg (0.91 mmol) der Verbindung aus Beispiel 23A und 182 mg (1.82 mmol) Cyanothioacetamid werden in 2 ml trockenem Ethanol vorgelegt und mit 184 mg (1.82 mmol) 4-Methylmorpholin versetzt. Das Reaktionsgemisch wird 4 h bei +78°C gerührt. Nach Abkühlen auf RT wird weitere 8 h bei dieser Temperatur nachgerührt. Nach Entfernen des Lösungsmittels am Rotationsverdampfer wird der Rückstand an Kieselgel 60 chromatographisch aufgereinigt (Laufmittel: Gradient Dichlormethan/Ethanol 20:1 → 1:1). Das erhaltene Produkt wird ohne weitere Reinigung in der Folgereaktion eingesetzt.
Ausbeute: 102 mg (17% d. Th., 55% Reinheit)
LC-MS (Methode 12): Rₜ = 1.13 min; MS (ESIpos): m/z = 360 [M+H]⁺.

### Beispiel 25A

### 2-{4-[2-Amino-6-({[2-(4-chlorphenyl)-1,3-thiazol-4-yl]methyl}thio)-3,5-dicyanopyridin-4-yl]-piperidin-1-yl}-2-oxoethyl-acetat

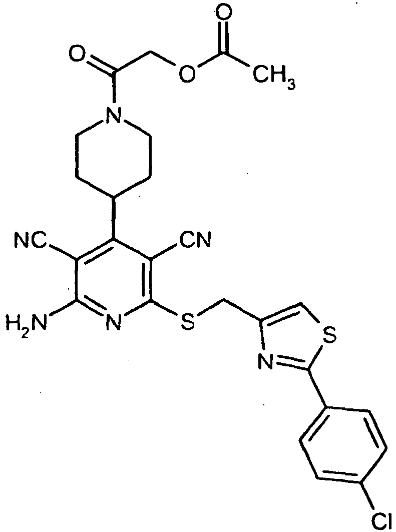

34 mg (0.06 mmol) der Verbindung aus Beispiel 24A werden in 1.2 ml trockenem DMF gelöst und mit 17 mg (0.07 mmol) 4-(Chlormethyl)-2-(4-chlorphenyl)-1,3-thiazol sowie 19 mg (0.23 mmol) Natriumhydrogencarbonat versetzt. Das Reaktionsgemisch wird 8 h bei RT gerührt. Der Ansatz wird danach filtriert und das Filtrat mit ca. 0.5 ml Wasser versetzt. Der ausgefallene Niederschlag wird abgesaugt und im Vakuum bei +50°C getrocknet.
Ausbeute: 29 mg (74% d. Th., 88% Reinheit)
LC-MS (Methode 3): R, = 2.58 min; MS (ESIpos): m/z = 567 [M+H]⁺.

### Beispiel 26A

### 3-(4-({[tert.-Butyl(diphenyl)silyl]oxy}methyl)piperidin-1-yl]propyl-acetat

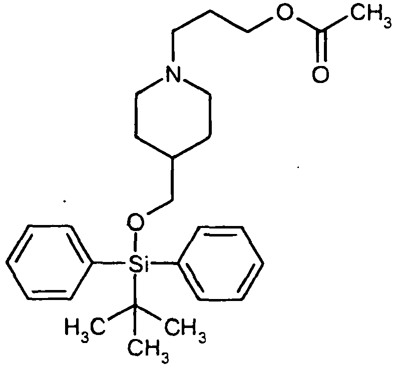

5.00 g (12.44 mmol) der Verbindung aus Beispiel 20A werden in 57 ml Acetonitril gelöst und mit 4.51 g (24.89 mmol) 3-Brompropylacetat sowie 3.44 g (24.89 mmol) Kaliumcarbonat versetzt. Das Reaktionsgemisch wird 8 h bei +80°C gerührt. Nach Filtration wird das Lösungsmittel am Rotationsverdampfer entfernt. Der Rückstand wird in 50 ml Ethylacetat aufgenommen und jeweils einmal mit 10 ml Wasser und 10 ml ges. wässriger Natriumhydrogencarbonat-Lösung gewaschen. Die organische Phase wird über Magnesiumsulfat getrocknet und das Lösungsmittel am Rotationsverdampfer entfernt. Das Rohprodukt wird ohne weitere Reinigung in der Folgereaktion eingesetzt.
Ausbeute: 6.38 g (93% d. Th., 82% Reinheit)
LC-MS (Methode 2): Rₜ = 4.64 min; MS (ESIpos): m/z = 454 [M+H)⁺.

### Beispiel 27A

### 3-[4-(Hydroxymethyl)piperidin-1-yl]propyl-acetat

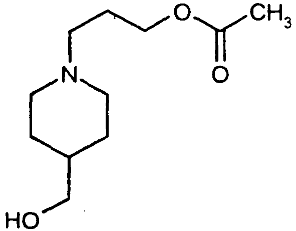

6.38 g (14.06 mmol) des Rohprodukts aus Beispiel 26A werden in 108 ml trockenem THF gelöst und mit 4.04 g (15.47 mmol) Tetra-n-butylammoniumfluorid versetzt. Das Reaktionsgemisch wird 48 h bei RT gerührt. Nach Entfernen des Lösungsmittels wird der Rückstand direkt an Kieselgel 60 chromatographisch gereinigt (Laufmittel: Gradient Dichlonnethan/Ethanol 20:1 → 1:1).
Ausbeute: 1.59 g (53% d. Th.)
¹H-NMR (400 MHz, DMSO-d₆): δ = 4.39 (t, 1H), 4.00 (t, 2H), 3.22 (t, 2H), 2.89-2.78 (m, 2H), 2.37-2.24 (m, 2H), 2.00 (s, 3H), 1.90-1.78 (m, 2H), 1.76-1.67 (m, 2H), 1.66-1.53 (m, 2H), 1.39-1.23 (m, 1H), 1.17-1.02 (m, 2H).
MS (ESIpos): m/z = 216 [M+H]⁺.

### Beispiel 28A

### 3-(4-Formylpiperidin-1-yl)propyl-acetat

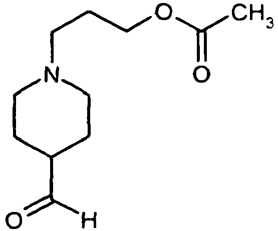

1.36 g (6.30 mmol) der Verbindung aus Beispiel 27A werden in 14 ml trockenem Dichlormethan gelöst und nacheinander mit 3.39 g Molekularsieb (4Å), 1.11 g (9.44 mmol) N-Methylmorpholin-N-oxid sowie 111 mg (0.32 mmol) Tetrapropylammoniumperruthenat versetzt. Das Reaktionsgemisch wird 1 h bei RT gerührt und dann direkt an Kieselgel 60 chromatographisch gereinigt (Laufmittel: Gradient Dichlormethan/Ethanol 100:1 → 10:1).
Ausbeute: 634 mg (48% d. Th.)
GC-MS (Methode 8): Rₜ = 5.53 min; MS (ESIpos): m/z = 214 [M+H]⁺.

### Beispiel 29A

### 3-[4-(2-Amino-3,5-dicyano-6-mercaptopyridin-4-yl)piperidin-1-yl]propyl-acetat

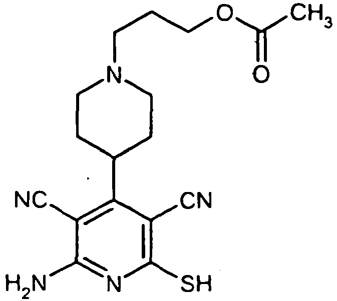

508 mg (2.38 mmol) der Verbindung aus Beispiel 28A und 477 mg (4.77 mmol) Cyanothioacetamid werden in 5 ml Ethanol vorgelegt und mit 482 mg (4.77 mmol) 4-Methylmorpholin versetzt. Das Reaktionsgemisch wird 4 h bei +78°C gerührt. Nach Abkühlen auf RT wird für weitere 8 h bei dieser Temperatur nachgerührt. Das Lösungsmittel wird am Rotationsverdampfer entfernt, der Rückstand auf Diatomeenerde aufgezogen und dann an Kieselgel 60 chromatographisch gereinigt (Laufmittel: Gradient Dichlormethan/Ethanol 50:1 → 3: 1).
Ausbeute: 435 mg (48% d. Th.)
LC-MS (Methode 14): Rₜ = 2.09 min; MS (ESIpos): m/z = 360 [M+H]⁺.

### Beispiel 30A

### Tetrahydro-2H-pyran-2-carbaldehyd

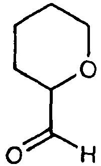

1000 mg (8.61 mmol) 2-(Nydroxymethyl)-tetrahydro-2*H*-pyran werden in 27 ml trockenem Dichlormethan gelöst und mit 1.5 g gepulvertem Molekularsieb (4Å) sowie 1.5 g (12.91 mmol) N-Methylmorpholin-*N*-oxid versetzt. Anschließend werden 151 mg (0.43 mmol) Tetrapropylammoniumperruthenat zugegeben und das Reaktionsgemisch 1 h bei RT gerührt. Nach Entfernen des Lösungsmittels wird der Ansatz an Kieselgel 60 chromatographisch vorgereinigt (Laufmittel: Gradient Dichlormethan/Ethanol 200:1 → 20:1). Das erhaltene Produkt wird ohne weitere Reinigung in der Folgereaktion eingesetzt.
Ausbeute: 502 mg (51% d. Th.)
GC-MS (Methode 8): Rₜ = 2.07 min; MS (ESIpos): m/z = 114 [M+H]⁺.

### Beispiel 31A

### 2-Amino-6-mercapto-4-(tetrahydro-2H-pyran-2-yl)pyridin-3,5-dicarbonitril

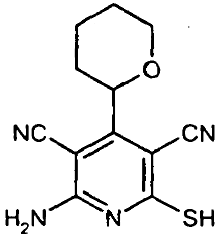

370 mg (2.66 mmol) des Rohprodukts aus Beispiel 30A und 921 mg (9.20 mmol) Cyanothioacetamid werden in 8.2 ml Ethanol gelöst und mit 930 mg (9.20 mmol) 4-Methylmorpholin versetzt. Das Reaktionsgemisch wird 3 h bei +78°C gerührt. Nach Abkühlen auf RT wird für weitere 8 h bei dieser Temperatur nachgerührt. Nach Entfernen des Lösungsmittels wird der Ansatz direkt an Kieselgel 60 chromatographisch vorgereinigt (Laufmittel: Gradient Dichlormethan/Ethanol 200:1 → 20:1). Das erhaltene Produkt wird ohne weitere Reinigung in der Folgereaktion eingesetzt.
Ausbeute: 387 mg (26% d. Th., 76% Reinheit)
LC-MS (Methode 7): Rₜ = 1.93 min; MS (ESIpos): m/z = 261 [M+H]⁺.

### Beispiel 32A

### 2-Amino-6-mercapto-4-(tetrahydro-2H-pyran-3-yl)pyridin-3,5-dicarbonitril

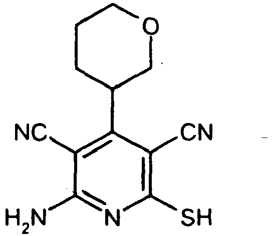

670 mg (5.87 mmol) Tetrahydro-2*H*-pyran-3-carbaldehyd [herstellbar gemäß E. J. Corey et al., J. Am. Chem. Soc. 120, 13000-13001 (1998)] und 1.23 g (12.33 mmol) Cyanothioacetamid werden in 11 ml Ethanol gelöst und mit 1.23 g (12.33 mmol) 4-Methylmorpholin versetzt. Das Reaktionsgemisch wird 3 h bei +78°C gerührt. Nach Abkühlen auf RT wird für weitere 8 h bei dieser Temperatur nachgerührt. Dabei fällt ein gelber Niederschlag aus. Dieser wird abfiltriert, auf Diatomeenerde adsorbiert und an Kieselgel 60 chromatographisch vorgereinigt (Laufmittel: Gradient Dichlormethan/Ethanol 200:1 → 1:1). Das erhaltene Produkt wird ohne weitere Reinigung in der Folgereaktion eingesetzt.
Ausbeute: 564 mg (25% d. Th., 69% Reinheit)
LC-MS (Methode 7): Rₜ = 1.62 min; MS (ESIpos): m/z = 261 [M+H]⁺.

### Beispiel 33A

### 2-Amino-4-cyclohexyl-6-mercaptopyridin-3,5-dicarbonitril

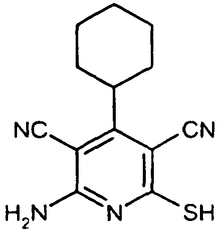

4.00 g (35.66 mmol) Cyclohexylcarbaldehyd und 7.14 g (71.32 mmol) Cyanothioacetamid werden in 80 ml Ethanol gelöst und mit 7.21 g (71.32 mmol) 4-Methylmorpholin versetzt. Das Reaktionsgemisch wird 3 h bei +78°C gerührt. Nach Abkühlen auf RT wird für weitere 8 h bei dieser Temperatur nachgerührt. Nach Absaugen des entstandenen Niederschlags wird das Filtrat am Rotationsverdampfer eingeengt und der verbleibende Rückstand an Kieselgel 60 chromatographisch gereinigt (Laufmittel: Gradient Dichlormethan/Ethanol 100:1 → 20:1).
Ausbeute: 7.78 g (82% d. Th.)
¹H-NMR (400 MHz, DMSO-d₆): δ = 7.37 (br. s, 2H), 2.92-2.79 (m, 1H), 2.09-1.92 (m, 2H), 1.90-1.77 (m, 2H), 1.75-1.54 (m, 3H), 1.37-1.08 (m, 3H).
LC-MS (Methode 4): Rₜ = 2.21 min; MS (ESIpos): m/z = 259 [M+H]⁺.

### Beispiel 34A

### 2-Amino-6-mercapto-4-(tetrahydro-2H-pyran-4-yl)pyridin-3,5-dicarbonitril

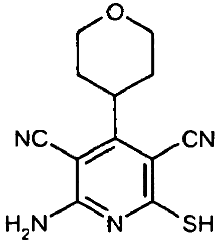

600 mg (5.26 mmol) Tetrahydropyran-4-carbaldehyd und 1.05 g (10.50 mmol) Cyanothioacetamid werden in 10 ml Ethanol gelöst und mit 1.06 g (10.50 mmol) 4-Methylmorpholin versetzt. Das Reaktionsgemisch wird 3 h bei +80°C gerührt. Der entstandene Niederschlag wird abfiltriert und ohne weitere Reinigung in der Folgereaktion eingesetzt.
Ausbeute: 720 mg (45% d. Th., 87% Reinheit)
¹H-NMR (400 MHz, DMSO-d₆): δ = 12.94 (br. s, 1H), 7.82 (br. s, 2H), 3.99 (dd, 2H), 3.38 (dd, 2H), 3.13 (m, 1H), 2.29-2.19 (m, 2H), 1.60 (d, 2H).
LC-MS (Methode 12): Rₜ = 1.15 min; MS (ESIpos): m/z = 261 [M+H]⁺.

### Beispiel 35A

### [6-(Pyridin-4-ylamino)pyridin-2-yl]methanol

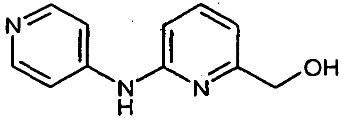

1.35 g (14.3 mmol) 4-Aminopyridin und 1.34 g (7.1 mmol) (6-Brompyridin-2-yl)-methanol werden für 4 h bei 150°C gerührt. Nach Abkühlen auf RT wird das Reaktionsgemisch mit 50 ml Acetonitril versetzt und 20 min gerührt. Der entstandene Niederschlag wird bei 0°C abgesaugt und mit 10 ml Acetonitril gewaschen.
Ausbeute: 1.25 g (39% d. Th., 89% Reinheit)
LC-MS (Methode 14): Rₜ = 1.76 min; MS (ESIpos): m/z = 202 [M+H]⁺.

### Beispiel 36A

### 6-(Chlormethyl)-N-pyridin-4-yl-pyridin-2-amin

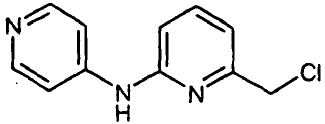

50 mg (0.22 mmol) der Verbindung aus Beispiel 35A und 53 mg (0.44 mmol) Thionylchlorid werden bei 0°C in 1.5 ml Dichlormethan vorgelegt und nach Erwärmen auf RT 12 h bei dieser Temperatur gerührt. Das Lösungsmittel wird am Rotationsverdampfer entfernt und das verbleibende Produkt direkt weiter umgesetzt.
Ausbeute: 65 mg (99% d. Th., 74% Reinheit)
LC-MS (Methode 14): Rₜ = 2.26 min; MS (ESIpos): m/z = 220 [M+H]⁺.

### Beispiel 37A

### 2-Chlor-6-({[2-(4-chlorphenyl)-1,3-thiazol-4-yl]methyl}thio)-4-cyclohexylpyridin-3,5-dicarbonitril

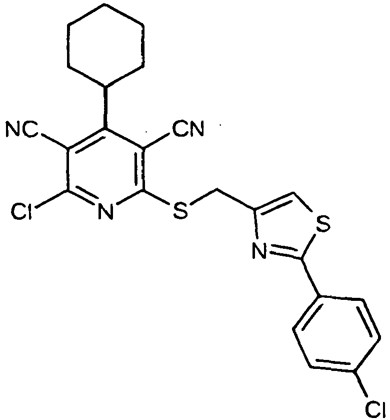

1.63 g (13.91 mmol) Isopentylnitrit und 1.87 g (13.91 mmol) Kupfer(II)-chlorid werden in 18 ml Acetonitril vorgelegt und mit 1.08 g (2.32 mmol) der Verbindung aus Beispiel 29 versetzt. Das Reaktionsgemisch wird 3 h bei +60°C gerührt. Nach Abkühlen auf RT wird das Reaktionsgemisch mit 20 ml 1 N Salzsäure versetzt. Die wässrige Phase wird zweimal mit je 30 ml Ethylacetat extrahiert. Die vereinigten organischen Phasen werden einmal mit 15 ml ges. wässriger Natriumhydrogencarbonat-Lösung und einmal mit 15 ml ges. wässriger Natriumchlorid-Lösung gewaschen. Nach Trocknen über Magnesiumsulfat wird das Lösungsmittel am Rotationsverdampfer entfernt. Der Rückstand wird an Kieselgel 60 chromatographisch gereinigt (Laufmittel: Gradient Cyclohexan/Ethylacetat 100:1 → 5:1).
Ausbeute: 626 mg (56% d. Th.)
¹H-NMR (400 MHz, DMSO-d₆): δ = 7.94 (d, 2H), 7.72 (s, 1H), 7.58 (d, 2H), 4.72 (s, 2H), 3.08-2.96 (m, 1H), 2.04-1.78 (m, 6H), 1.77-1.57 (m, 2H), 1.42-1.11 (m, 2H).
LC-MS (Methode 12): Rₜ = 3.37 min; MS (ESIpos): m/z = 485 [M+H]⁺.

### Beispiel 38A

### 2-Amino-4-cyclohex-3-en-1-yl-6-mercaptopyridin-3,5-dicarbonitril

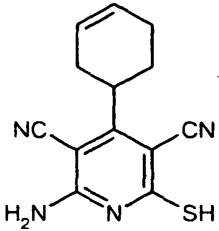

Die Titelverbindung ist analog zu Beispiel 33A durch Umsetzung von Cyclohex-3-en-1-carbaldehyd mit 2 Äquivalenten Cyanothioacetamid in Gegenwart von 4-Methylmorpholin erhältlich.
¹H-NMR (400 MHz, CDCl₃): δ = 12.91 (s, 1H), 8.09-7.56 (br. s, 2H), 5.82-5.71 (m, 2H), 3.14-3.02 (m, 1H), 2.61-2.52 (m, 1H), 2:29-2.03 (m, 4H), 1.78 (d, 1H).
LC-MS (Methode 5): Rₜ = 2.82 min; MS (ESIpos): m/z = 257 [M+H]⁺.

### Beispiel 39A

### 2-Amino-6-methyl-4-(tetrahydro-2H-pyran-2-yl)pyridin-3,5-dicarbonitril

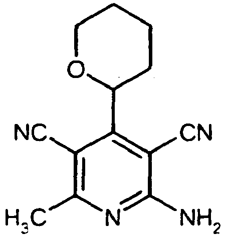

870 mg (7.62 mmol) Tetrahydro-2*H*-pyran-2-carbaldehyd, 504 mg (7.62 mmol) Malonsäuredinitril und 626 mg (7.62 mmol) 3-Aminocrotonsäurenitril werden in 8.4 ml trockenem Ethanol gelöst. Die Lösung wird auf 0°C gekühlt und portionsweise mit 412 mg (7.62 mmol) Natriummethylat versetzt. Das Reaktionsgemisch wird anschließend auf RT erwärmt und 10 min gerührt. Danach wird 10 h zum Rückfluss erhitzt. Nach dem Abkühlen wird der entstandene braune Niederschlag abgesaugt, dreimal mit insgesamt 100 ml Ethanol gewaschen und anschließend im Vakuum bei 50°C getrocknet. Das Produkt wird ohne weitere Aufreinigung in der Folgereaktion eingesetzt. Eine Reinigung kann mittels präparativer HPLC erfolgen (Säule: YMC GEL ODS-AQ S-5, 15 µm; Laufmittelgradient: Acetonitril/Wasser 10:90 → 95:5).
Ausbeute: 610 mg (20% d. Th., 61 % Reinheit)
LC-MS (Methode 7): Rₜ = 2.45 min; MS (ESIpos): m/z = 243 [M+H]⁺.

### Beispiel 40A

### 2-Chlor-6-methyl-4-(tetrahydro-2H-pyran-2-yl)pyridin-3,5-dicarbonitril

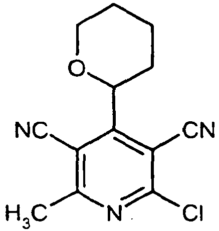

610 mg (2.52 mmol) der Verbindung aus Beispiel 39A werden in 44 ml Acetonitril vorgelegt und mit 590 mg (5.04 mmol) Isopentylnitrit sowie 677 mg (5.04 mmol) Kupfer(II)chlorid versetzt. Das Reaktionsgemisch wird 30 min bei 60°C gerührt. Der Ansatz wird danach mit 40 ml 1 N Salzsäure versetzt und das Gemisch dreimal mit je 100 ml Ethylacetat extrahiert. Die vereinigten organischen Phasen werden über Magnesiumsulfat getrocknet und das Lösungsmittel am Rotationsverdampfer entfernt. Das Produkt wird ohne weitere Reinigung in der Folgereaktion eingesetzt.
Ausbeute: 471 mg (53% d. Th., 75% Reinheit)
LC-MS (Methode 4): Rₜ = 2.50 min; MS (ESIpos): m/z = 262 [M+H]⁺.

### Beispiel 41 A

### 2-Methyl-6-sulfanyl-4-(tetrahydro-2H-pyran-2-yl)pyridin-3,5-dicarbonitril

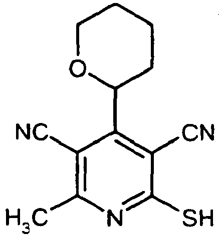

471 mg (1.35 mmol) der Verbindung aus Beispiel 40A werden in 4 ml trockenem DMF vorgelegt und mit 126 mg (1.62 mmol) Natriumsulfid versetzt. Das Reaktionsgemisch wird 12 h bei RT gerührt. Das Lösungsmittel wird anschließend am Rotationsverdampfer entfernt und der Rückstand an Kieselgel 60 chromatographisch gereinigt (Laufmittel: Gradient Dichlormethan/Ethanol 200:1 → 5:1).
Ausbeute: 232 mg (64% d. Th.)
LC-MS (Methode 4): Rₜ = 2.01 min; MS (ESIpos): m/z = 260 [M+H]⁺.

### Beispiel 42A

### 4-(Chlormethyl)-2-(4-chlorphenyl)-1,3-oxazol

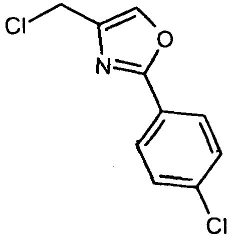

408 mg (3.21 mmol) 1,3-Dichloraceton und 500 mg (3.21 mmol) 4-Chlorbenzamid werden vereint und 1 h bei 135°C gerührt. Anschließend wird auf RT abgekühlt, vorsichtig mit 1.1 ml konz. Schwefelsäure versetzt und 5 min nachgerührt. Das Gemisch wird dann vorsichtig auf Eis gegossen. Der Niederschlag wird abgesaugt und mit Wasser nachgewaschen. Nach Trocknen wird das Rohprodukt ohne weitere Reinigung in der Folgereaktion eingesetzt.
Ausbeute: 426 mg (49% d. Th., 85% Reinheit)
LC-MS (Methode 14): Rₜ = 3.78 min; MS (ESIpos): m/z = 228 [M+H]⁺.

### Beispiel 43A

### rac-2-Chlor-6-({[2-(4-chlorphenyl)-1,3-oxazol-4-yl]methyl}sulfanyl)-4-(tetrahydro-2H-pyran-3-yl)pyridin-3,5-dicarbonitril

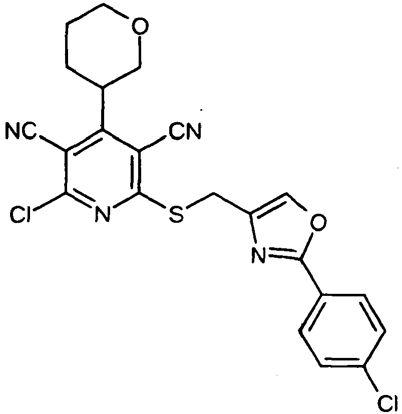

244 mg (2.08 mmol) Isopentylnitrit und 280 mg (2.08 mmol) Kupfer(II)chlorid werden in 26 ml Acetonitril vorgelegt und bei RT mit 470 mg (1.04 mmol) der Verbindung aus Beispiel 58 versetzt. Es wird 3 h bei 60°C gerührt. Nach Abkühlen auf RT wird die Reaktionslösung mit 20 ml 1 N Salzsäure versetzt. Es wird zweimal mit je 20 ml Ethylacetat extrahiert. Die organischen Phasen werden über Magnesiumsulfat getrocknet. Nach Entfernen des Lösungsmittels am Rotationsverdampfer wird der Rückstand in 20 ml Ethanol suspendiert und der Niederschlag abgesaugt. Der erhaltene Feststoff wird im Vakuum getrocknet. Das Rohprodukt wird ohne weitere Reinigung in der Folgereaktion eingesetzt. Eine Reinigung ist mittels präparativer HPLC möglich (Säule: YMC GEL ODS-AQ S-5, 15 µm; Laufmittelgradient: Acetonitril/Wasser 10:90 → 95:5).
Ausbeute: 239 mg (40% d. Th., 82% Reinheit)
LC-MS (Methode 2): Rₜ = 6.45 min; MS (ESIpos): m/z = 471 [M+H]⁺.

### Beispiel 44A

### 4-(Chlormethyl)-2-(4-fluor-3-methylphenyl)-1,3-oxazol

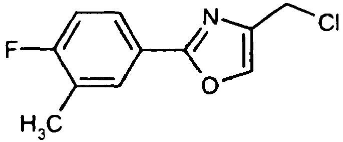

2.00 g (12.80 mmol) 4-Fluor-3-methylbenzamid und 1.79 g (14.08 mmol) 1,3-Dichloraceton werden 2 Tage lang bei 130°C gerührt, wobei sich eine Schmelze bildet. Der Ansatz wird danach auf RT abgekühlt, bei dieser Temperatur vorsichtig mit 3.0 ml konz. Schwefelsäure versetzt und 15 min lang gerührt. Die erhaltene Suspension wird auf 20 ml Eiswasser gegossen und über Nacht bei RT gerührt. Der entstandene Niederschlag wird abfiltriert und über Nacht bei 40°C im Vakuumtrockenschrank getrocknet.
Ausbeute: 2.05 g (64% d. Th., 90% Reinheit)
LC-MS (Methode 7): Rₜ = 2.05 min; MS (ESIpos): m/z = 226 [M+H]⁺.

Die weiteren zur Synthese von Ausführungsbeispielen eingesetzten 4-(Chlormethyl)-2-aryl-1,3-oxazole werden aus den entsprechenden kommerziell erhältlichen Edukten auf analoge Weise hergestellt.

### Beispiel 45A

### 2-(4-Chlorphenyl)-4,5-dimethyl-1,3-oxazol-3-oxid

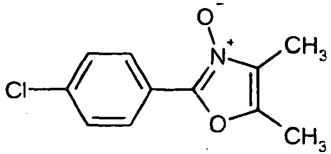

1.00 g (9.89 mmol) Diacetylmonoxim und 1.53 g (10.88 mmol) 4-Chlorbenzaldehyd werden in 2 ml (34.94 mmol) Eisessig vorgelegt. Dann wird 30 min lang Chlorwasserstoff-Gas unter Eiskühlung des Reaktionsgemisches eingeleitet. Anschließend wird das Reaktionsgemisch mit 10 ml Diethylether versetzt. Es fällt ein Niederschlag aus, der abgesaugt und zweimal mit je 2 ml Diethylether gewaschen wird. Der Niederschlag wird in ca. 5 ml Wasser re-suspendiert und die Suspension mit Ammoniak basisch gestellt. Es wird dann viermal mit je 10 ml Dichlormethan extrahiert. Die vereinigten organischen Phasen werden über Magnesiumsulfat getrocknet und das Lösungsmittel am Rotationsverdampfer entfernt. Der Rückstand wird ohne weitere Reinigung in der Folgereaktion eingesetzt.
Ausbeute: 1.85 g (84% d. Th.)
LC-MS (Methode 5): Rₜ = 2.29 min; MS (ESIpos): m/z = 224 [M+H]⁺.

### Beispiel 46A

### 4-(Chlormethyl)-2-(4-chlorphenyl)-5-methyl-1,3-oxazol

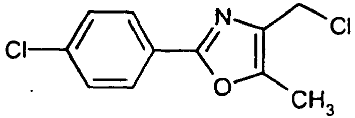

1.00 g (4.47 mmol) der Verbindung aus Beispiel 45A werden in 15 ml Chloroform vorgelegt und vorsichtig mit 1.5 ml (16.10 mmol) Phosphorylchlorid versetzt. Das Reaktionsgemisch wird 30 min unter Rühren zum Rückfluss erhitzt. Der Ansatz wird anschließend auf 0°C abgekühlt und durch Zugabe von Ammoniak schwach basisch gestellt. Das Gemisch wird dreimal mit je 20 ml Ethylacetat extrahiert. Die vereinigten organischen Phasen werden zweimal mit je 5 ml Wasser gewaschen und anschließend über Magnesiumsulfat getrocknet. Das Lösungsmittel wird am Rotationsverdampfer entfernt. Der Rückstand wird ohne weitere Reinigung in den Folgestufen eingesetzt.
Ausbeute: 1.33 g (96% d. Th., 78% Reinheit)
¹H-NMR (400 MHz, DMSO-d₆): δ = 7.95 (d, 2H), 7.60 (d, 2H), 4.77 (s, 2H), 2.44 (s, 3H).
LC-MS (Methode 3): Rₜ = 2.80 min; MS (ESIpos): m/z = 242 [M+H]⁺.

### Beispiel 47A

### Methyl-3-cyanobenzoat

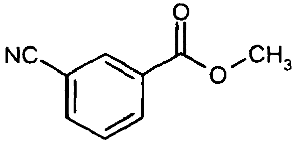

100 mg (0.68 mmol) 3-Cyanobenzoesäure werden in 4 ml Toluol und 3.5 ml Methanol vorgelegt und bei RT mit 0.51 ml (1.02 mnmol) einer 2 M Lösung von Trimethylsilyldiazomethan in n-Hexan versetzt. Das Reaktionsgemisch wird 1.5 h bei RT gerührt. Nach Entfernen des Lösungsmittels am Rotationsverdampfer wird der Rückstand im Vakuum getrocknet. Das Produkt wird rein erhalten und direkt weiter umgesetzt.
Ausbeute: 116 mg (100% d. Th.)
LC-MS (Methode 4): Rₜ = 1.93 min; MS (ESIpos): m/z = 162 [M+H]⁺.

### Beispiel 48A

### Methyl-3-(1H-tetrazol-5-yl)benzoat

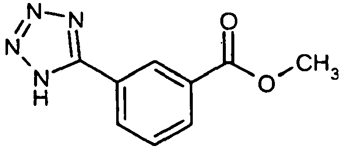

1.00 g (6.21 mmol) der Verbindung aus Beispiel 47A werden in 18 ml trockenem DMF vorgelegt. Anschließend werden 2.42 g (37.23 mmol) Natriumazid und 1.99 g (37.23 mmol) Ammoniumchlorid hinzugegeben. Das Reaktionsgemisch wird 2.5 h bei 120°C gerührt. Nach Abkühlen auf RT wird der Ansatz auf ein Gemisch von 30 ml Eiswasser und 10 ml Ethylacetat gegossen. Zu dieser Mischung werden 2.57 g (37.23 mmol) Natriumnitrit gegeben, um überschüssiges Azid zu vernichten. Anschließend wird durch Zugabe von 6 N Salzsäure der pH-Wert auf 1-2 gestellt und 30 min bei RT gerührt. Die Mischung wird dreimal mit je 20 ml eines 1:1-Gemisches von Ethylacetat und THF extrahiert. Der ausgefallene Niederschlag wird abgesaugt und im Vakuum getrocknet. Zur Isolierung weiteren Produkts wird die abgetrennte organische Phase je einmal mit je 10 ml Wasser und gesättigter wässriger Natriumchlorid-Lösung gewaschen. Nach Trocknen über Magnesiumsulfat wird das Lösungsmittel am Rotationsverdampfer entfernt. Beide Feststoffe werden vereint und in der Folgestufe eingesetzt.
Ausbeute: 1.18 g (91% d. Th.)
LC-MS (Methode 5): Rₜ = 1.87 min; MS (ESIpos): m/z = 205 [M+H]⁺.

### Beispiel 49A

### [3-(1H-Tetrazol-5-yl)phenyl]methanol

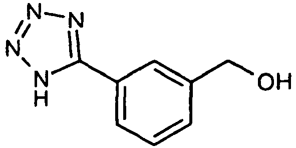

438 mg (11.56 mmol) Lithiumaluminiumhydrid werden in 60 ml trockenem THF vorgelegt, auf 0°C abgekühlt und mit einer Lösung von 1.18 g (5.78 mmol) der Verbindung aus Beispiel 48A in 40 ml trockenem THF versetzt. Man lässt die Reaktionsmischung auf RT kommen und rührt 2 h bei dieser Temperatur. Das Reaktionsgemisch wird dann vorsichtig bei 0°C mit 4 M Salzsäure versetzt, bis kein Wasserstoff mehr entwickelt wird. Die Lösung wird danach dreimal mit je 10 ml Ethylacetat extrahiert. Die vereinigten organischen Phasen werden je einmal mit je 10 ml Wasser und gesättigter wässriger Natriumchlorid-Lösung gewaschen und über Magnesiumsulfat getrocknet. Nach Entfernen des Lösungsmittels am Rotationsverdampfer wird der Rückstand im Vakuum getrocknet. Das Produkt wird ohne weitere Reinigung in der Folgereaktion eingesetzt.
Ausbeute: 0.80 g (77% d. Th.)
LC-MS (Methode 5): Rₜ = 0.90 min; MS (ESIpos): m/z = 177 [M+H]⁺.

### Beispiel 50A

### 3-(1H-Tetrazol-5-yl)benzylmethansulfonat

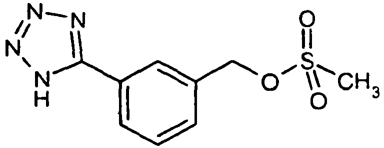

50 mg (0.28 mmol) der Verbindung aus Beispiel 49A werden in 5 ml trockenem Dichlormethan gelöst und mit 33 µl (0.43 mmol) Methansulfonsäurechlorid sowie 0.06 ml (0.43 mmol) Triethylamin versetzt. Das Reaktionsgemisch wird 10 h bei RT gerührt. Der Reaktionsansatz wird dann mit 10 ml Dichlormethan verdünnt und je einmal mit je 5 ml Wasser, 1 N Salzsäure und gesättigter wässriger Natriumchlorid-Lösung gewaschen. Die organische Phase wird über Magnesiumsulfat getrocknet. Nach Entfernen des Lösungsmittels am Rotationsverdampfer erhält man einen Feststoff, der ohne weitere Reinigung in der Folgereaktion eingesetzt wird.
Ausbeute: 65 mg (40% d. Th., 44% Reinheit)
LC-MS (Methode 4): Rₜ = 1.58 min; MS (ESIpos): m/z = 255 [M+H]⁺.

### Beispiel 51A

### Methyl-cis-4-hydroxycyclohexancarboxylat

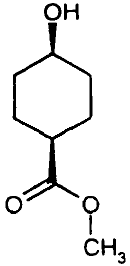

2.95 Liter Methanol und 9.7 ml konz. Schwefelsäure werden vorgelegt und bei RT portionsweise mit 175.0 g (1.21 mol) cis-4-Hydroxycyclohexancarbonsäure versetzt. Es wird 16 h bei RT gerührt. Das Lösungsmittel wird danach nahezu vollständig am Rotationsverdampfer entfernt und der Rückstand in 2 Liter eines 1:1-Gemisches von gesättigter wässriger Natriumhydrogencarbonat-Lösung und Ethylacetat aufgenommen. Die Phasen werden getrennt, und die organische Phase wird mit 1 Liter 10%-iger wässriger Ammoniumchlorid-Lösung gewaschen und anschließend über Magnesiumsulfat getrocknet. Nach Entfernen des Lösungsmittels am Rotationsverdampfer wird der Rückstand an Kieselgel 60 chromatographisch gereinigt (Laufmittel: Gradient Ethylacetat/ Petrolether 3:7 → 1:1).
Ausbeute: 128.4 g (60% d. Th., 89% Reinheit)
¹H-NMR (400 MHz, CDCl₃): δ = 3.89 (s, 1H), 3.68 (s, 3H), 2.44-2.35 (m, 1H), 2.07-1.90 (m, 2H), 1.90-1.58 (m, 8H).
GC-MS (Methode 8): Rₜ = 4.10 min; MS (ESIpos): m/z = 140 [M-H₂O]⁺.

### Beispiel 52A

### Methyl-cis-4-{[tert.-butyl(dimethyl)silyl]oxy}cyclohexancarboxylat

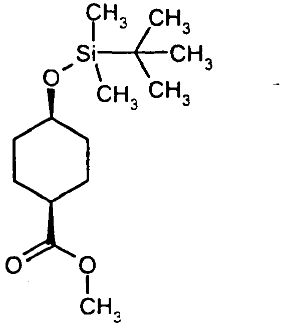

99.0 g (0.63 mol) der Verbindung aus Beispiel 51A werden in 2 Liter trockenem DMF gelöst und bei RT mit 132.0 g (0.88 mol) *tert*.-Butyldimethylsilylchlorid sowie 80.9 g (1.19 mol) Imidazol versetzt. Es wird 16 h bei RT gerührt. Das Lösungsmittel wird danach am Rotationsverdampfer fast bis zur Trockene entfernt und der Rückstand in 2 Liter eines 1:1-Gemisches von *tert*.-Butylmethylether und gesättigter wässriger Natriumhydrogencarbonat-Lösung aufgenommen. Die Phasen werden getrennt, die organische Phase über Magnesiumsulfat getrocknet und das Lösungsmittel am Rotationsverdampfer entfernt. Das Produkt wird ohne weitere Reinigung in der Folgereaktion eingesetzt.
Ausbeute: 160.3 g (94% d. Th.)
¹H-NMR (400 MHz, CDCl₃): δ = 3.89 (s, 1H), 3.65 (s, 3H), 2.35-2.25 (m, 1H), 1.99-1.88 (m, 2H), 1.70-1.55 (m, 4H), 1.55-1.41 (m, 2H), 0.88 (s, 9H), 0.04 (s, 6H).
GC-MS (Methode 8): Rₜ = 4.96 min; MS (ESIpos): m/z = 273 [M+H]⁺.

### Beispiel 53A

### (cis-4-{[tert.-Butyl(dimethyl)silyl]oxy}cyclohexyl)methanol

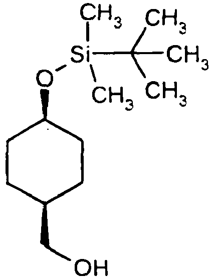

2.8 g (73.96 mmol) Lithiumaluminiumhydrid werden in 250 ml *tert*.-Butylmethylether vorgelegt und bei RT tropfenweise mit einer Lösung von 22.9 g (84.05 mmol) der Verbindung aus Beispiel 52A in 250 ml *tert*.-Butylmethylether versetzt. Es wird 16 h bei 40°C gerührt. Anschließend werden weitere 0.7 g (18.44 mmol) Lithiumaluminiumhydrid hinzugegeben und das Reaktionsgemisch 10 h zum Rückfluss erhitzt. Nach Abkühlen auf RT wird vorsichtig mit 20 ml Wasser versetzt. Anschließend werden 20 ml einer 15%-igen Lösung von Kaliumhydroxid in Wasser zugegeben. Die Phasen werden getrennt, die organische Phase über Natriumsulfat getrocknet und das Lösungsmittel am Rotationsverdampfer entfernt. Das erhaltene Produkt wird ohne weitere Reinigung in der Folgereaktion eingesetzt.
Ausbeute: 21.1 g (100% d. Th.)
¹H-NMR (400 MHz, CDCl₃): δ = 3.96-3.91 (br. s, 1H), 3.48-3.40 (br. s, 2H), 1.68-1.58 (m, 2H), 1.53-1.35 (m, 8H), 0.87 (s, 9H), 0.01 (s, 6H).
GC-MS (Methode 8): Rₜ = 4.77 min; MS (ESIpos): m/z = 245 [M+H]⁺.

### Beispiel 54A

### cis-4-{[tert.-Butyl(dimethyl)silyl]oxy} cyclohexancarbaldehyd

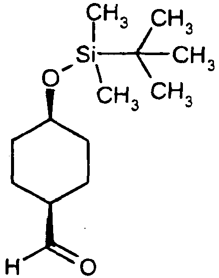

16.4 g (129.47 mmol) Oxalsäuredichlorid werden in 600 ml Dichlormethan gelöst und auf -78°C gekühlt. Dann werden langsam 20.2 g (258.95 mmol) Dimethylsulfoxid zugetropft. Es wird 5 min nachgerührt. Nach Zugabe einer Lösung von 21.1 g (86.32 mmol) der Verbindung aus Beispiel 53A in 200 ml Dichlormethan wird 1 h bei -78°C gerührt. Dann werden langsam 60 ml (431.58 mmol) Triethylamin zugetropft. Anschließend wird innerhalb einer Stunde auf RT erwärmt und das Reaktionsgemisch danach mit 500 ml gesättigter wässriger Natriumhydrogencarbonat-Lösung versetzt. Die Phasen werden getrennt, die organische Phase über Natriumsulfat getrocknet und das Lösungsmittel am Rotationsverdampfer entfernt. Der Rückstand wird an Kieselgel 60 chromatographisch gereinigt (Laufmittel: Gradient Petrolether → Petrolether/Ethylacetat 9:1).
Ausbeute: 15.6 g (75% d. Th.)
¹H-NMR (400 MHz, CDCl₃): δ = 3.90-3.82 (br. s, 1H), 2.22-2.12 (m, 1H), 1.91-1.77 (m, 2H), 1.67-1.47 (m, 6H), 0.85 (s, 9H), 0.01 (s, 6H).

### Beispiel 55A

### 2-Amino-4-(cis-4-hydroxycyclohexyl)-6-sulfanylpyridin-3,5-dicarbonitril

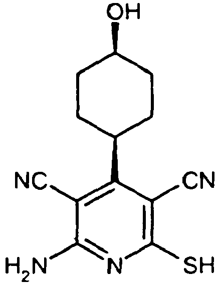

15.6 g (64.35 mmol) der Verbindung aus Beispiel 54A und 13.53 g (135.13 mmol) Cyanothioacetamid werden in 450 ml Ethanol vorgelegt und mit 9.86 g (135.13 mmol) 4-Methylmorpholin versetzt. Es wird 4 h zum Rückfluss erhitzt und anschließend 16 h bei RT nachgerührt. Es fällt ein Niederschlag aus, der abgesaugt wird (und der *tert*.-Butyldimethylsilyl-geschützten Zielverbindung entspricht). Das Filtrat wird am Rotationsverdampfer vom Lösungsmittel befreit und der Rückstand mittels präparativer HPLC (Methode 20) aufgereinigt.
Ausbeute: 2.55 g (14% d. Th.)
¹H-NMR (400 MHz, DMSO-d₆): δ = 12.95-12.83 (br. s, 1H), 8.06-7.52 (br. s, 2H), 4.88-4.46 (br. s, 1H), 3.46-3.22 (m, 1H), 2.84-2.73 (m, 1H), 2.13-1.92 (m, 4H), 1.77-1.67 (m, 3H), 1.28-1.12 (m, 3H).
LC-MS (Methode 21): Rₜ = 0.53 min; MS (ESIpos): m/z = 275 [M+H]⁺.

### Ausführungsbeispiele:

### Beispiel 1

### 2-Amino-6-({[2-(4-chlorphenyl)-1,3-thiazol-4-yl]methyl}thio)-4-[trans-4-(2-hydroxyethoxy)cyclohexyl]pyridin-3,5-dicarbonitril

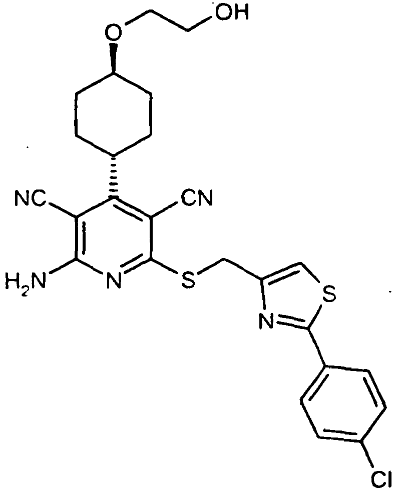

35 mg (0.05 mmol) der Verbindung aus Beispiel 7A (*trans*-Isomer) werden in 5 ml trockenem THF gelöst, mit 18 mg (0.07 mmol) Tetra-*n*-butylammoniumfluorid versetzt und 20 h bei RT gerührt. Der Ansatz wird dann mit 15 ml Ethylacetat versetzt. Es wird zweimal mit je 3 ml ges. wässriger Natriumhydrogencarbonat-Lösung gewaschen. Die organische Phase wird über Magnesiumsulfat getrocknet. Nach Entfernen des Lösungsmittels am Rotationsverdampfer wird das Rohprodukt über präparative HPLC gereinigt (Säule: YMC GEL ODS-AQ S-5 / 15 µm; Laufmittelgradient: Acetonitril/Wasser 10:90 → 95:5).
Ausbeute: 19 mg (79% d. Th.)
¹H-NMR (400 MHz, DMSO-d₆): δ = 8.32-7.77 (br. s, 2H), 7.92 (d, 2H), 7.88 (s, 1H), 7.56 (d, 2H), 4.61-4.51 (m, 1H), 4.58 (s, 2H), 3.51-3.42 (m, 4H), 3.30-3.20 (m, 1H), 2.89-2.78 (m, 1H), 2.19-2.09 (m, 2H), 2.08-1.93 (m, 2H), 1.81-1.71 (m, 2H), 1.26-1.12 (m, 2H).
LC-MS (Methode 5): Rₜ = 3.86 min; MS (ESIpos): m/z = 526 [M+H]⁺.

### Beispiel 2

### 2-Amino-6-({[2-(4-chiorphenyl)-1,3-thiazol-4-yl]methyl}thio)-4-[cis-4-(2-hydroxyethoxy)cyc!o-hexyl]pyridin-3,5-dicarbonitril

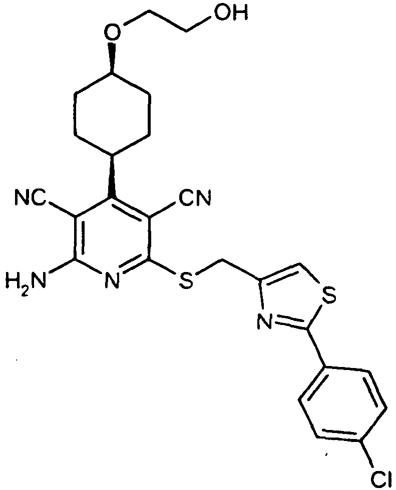

43 mg (0.06 mmol) der Verbindung aus Beispiel 7A (cis-Isomer) werden in 5 ml trockenem THF gelöst, mit 22 mg (0.08 mmol) Tetra-n-butylammoniumfluorid versetzt und 20 h bei RT gerührt. Der Ansatz wird dann mit 15 ml Ethylacetat versetzt. Es wird zweimal mit je 3 ml ges. wässriger Natriumhydrogencarbonat-Lösung gewaschen. Die organische Phase wird über Magnesiumsulfat getrocknet. Nach Entfernen des Lösungsmittels am Rotationsverdampfer wird das Rohprodukt zunächst über präparative HPLC (Säule: YMC GEL ODS-AQ S-5 / 15 µm; Laufmittelgradient: Acetonitril/Wasser 10:90 → 95:5) und dann nochmals an Kieselgel 60 (Laufmittel: Gradient Dichlormethan/Ethanol 200:1 → 10:1) chromatographisch gereinigt.
Ausbeute: 21 mg (68% d. Th.)
¹H-NMR (400 MHz, DMSO-d₆): δ = 8.18-7.82 (br. s, 2H), 7.93 (d, 2H), 7.88 (s, 1H), 7.57 (d, 2H), 4.58 (s, 2H), 4.31 (t, 1H), 3.61 (s, 1H), 3.56-3.48 (m, 2H), 3.43-3.36 (m, 2H), 2.95-2.84 (m, 1H), 2.36-2.20 (m, 2H), 2.03-1.92 (m, 2H), 1.49-1.37 (m, 4H).
LC-MS (Methode 7): Rₜ = 3.89 min; MS (ESIpos): m/z = 526 [M+H]⁺.

### Beispiel 3

### 2-Anuno-6-({[2-(4-chlorphenyl)-1,3-thiazol-4-yl]methyl}thio)-4-(trans-4-hydroxycyclohexyl)-pyridin-3,5-dicarbonitril

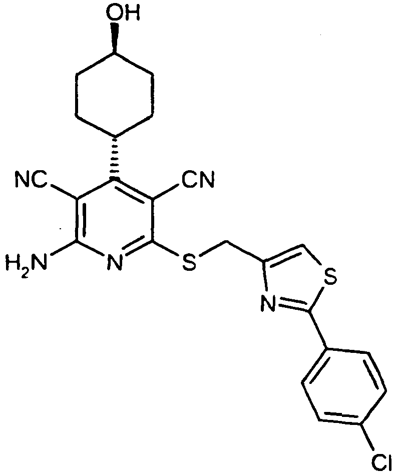

25 mg (0.04 mmol) der Verbindung aus Beispiel 13A (trans-Isomer) werden in 1 ml trockenem Acetonitril gelöst und mit 0.1 ml (2.30 mmol) 40%-iger Fluorwasserstoffsäure versetzt. Das Reaktionsgemisch wird 2 h bei RT gerührt. Der Ansatz wird danach direkt über präparative HPLC gereinigt (Säule: YMC GEL ODS-AQ S-5 / 15 µm; Laufmittelgradient: Acetonitril/Wasser 10:90 → 95:5).
Ausbeute: 20 mg (99% d. Th.)
¹H-NMR (400 MHz, DMSO-d₆): δ = 8.21-7.81 (br. s, 2H), 7.94 (d, 2H), 7.88 (s, 1H), 7.56 (d, 2H), 4.70 (d, 1H), 4.58 (s, 2H), 3.48-3.37 (m, 1H), 2.85-2.75 (m, 1H), 2.06-1.92 (m, 4H), 1.77-1.68 (m, 2H), 1.28-1.14 (m, 2H).
LC-MS (Methode 7): R, = 3.65 min; MS (ESIpos): m/z = 482 [M+H]⁺.

Die in der folgenden Tabelle aufgeführten Verbindungen werden analog zu Beispiel 3 aus den entsprechenden Ausgangsverbindungen hergestellt:

| **Beispiel Nr.** | **Struktur (Ausbeute)** | **LC-MS: R, [min] (Methode); MS (ESI): m/z [M+H]⁺** | **¹H-NMR (DMSO-d₆): δ =** |
|---|---|---|---|
| **4** | 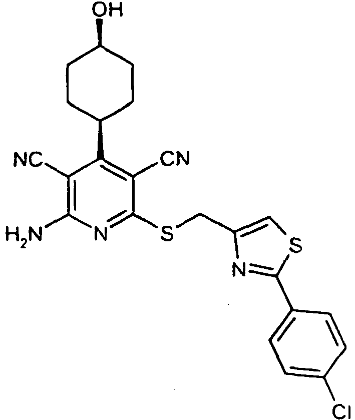 | 3.75 min (7); m/z = 482 | 8.21-7.80 (br. s, 2H), 7.92 (d, 2H), 7.88 (s, 1H), 7.56 (d, 2H), 4.59 (s, 2H), 4.38 (d, 1 H), 3.90 (br. s, 1H), 2.89-2.79 (m, 1H), 2.45-2.31 (m, 2H), 1.83-1.73 (m, 2H), 1.52-1.35 (m, 4H). |
| | (88% d. Th.) | | |
| **5** | 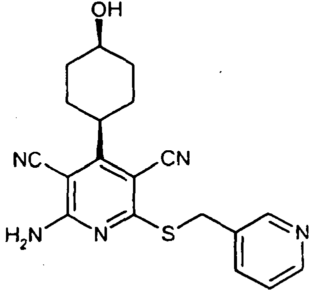 | 1.49 min (4); m/z = 366 | 8.74 (s, 1H), 8.43 (d, 1H), 8.27-7.70 (br. s, 2H), 7.92 (d, 1H), 7.37-7.29 (m, 1H), 4.45 (s, 2H), 4.38 (br. s, 1H), 3.89 (br. s, 1H), 2.99-2.78 (m, 1H), 2.45-2.29 (m, 2H), 1.83-1.72 (m, 2H), 1.51-1.36 (m, 4H). |
| | (33% d. Th.) | | |
| **6** | 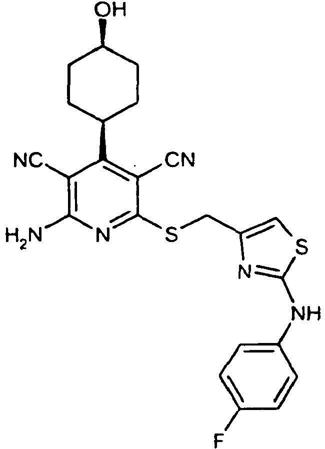 | 2.40 min (13); m/z = 481 | 10.21 (s, 1H), 8.16-7.75 (br. s, 2H), 7.60 (dd, 2H), 7.12 (pseudo-t, 2H), 6.93 (s, 1H), 4.40 (s, 2H), 4.38 (d, 1H), 3.90 (br. s, 1 H), 2.90-2.80 (m, 1H), 2.47-2.31 (m, 2H), 1.83-1.73 (m, 2H), 1.51-1.38 (m, 4H). |
| | (96% d. Th.) | | |

### Beispiel 7

### 2-Amino-6-[({2-[(4-fluorphenyl)amino]-1,3-thiazol-4-yl}methyl)thio]-4-piperidin-4-ylpyridin-3,5-dicarbonitril

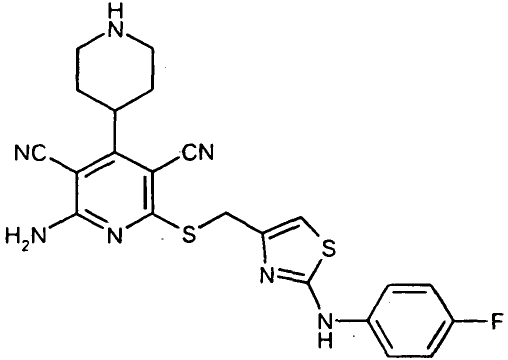

40 mg (0.07 mmol) der Verbindung aus Beispiel 18A werden in 0.9 ml Dioxan gelöst und mit 0.9 ml einer 4 M Lösung von Chlorwasserstoff in Dioxan versetzt. Das Reaktionsgemisch wird 2 h bei RT gerührt und anschließend direkt über präparative HPLC gereinigt (Säule: YMC GEL ODS-AQ S-5, 15 µm; Laufmittelgradient: Acetonitril/Wasser 10:90 → 95:5).
Ausbeute: 17 mg (52% d. Th.)
¹H-NMR (500 MHz, DMSO-d₆): δ = 10.27 (s, 1H), 8.20-7.72 (br. s, 2H), 7.61 (dd, 2H), 7.12 (dd, 2H), 6.93 (s, 1H), 4.40 (s, 2H), 3.77-3.66 (m, 1H), 3.53-3.37 (m, 2H), 3.04 (d, 2H), 2.96-2.87 (m, 1H), 2.10-1.91 (m, 2H), 1.54 (d, 2H).
LC-MS (Methode 12): Rₜ = 1.52 min; MS (ESIpos): m/z = 466 [M+H]⁺.

### Beispiel 8

### 2-Amino-6-({[2-(4-chlorphenyl)-1,3-thiazol-4-yl]methyl}thio)-4-piperidin-4-ylpyridin-3,5-dicarbonitril

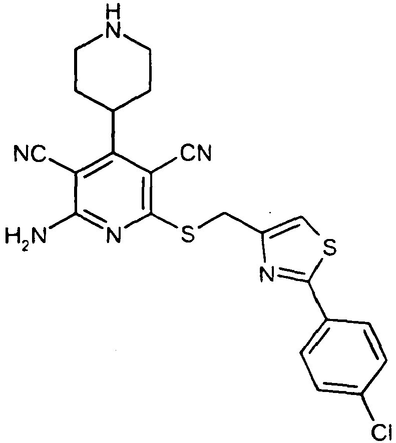

Die Titelverbindung wird auf analoge Weise ausgehend von Beispiel 19A erhalten.
Ausbeute: 57% d. Th.
LC-MS (Methode 12): Rₜ = 1.74 min; MS (ESIpos): m/z = 467 [M+H]⁺.

### Beispiel 9

### 2-Amino-6-({[2-(4-chlorphenyl)-1,3-thiazol-4-yl]methyl}thio)-4-(1-glycoloylpiperidin-4-yl)-pyridin-3,5-dicarbonitril

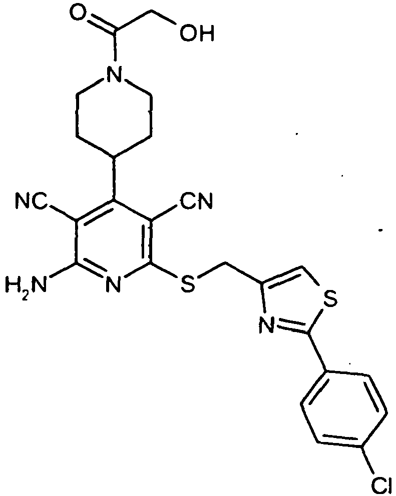

29 mg (0.05 mmol) der Verbindung aus Beispiel 25A werden in einem Gemisch aus 1.5 ml Dioxan und 0.8 ml Wasser gelöst und mit 2.4 mg (0.10 mmol) Lithiumhydroxid versetzt. Das Reaktionsgemisch wird zwei Stunden bei RT gerührt. Nach Entfernen des Lösungsmittels am Rotationsverdampfer wird der Rückstand über präparative HPLC gereinigt (Säule: YMC GEL ODS-AQ S-5, 15 µm; Laufmittelgradient: Acelonitril/Wasser 10:90 → 95:5).
Ausbeute: 13 mg (47% d. Th.)
¹H-NMR (400 MHz, DMSO-d₆): δ = 8.29-7.83 (br. s, 2H), 7.94 (d, 2H), 7.89 (s, 1H), 7.57 (d, 2H), 4.61-4.47 (m, 2H), 4.48 (s, 2H), 4.17-4.03 (m, 2H), 3.89-3.78 (m, 1H), 3.19-2.97 (m, 2H), 2.72-2.61 (m, 1H), 2.13-1.90 (m, 2H), 1.80-1.69 (m, 2H).
LC-MS (Methode 3): Rₜ = 2.47 min; MS (ESIpos): m/z = 525 [M+H]⁺.

### Beispiel 10

### 3-{4-[2-Amino-6-({[2-(4-chlorphenyl)-1,3-thiazol-4-yl]methyl}thio)-3,5-dicyanopyridin-4-yl]-piperidin-1-yl}propyl-acetat

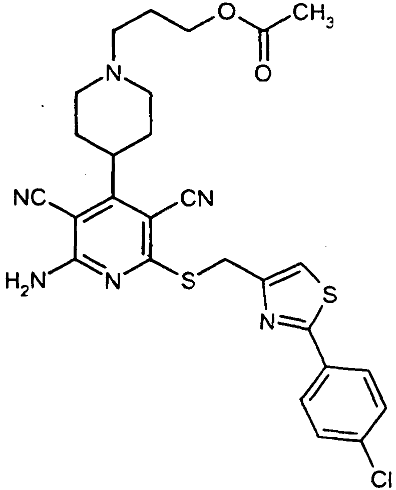

60 mg (0.16 mmol) der Verbindung aus Beispiel 29A werden in 3 ml trockenem DMF gelöst und nacheinander mit 46 mg (0.19 mmol) 4-(Chlormethyl)-2-(4-chlorphenyl)-1,3-thiazol und 53 mg (0.63 mmol) Natriumhydrogencarbonat versetzt. Das Reaktionsgemisch wird 8 h bei RT gerührt. Nach Entfernen des Lösungsmittels am Rotationsverdampfer wird der Rückstand direkt über präparative HPLC gereinigt (Säule: YMC GEL ODS-AQ S-5, 15 µm; Laufmittelgradient: Acetonitril/Wasser 10:90 → 95:5).
Ausbeute: 65 mg (72% d. Th.)
LC-MS (Methode 3): R, = 1.77 min; MS (ESIpos): m/z = 568 [M+H]⁺.

Die in der folgenden Tabelle aufgeführten Verbindungen werden analog zu Beispiel 10 aus den entsprechenden Ausgangsverbindungen hergestellt:

| **Beispiel Nr.** | **Struktur (Ausbeute)** | **LC-MS: R, [min] (Methode); MS (ESI): m/z [M+H]⁺** | **¹H-NMR (DMSO-d₆): δ =** |
|---|---|---|---|
| **11** | 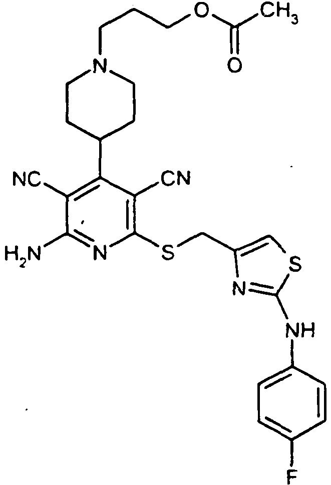 | 1.71 min (4); m/z = 566 | 10.21 (s, 1H), 8.20-7.87 (br. s, 2H), 7.59 (dd, 2H), 7.12 (pseudo-t, 2H), 6.92 (s, 1H), 4.41 (s, 2H), 4.02 (t, 2H), 3.06-2.97 (m, 2H), 2.88-2.78 (m, 1 H), 2.42-2.30 (m, 2H), 2.24-2.11 (m, 2H), 2.01 (s, 3H), 1.98-1.87 (m, 2H), 1.80-1.69 (m, 2H), 1.69-1.60 (m, 2H). |
| | (12% d. Th.) | | |
| **12** | 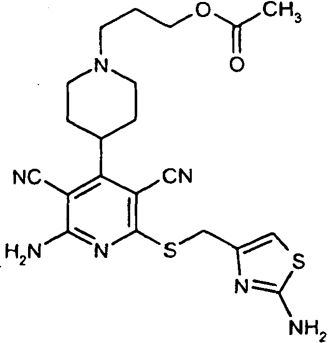 | 2.27 min (14); m/z=472 | |
| | (54% d. Th.) | | |

### Beispiel 13

### 2-Amino-6-({[2-(4-chlorphenyl)-1,3-thiazol-4-yl]methyl}thio)-4-[1-(3-hydroxypropyl)piperidin-4-yl]pyridin-3,5-dicarbonitril

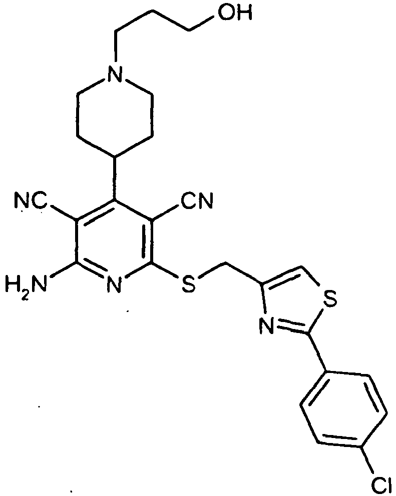

65 mg (0.12 mmol) der Verbindung aus Beispiel 10 werden in einem Gemisch aus 3.5 ml Dioxan und 1.7 ml Wasser vorgelegt und mit 11 mg Lithiumhydroxid versetzt. Es wird zunächst 8 h bei RT gerührt. Danach werden nochmals 22 mg Lithiumhydroxid zugegeben und die Mischung für weitere 16 h bei RT gerührt. Das Lösungsmittel wird dann am Rotationsverdampfer entfernt. Der Rückstand wird in 10 ml Ethylacetat aufgenommen und einmal mit 3 ml ges. wässriger Natriumhydrogencarbonat-Lösung gewaschen. Es wird über Magnesiumsulfat getrocknet. Nach Entfernen des Lösungsmittels am Rotationsverdampfer wird der Rückstand an Kieselgel 60 chromatographisch gereinigt (Laufmittel: Gradient Dichlormethan/Ethano) 50:1 → 10:1).
Ausbeute: 12 mg (19% d. Th.)
¹H-NMR (400 MHz, DMSO-d₆): δ = 8.25-7.77 (br. s, 2H), 7.94 (d, 2H), 7.87 (s, 1H), 7.56 (d, 2H), 4.59 (s, 2H), 4.41 (br. s, 1H), 3.42 (br. s, 2H), 3.00 (d, 2H), 2.88-2.77 (m, 1H), 2.40-2.29 (m, 2H), 2.24-2.10 (m, 2H), 1.97-1.85 (m, 2H), 1.70-1.52 (m, 4H).
LC-MS (Methode 5): Rₜ = 2.67 min; MS (ESIpos): m/z = 525 [M+H]⁺.

### Beispiel 14

### rac-2-Amino-6-({[2-(4-chlorphenyl)-1,3-thiazol-4-yl]methyl}thio)-4-(tetrahydro-2H-pyran-2-yl)-pyridin-3,5-dicarbonitril

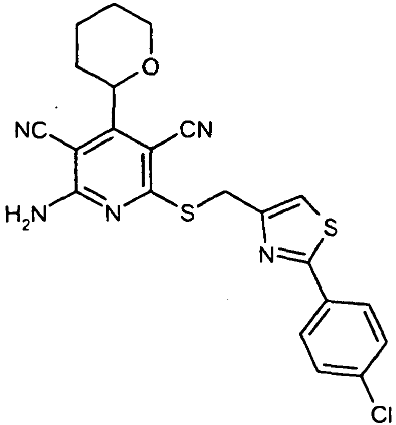

33 mg (0.10 mmol) der Verbindung aus Beispiel 31A, 29 mg (0.12 mmol) 4-(Chlormethyl)-2-(4-chlorphenyl)-1,3-thiazol und 33 mg (0.39 mmol) Natriumhydrogencarbonat werden in 2 ml trockenem DMF suspendiert. Das Reaktionsgemisch wird 20 h bei RT gerührt. Nach Filtration des Ansatzes wird direkt über präparative HPLC gereinigt (Säule: YMC GEL ODS-AQ S-5, 15 µm; Laufmittelgradient: Acetonitril/Wasser 10:90 → 95:5).
Ausbeute: 30 mg (66% d. Th.)
¹H-NMR (400 MHz, DMSO-d₆): δ = 8.27-7.82 (br. s, 2H), 7.93 (d, 2H), 7.87 (s, 1H), 7.55 (d, 2H), 4.59 (s, 2H), 4.57-4.50 (m, 1H), 4.01 (d, 1H), 3.55-3.47 (m, 1H), 1.97-1.83 (m, 1H), 1.74-1.48 (m, 5H).
LC-MS (Methode 4): Rₜ = 3.15 min; MS (ESIpos): m/z = 468 [M+H]⁺.

### Beispiel 15 und Beispiel 16

### ent-2-Amino-6-({[2-(4-chlorphenyl)-1,3-thiazol-4-yl]methyl}thio)-4-(tetrahydro-2H-pyran-2-yl)-pyridin-3,5-dicarbonitril (Enantiomer 1 und Enantiomer 2)

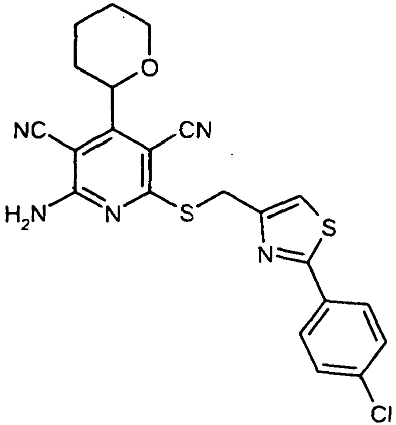

20 mg der Verbindung aus Beispiel 14 werden in 0.5 ml Methanol sowie 4.5 ml *tert*.-Butylmethylether gelöst und mittels präparativer HPLC an chiraler Phase (Methode 10) in die Enantiomere getrennt:

### Beispiel 15 (Enantiomer 1):

Ausbeute: 8 mg
HPLC (Methode 10): Rₜ = 8.13 min; ee >98%.

### Beispiel 16 (Enantiomer 2):

Ausbeute: 9 mg
HPLC (Methode 10): Rₜ = 8.62 min; ee >98%.

Die in der folgenden Tabelle aufgeführten Verbindungen werden analog zu Beispiel 14 aus den entsprechenden Ausgangsverbindungen in racemischer Form hergestellt:

| **Beispiel Nr.** | **Struktur (Ausbeute)** | **LC-MS: Rₜ [min] (Methode); MS (ESI): m/z [M+H]⁺** | **¹H-NMR (DMSO-d₆): δ =** |
|---|---|---|---|
| **17** | 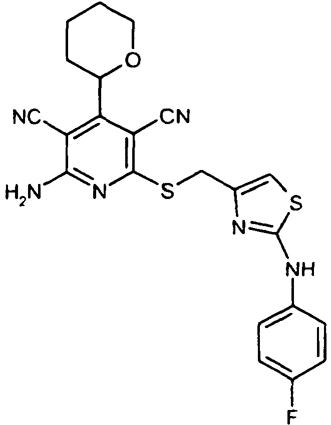 | 1.71 min (4); m/z = 467 | 10.20 (s, 1 H), 8.19-7.83 (br. s, 2H), 7.58 (dd, 2H), 7.11 (pseudo-t, 2H), 6.91 (s, 1 H), 4.59-4.52 (m, 1H), 4.40 (s, 2H), 4.03 (d, 1H), 3.56-3.47 (m, 1H), 1.96-1.87 (m, 1H), 1.75-3.50 (m, 5H). |
| | (39% d. Th.) | | |
| **18** | 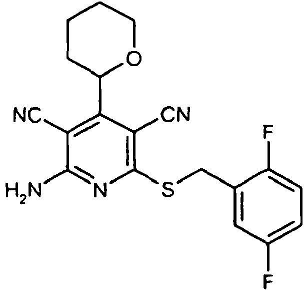 | 2.48 min (12); m/z = 387 | 8.36-7.74 (br. s, 2H), 7.65-7.59 (m, 1H), 7.31-7.22 (m, 1H), 7.21-7.12 (m, 1H), 4.57-4.50 (m, 1H), 4.45 (s, 2H), 4.02 (s, 1H), 3.54-3.43 (m, 1H), 1.96-1.84 (m, 1H), 1.75-1.50 (m, 5H). |
| | (45% d. Th.) | | |
| **19** | 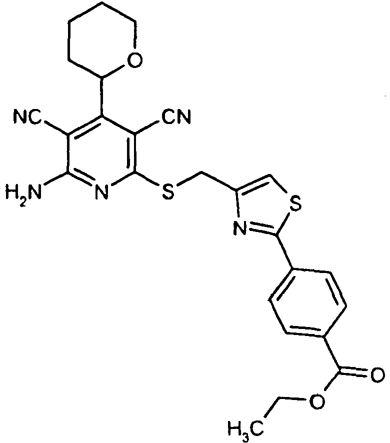 | 4.22 min (5); m/z = 506 | 8.30-7.89 (br. s, 2H), 8.03 (s, 4H), 7.96 (s, 1 H), 4.62 (s, 2H), 4.57-4.50 (m, 1H), 4.34 (q, 2H), 4.02 (d, 1 H), 3.54-3.46 (m, 1H), 1.93-1.87 (m, 1H), 1.73-1.51 (m, 5H), 1.33 (t, 3H). |
| | (49% d. Th.) | | |

### Beispiel 20 und Beispiel 21

### ent-Ethyl 4-[4-({[6-amino-3,5-dicyano-4-(tetrahydro-2H-pyran-2-yl)pyridin-2-yl]thio}methyl)-1,3-thiazol-2-yl]benzoat (Enantiomer 1 und Enantiomer 2)

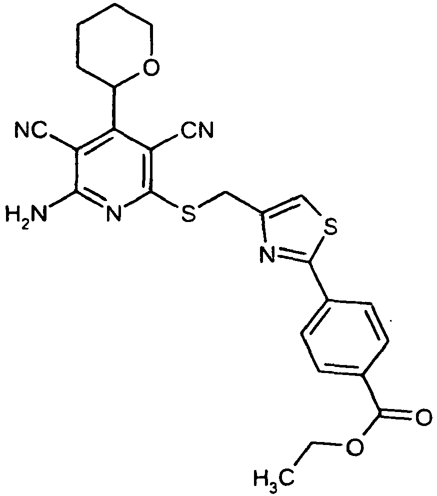

500 mg der Verbindung aus Beispiel 19 werden bei ca. 40°C in 35 ml 2-Propanol gelöst und mittels präparativer HPLC an chiraler Phase (Methode 11) in die Enantiomere getrennt:

### Beispiel 20 (Enantiomer 1):

Ausbeute: 231 mg
HPLC (Methode 11): Rₜ = 9.70 min; ee >99%
optische Drehung: -0.059° (c = 0.45 g / 100 ml, Chloroform).

### Beispiel 21 (Enantiomer 2):

Ausbeute: 209 mg
HPLC (Methode 11): Rₜ = 11.74 min; ee >98%
optische Drehung: +0.054° (c = 0.49 g / 100 ml, Chloroform).

### Beispiel 22

### rac-4-(4-({[6-Amino-3,5-dicyano-4-(tetrahydro-2H-pyran-2-yl)pyridin-2-yl]thio}methyl)-1,3-thiazol-2-yl]benzoesäure

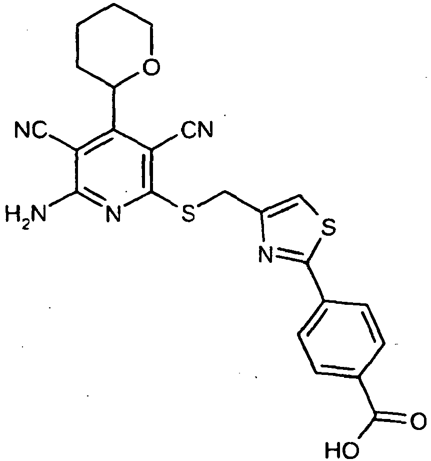

50 mg (0.10 mmol) der Verbindung aus Beispiel 19 und 16 mg (0.40 mmol) Natriumhydroxid werden in einer Mischung aus 4 ml 1,2-Dimethoxyethan, 1 ml Ethanol und 4 ml Wasser gelöst. Das Reaktionsgemisch wird 3 h bei RT gerührt. Nach Entfernen des Lösungsmittels wird direkt über präparative HPLC gereinigt (Säule: YMC GEL ODS-AQ S-5, 15 µm; Laufmittelgradient: Acetonitril/Wasser 10:90 → 95:5 unter Zusatz von 0.1% Salzsäure).
Ausbeute: 29 mg (61 % d. Th.)
¹H-NMR (400 MHz, DMSO-d₆): δ = 8.26-7.85 (br. s, 2H), 7.91 (d, 2H), 7.79 (s, 1H), 7.78 (d, 2H), 4.60 (s, 2H), 4.58-4.51 (m, 1H), 4.03 (d, 1H), 3.55-3.46 (m, 1H), 1.96-1.84 (m, 1H), 1.76-1.50 (m, 5H).
LC-MS (Methode 5): Rₜ = 3.53 min; MS (ESIpos): m/z = 478 [M+H]⁺.

Die in der folgenden Tabelle aufgeführten Enantiomere werden auf analoge Weise aus Beispiel 20 bzw. Beispiel 21 erhalten:

| **Beispiel Nr.** | **Struktur (Ausbeute)** | **LC-MS: Rₜ [min] (Methode); MS (ESI): m/z [M+H]⁺; optische Drehung** | **¹H-NMR (DMSO-d₆):** δ = |
|---|---|---|---|
| **23 (*ent*-1)** | 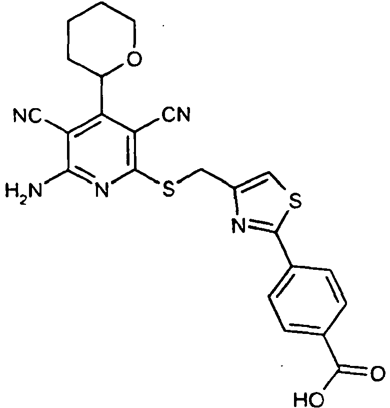 | 3.53 min (5); m/z = 478; +0.025° (c = 0.465 g / 100 ml, Methanol) | 13.18 (s, 1H), 8.31 -7.85 (br. s, 2H), 8.04 (s, 4H), 7.94 (s, 1H), 4.61 (s, 2H), 4.57-4.51 (m, 1H), 4.03 (dd, 1H), 3.55-3.46 (m, 1H), 1.95-1.86 (m, 1H), 1.74-1.50 (m, 5H). |
| | (69% d. Th.) | | |
| **24 (*ent*-2)** | 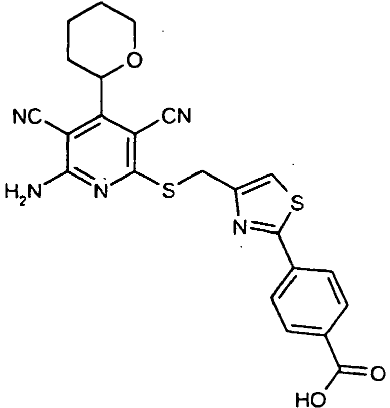 | 3.53 min (5); m/z = 478; -0.032° (c = 0.465 g / 100 ml, Methanol) | |
| | (59% d. Th.) | | |

### Beispiel 25

### rac-2-({[2-(4-Chlorphenyl)-1,3-thiazol-4-yl]methyl}thio)-4-(tetrahydro-2H-pyran-2-yl)pyridin-3,5-dicarbonitril

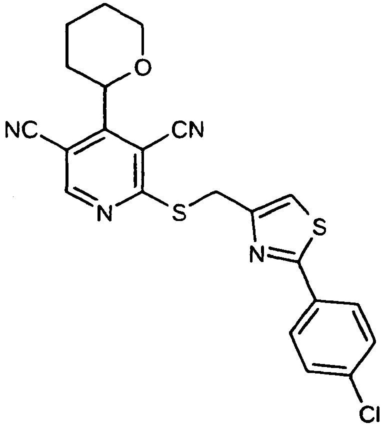

100 mg (0.21 mmol) der Verbindung aus Beispiel 14 werden in 5 ml trockenem THF vorgelegt und mit 167 mg (1.42 mmol) Isopentylnitrit und 3 mg (0.02 mmol) Kupfer(II)-chlorid versetzt. Das Reaktionsgemisch wird 8 h bei RT gerührt. Nach erneuter Zugabe von 3 mg (0.02 mmol) Kupfer(II)-chlorid wird das Reaktionsgemisch für weitere 12 h bei RT gerührt. Der Ansatz wird dann mit 6 ml 1 N Salzsäure versetzt. Die wässrige Phase wird zweimal mit je 10 ml Ethylacetat extrahiert. Die vereinigten organischen Phasen werden einmal mit 5 ml ges. wässriger Natriumhydrogencarbonat-Lösung und einmal mit 5 ml ges. wässriger Natriumchlorid-Lösung gewaschen. Anschließend wird über Magnesiumsulfat getrocknet. Nach Entfernen des Lösungsmittels wird der Rückstand über präparative HPLC gereinigt (Säule: YMC GEL ODS-AQ S-5, 15 µm; Laufmittelgradient: Acetonitril/Wasser 10:90 → 95:5).
Ausbeute: 43 mg (44% d. Th.)
¹H-NMR (400 MHz, DMSO-d₆): δ = 13.19 (s, 1H), 9.12 (s, 1H), 8.04 (s, 4H), 7.81 (s, 1H), 4.79-4.70 (m, 1H), 4.68 (s, 2H), 4.08 (dd, 1 H), 3.63-3.53 (m, 1H), 1.99-1.79 (m, 1H), 1.87-1.74 (m, 1 H), 1.73-1.52 (m, 4H).
LC-MS (Methode 4): Rₜ = 2.69 min; MS (ESIpos): m/z = 463 [M+H]⁺.

### Beispiel 26

### rac-2-Amino-6-({[2-(4-chlorphenyl)-1,3-thiazol-4-yl]methyl}thio)-4-(tetrahydro-2H-pyran-3-yl)-pyridin-3,5-dicarbonitril

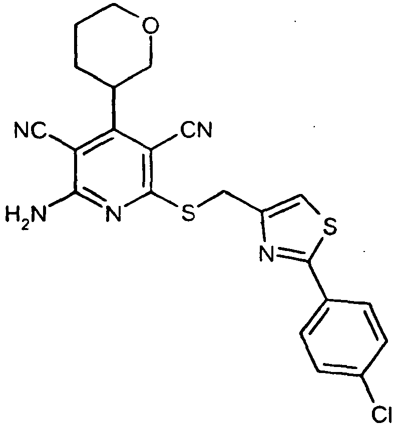

50 mg (0.13 mmol) der Verbindung aus Beispiel 32A, 39 mg (0.16 mmol) 4-(Chlormethyl)-2-(4-chlorphenyl)-1,3-thiazol und 45 mg (0.54 mmol) Natriumhydrogencarbonat werden in 3 ml trockenem DMF vorgelegt. Das Reaktionsgemisch wird 12 h bei RT gerührt und dann direkt über präparative HPLC gereinigt (Säule: YMC GEL ODS-AQ S-5, 15 µm; Laufmittelgradient: Acetonitril/Wasser 10:90 → 95:5).
Ausbeute: 37 mg (58% d. Th.)
¹H-NMR (400 MHz, DMSO-d₆): δ = 8.28-7.83 (br. s, 2H), 7.93 (d, 2H), 7.88 (s, 1H), 7.57 (d, 2H), 4.59 (s, 2H), 3.97-3.88 (m, 1H), 3.86-3.79 (m, 2H), 3.20-3.09 (m, 1H), 2.23 (dq, 1H), 1.93-1.84 (m, 1H), 1.77-1.56 (m, 2H).
LC-MS (Methode 7): Rₜ = 3.98 min; MS (ESIpos): m/z = 468 [M+H]⁺.

Die in der folgenden Tabelle aufgeführten Verbindungen werden analog zu Beispiel 26 aus den entsprechenden Ausgangsverbindungen hergestellt:

| **Beispiel Nr.** | **Struktur (Ausbeute)** | **LC-MS: Rₜ [min] (Methode); MS(ESI): m/z [M+H]⁺** | **¹H-NMR (DMSO-d₆): δ =** |
|---|---|---|---|
| **27** | 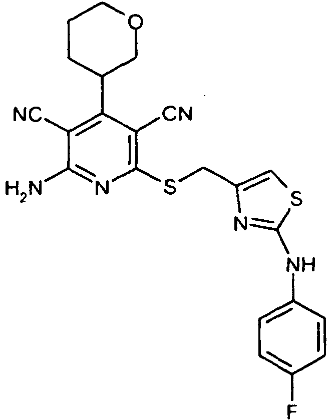 | 3.54 min (7); m/z = 467 | 10.21 (s, 1 H), 8.26-7.86 (br. s, 2H), 7.60 (dd, 2H), 7.13 (pseudo-t, 2H), 6.93 (s, 1 H), 4.51 (s, 2H), 3.96-3.88 (m, 1 H), 3.82 (d, 2H), 3.21-3.09 (m, 1H), 2.24 (dq, 1H), 1.89 (d, 1H), 1.78-1.56 (m, 2H). |
| | (31% d. Th.) | | |
| **28** | 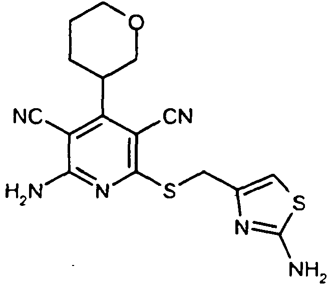 | 2.07 min (5); m/z = 373 | 8.19-7.80 (br. s, 2H), 6.98 (s, 2H), 6.61 (s, 1 H), 4.24 (s, 2H), 3.96-3.89 (m, 1H), 3.86-3.79 (m, 2H), 3.20-3.09 (m, 1H), 2.24 (dq, 1H), 1.94-1.84 (m, 1H), 1.77-1.55 (m, 2H). |
| | (40% d. Th.) | | |

### Beispiel 29

### 2-Amino-6-({[2-(4-chlorphenyl)-1,3-thiazol-4-yl]methyl}thio)-4-cyclohexylpyridin-3,5-dicarbonitril

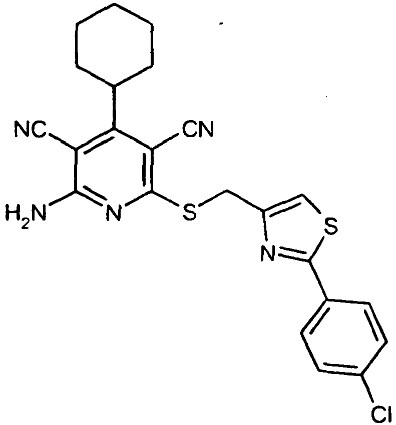

2.00 g (5.42 mmol) der Verbindung aus Beispiel 33A werden in 111 ml trockenem DMF vorgelegt und mit 1.46 g (5.96 mmol) 4-(Chlormethyl)-2-(4-chlorphenyl)-1,3-thiazol sowie 1.82 g (21.68 mmol) Natriumhydrogencarbonat versetzt. Das Reaktionsgemisch wird 12 h bei RT gerührt. Der Ansatz wird danach mit 20 ml Wasser verdünnt und zweimal mit je 100 ml Ethylacetat extrahiert. Die vereinigten organischen Phasen werden einmal mit 15 ml ges. wässriger Natriumhydrogencarbonat-Lösung gewaschen und über Magnesiumsulfat getrocknet. Nach Entfernen des Lösungsmittels wird der Rückstand an Kieselgel 60 chromatographisch gereinigt (Laufmittel: Gradient Cyclohexan/Ethviacetat 50:1 → 2:1).
Ausbeute: 1.08 g (40% d. Th.)
¹H-NMR (400 MHz, DMSO-d₆): δ = 8.20-7.80 (br. s, 2H), 7.93 (d, 2H), 7.88 (s, 1H), 7.57 (d, 1H), 4.59 (s, 2H), 2.92-2.80 (m, 1H), 2.01-1.88 (m, 2H), 1.87-1.77 (m, 2H), 1.76-1.60 (m, 3H), 1.37-1.10 (m, 3H).
LC-MS (Methode 12): Rₜ = 3.10 min; MS (ESIpos): m/z = 466 [M+N]⁺.

Die in der folgenden Tabelle aufgeführten Verbindungen werden analog zu Beispiel 29 aus den entsprechenden Ausgangsverbindungen hergestellt:

| **Beispiel Nr.** | **Struktur (Ausbeute)** | **LC-MS: Rₜ [min] Methode); MS (ESI): m/z [M+H]⁺** | **¹H-NMR (DMSO-d₆):** δ = |
|---|---|---|---|
| **30** | 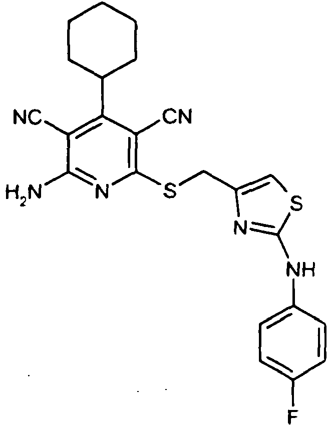 | 2.79 min (12); m/z = 465 | 10.20 (s, 1 H), 7.96 (br. s, 2H), 7.59 (dd, 2H), 7.11 (dd, 2H), 6.92 (s, 1H), 4.39 (s, 2H), 2.90-2.84 (m, 1 H), 1.99-1.90 (m, 2H), 1.85-1.82 (m, 2H), 1.70-1.66 (m, 3H), 1.39-1.12 (m, 3H). |
| | (31 % d. Th.) | | |
| **31** | 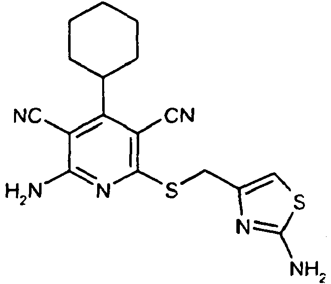 | 2.15 min (15); m/z = 371 | 7.92 (br. s, 2H), 6.96 (s, 2H), 6.60 (s, 1H), 4.26 (s, 2H), 2.90-2.84 (m, 1H), 2.00-1.90 (m, 2H), 1.85-1.82 (m, 2H), 1.72-1.69 (m, 3H), 1.38-1.12 (m, 3H). |
| | (40% d. Th.) | | |
| **32** | 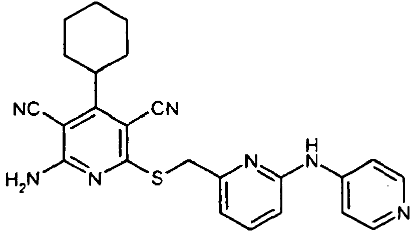 | 1.87 min (13); m/z = 442 | 8.83 (d, 2H), 8.69 (s, 2H), 8.29-7.78 (br. s, 2H), 8.11 (t, 1H), 7.88 (d, 1 H), 7.80 (d, 1 H), 7.01 (d, 2H), 4.61 (s, 2H), 2.93-2.82 (m, 1H), 2.01-1.89 (m, 2H), 1.89-1.79 (m, 2H), 1.77-1.64 (m, 3N), 1.37-1.12 (m, 3H). |
| | (10% d. Th.) | | |

### Beispiel 33

### 2-({[2-(4-Chlorphenyl)-1,3-thiazol-4-yl]methyl}thio)-4-cyclohexyl-6-{[3-(diethylamino)propyl]-amino}pyridin-3,5-dicarbonitril

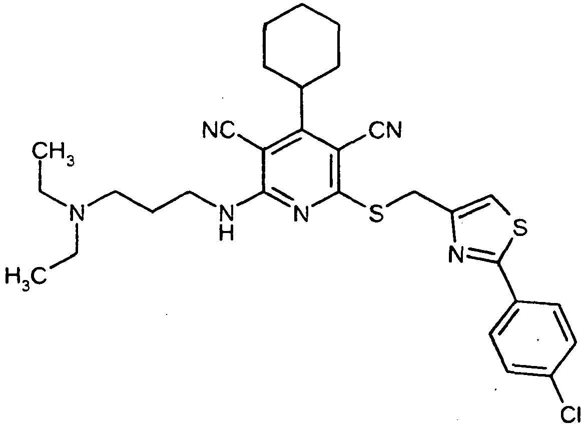

50 mg (0.10 mmol) der Verbindung aus Beispiel 37A und 30 mg (0.23 mmol) 3-(Diethylamino)-propylamin werden in 0.7 ml trockenem DMF gelöst. Das Reaktionsgemisch wird 12 h bei RT gerührt und dann direkt über präparative HPLC gereinigt (Säule: YMC GEL ODS-AQ S-5, 15 µm; Laufmittelgradient: Acetonitril/Wasser 10:90 → 95:5).
Ausbeute: 35 mg (58% d. Th.)
¹H-NMR (400 MHz, DMSO-d₆): δ = 8.58 (t, 1H), 7.93 (d, 2H), 7.63 (s, 1H), 7.55 (d, 2H), 4.66 (s, 2H), 3.53-3.45 (m, 2H), 2.92-2.84 (m, 1H), 2.43-2.31 (m, 6H), 2.02-1.90 (m, 2H), 1.89-1.81 (m, 2H), 1.76-1.68 (m, 3H), 1.65-1.56 (m, 2H), 1.39-1.14 (m, 3H), 0.89 (t, 6H).
LC-MS (Methode 2): Rₜ = 4.66 min; MS (ESIpos): m/z = 579 [M+H]⁺.

Die in der folgenden Tabelle aufgeführten Verbindungen werden analog zu Beispiel 33 aus den entsprechenden Ausgangsverbindungen hergestellt:

| **Beispiel Nr.** | **Struktur (Ausbeute)** | **LC-MS: R, [min] (Methode); MS (ESI): m/z [M+H]⁺** | **¹H-NMR (DMSO-d₆): δ =** |
|---|---|---|---|
| **34** | 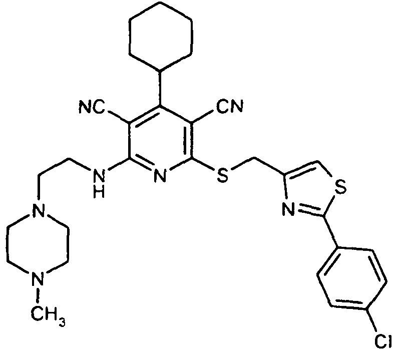 | 4.51 min (2); m/z = 592 | 7.93 (d, 2H), 7.86-7.79 (m, 1H), 7.65 (s, 1H), 7.56 (d, 2H), 4.67 (s, 2H), 3.57-3.49 (m, 2H), 2.94-2.86 (m, 1H), 2.42 (t, 2H), 2.37-2.11 (m, 8H), 2.08 (s, 3H), 2.03-1.89 (m, 2H), 1.88-1.71 (m, 2H), 1.77-1.67 (m, 3H), 1.39-1.15 (m, 3H). |
| | (64% d. Th.) | | |
| **35** | 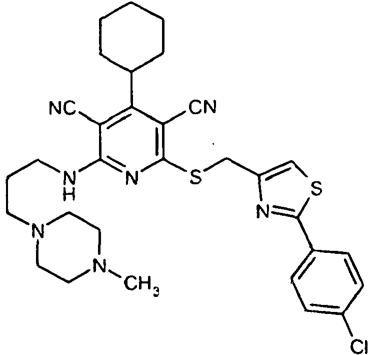 | 4.66 min (2); m/z = 606 | 8.11 (t, 1H), 7.93 (d, 2H), 7.63 (s, 1H), 7.57 (d, 2H), 4.66 (s, 2H), 3.47 (q, 2H), 2.94-2.84 (m, 1H), 2.32-2.10 (m, 9H), 2.07 (s, 3H), 2.02-1.89 (m, 2H), 1.88-1.79 (m, 2H), 1.77-1.67 (m, 3H), 1.64-1.54 (m, 2H), 1.38-1.14 (m, 4H). |
| | (8% d. Th.) | | |
| **36** | 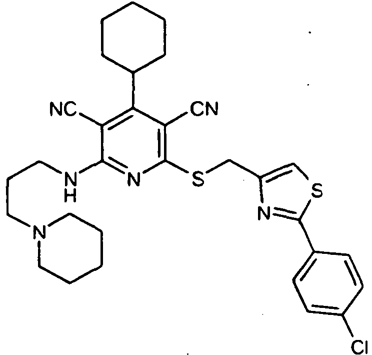 | 4.63 min (2); m/z = 591 | 8.21-8.15 (m, 1 H), 7.93 (d, 2H), 7.63 (s, 1 H), 7.56 (d, 2H), 4.65 (s, 2H), 3.52-3.43 (m, 2H), 2.96-2.85 (m, 1H), 2.28-2.11 (m, 6H), 2.03-1.90 (m, 2H), 1.89-1.80 (m, 2H), 1.78-1.67 (m, 3H), 1.66-1.54 (m, 2H), 1.48-1.39 (m, 4H), 1.39-1.14 (m, 5H). |
| | (69% d. Th.) | | |
| **37** | 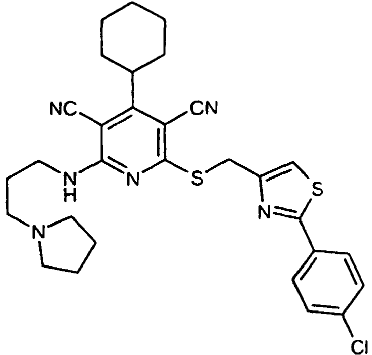 | 4.64 min (2); m/z = 577 | |
| | (71 % d. Th.) | | |
| **38** | 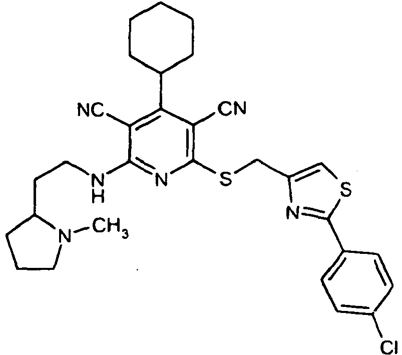 | 4.63 min (2); m/z = 577 | 8.79-8.73 (m, 1H), 7.92 (d, 2H), 7.63 (s, 1H), 7.56 (d, 2H), 4.67 (s, 2H), 3.61-3.02 (m, 1H), 3.48-3.33 (m, 1H), 2.93-2.84 (m, 2H), 2.19-2.12 (m, 1 H), 2.18 (s, 3H), 2.03-1.88 (m, 3H), 1.87-1.66 (m, 7H), 1.65-1.51 (m, 3H), 1.50-1.40 (m, 1H), 1.38-1.13 (m, 3H). |
| | (73% d. Th.) | | |
| **39** | 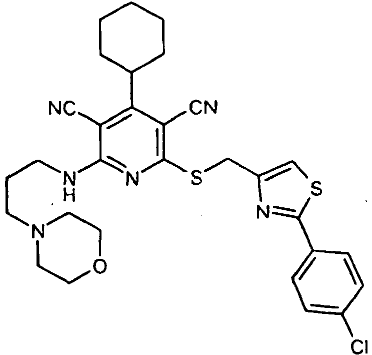 | 4.55 min (2); m/z = 593 | 8.18-8.09 (m, 1H), 7.93 (d, 2H), 7.64 (s, 1H), 7.58 (d, 2H), 4.66 (s, 2H), 3.54-3.41 (m, 6H), 2.96-2.84 (m, 1H), 2.30-2.16 (br. s, 6H), 2.03-1.91 (m, 2H), 1.89-1.79 (m, 2H), 1.77-1.66 (m, 3H), 1.65-1.55 (m, 2H), 1.39-1.13 (m, 3H). |
| | (93% d. Th.) | | |
| **40** | 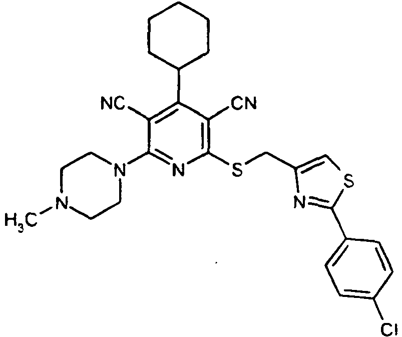 | 2.53 min (12); m/z = 549 | 7.94 (d, 2H), 7.67 (s, 1H), 7.58 (d, 2H), 4.63 (s, 2H), 3.80 (br. s, 4H), 3.07-2.96 (m, 1N), 2.39-2.29 (m, 4H), 2.13 (s, 3H), 2.07-1.93 (m, 2H), 1.90-1.81 (m, 2H), 1.78-1.67 (m, 3H), 1.38-1.16 (m, 3H). |
| | (92% d. Th.) | | |
| **41** | 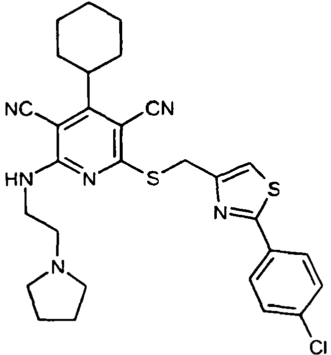 | 2.09 min (12); m/z = 563 | 7.99-7.90 (m, 3H), 7.64 (s, 1H), 7.57 (d, 2H), 4.68 (s, 2H), 3.53 (q, 2H), 2.95-2.84 (m, 1H), 2.39-2.31 (m, 4H), 2.03-1.90 (m, 2H), 1.89-1.82 (m, 2H), 1.77-1.68 (m, 3H), 1.61-1.53 (m, 4H), 1.38-1.15 (m, 3H). |
| | (47% d. Th.) | | |
| **42** | 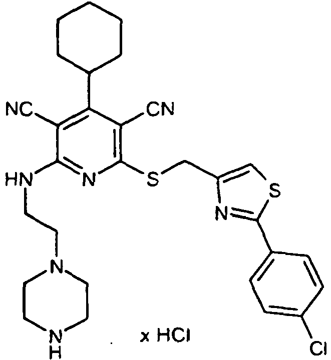 | 2.20 min (12); m/z = 578 | |
| | (77% d. Th.) | | |

### Beispiel 43

### 2-Amino-6-({[2-(4-chlorphenyl)-1,3-thiazol-4-yl]methyl}thio)-4-cyclohex-3-en-1-ylpyridin-3,5-di-carbonitril

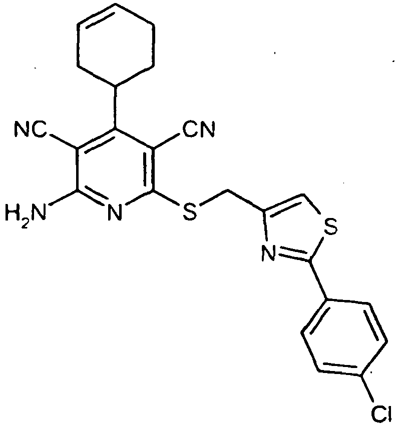

50 mg (0.20 mmol) der Verbindung aus Beispiel 38A, 52 mg (0.22 mmol) 4-(Chlormethyl)-2-(4-chlorphenyl)-1,3-thiazol sowie 49 mg (0.59 mmol) Natriumhydrogencarbonat werden in 2.5 ml trockenem DMF vorgelegt. Das Reaktionsgemisch wird 12 h bei RT gerührt und dann direkt über präparative HPLC gereinigt (Säule: YMC GEL ODS-AQ S-5, 15 µm; Laufmittelgradient: Acetonitril/Wasser 10:90 → 95:5).
Ausbeute: 42 mg (46% d. Th.)
¹H-NMR (400 MHz, DMSO-d₆): δ = 8.26-7.86 (br. s, 2H), 7.94 (d, 2H), 7.89 (s, 1H), 7.58 (d, 2H), 5.81-5.71 (m, 2H), 4.60 (s, 2H), 3.13-3.03 (m, 1H), 2.26-2.08 (m, 4H), 1.83-1.76 (m, 1 H).
LC-MS (Methode 13): R₁ = 3.20 min; MS (ESIpos): m/z = 464 [M+H]⁺.

Die in der folgenden Tabelle aufgeführten Verbindungen werden analog zu Beispiel 43 aus den entsprechenden Ausgangsverbindungen hergestellt:

| **Beispiel Nr.** | **Struktur (Ausbeute)** | **LC-MS: R, [min] (Methode); MS (ESI): m/z [M+H]⁺** | **¹H-NMR (DMSO-d₆): δ =** |
|---|---|---|---|
| **44** | 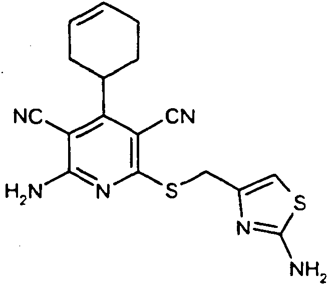 | 1.82 min (12); m/z = 369 | 8.14-7.83 (br. s, 2H), 6.98 (s, 2H), 6.60 (s, 1H), 5.81-5.71 (m, 2H), 4.26 (s, 2H), 3.13-3.03 (m, 1H), 2.28-2.06 (m, 4H), 1.83-1.75 (m, 1 H). |
| | (47% d. Th.) | | |
| **45** | 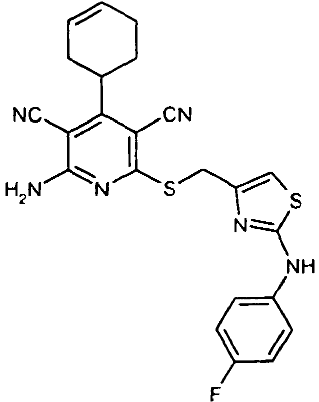 | 2.83 min (13); m/z = 463 | 8.20-7.83 (br. s, 2H), 7.60 (dd, 2H), 7.13 (pseudo-t, 2H), 6.93 (s, 1H), 5.81-5.71 (m, 2H), 4.40 (s, 2H), 3.13-3.04 (m, 1H), 2.28-2.08 (m, 4H), 1.81-1.75 (m, 1H). |
| | (23% d. Th.) | | |

### Beispiel 46

### 2-Amino-6-({[2-(4-chlorphenyl)-1,3-thiazol-4-yl]methyl}thio)-4-(tetrahydro-2H-pyran-4-yl)-pyridin-3,5-dicarbonitril

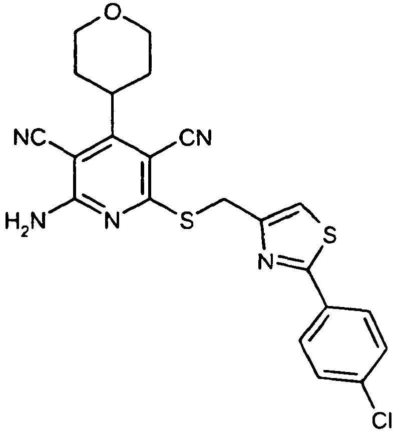

100 mg (0.34 mmol) der Verbindung aus Beispiel 34A, 83 mg (0.34 mmol) 4-(Chlormethyl)-2-(4-chlorphenyl)-1,3-thiazol sowie 57 mg (0.68 mmol) Natriumhydrogencarbonat werden in 5 ml trockenem DMF vorgelegt. Das Reaktionsgemisch wird 16 h bei RT gerührt. Nach Entfernen des Lösungsmittels am Rotationsverdampfer wird der Rückstand mit ca. 2 ml Acetonitril ausgerührt. Dabei fällt ein Niederschlag aus, der abgesaugt und getrocknet wird.
Ausbeute: 72 mg (45% d. Th.)
¹H-NMR (400 MHz, DMSO-d₆): δ = 8.04 (br. s, 2H), 7.93 (d, 2H), 7.88 (s, 1H), 7.56 (d, 2H), 4.59 (s, 2H), 3.98 (dd, 2H), 3.39 (dd, 2H), 3.13 (m, 1H), 2.24-2.15 (m, 2H), 1.61 (d, 2H).
LC-MS (Methode 15): Rₜ = 2.96 min; MS (ESIpos): m/z = 468 [M+H]⁺.

Die in der folgenden Tabelle aufgeführten Verbindungen werden analog zu Beispiel 46 aus den entsprechenden Ausgangsverbindungen hergestellt:

| **Beispiel Nr.** | **Struktur (Ausbeute)** | **LC-MS: R, [min] (Methode); MS (ESI): m/z [M+H]⁺** | **¹H-NMR (DMSO-d₆): δ =** |
|---|---|---|---|
| **47** | 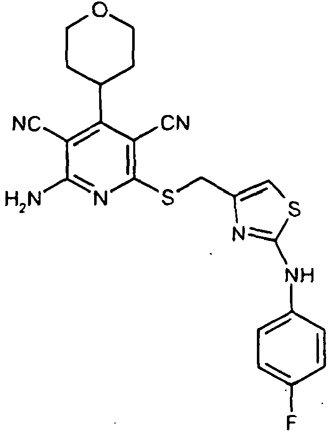 | 2.49 min (13); m/z = 467 | 10.21 (s, 1H), 8.05 (br. s, 1H), 7.60 (dd, 2H), 7.13 (t, 2H), 6.94 (s, 1H), 4.41 (s, 2H), 4.00 (dd, 2H), 3.38 (dd, 2H), 3.14 (m, 1 H), 2.24-2.16 (m, 2H), 1.61 (d, 2H). |
| | (13% d. Th.) | | |
| **48** | 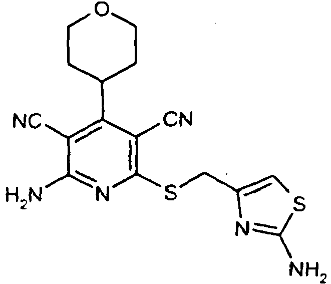 | 1.55 min (13); m/z = 371 | 7.98 (br. s, 2H), 6.70 (s, 1H), 6.60 (s, 1H), 4.26 (s, 2H), 3.98 (dd, 2H), 3.38 (dd, 2H), 3.13 (m, 1 H), 2.24-2.16 (m, 2H), 1.61 (d, 2H). |
| | (35% d. Th.) | | |

### Beispiel 49

### Melhyl 3-({[6-amino-3,5-dicyano-4-(tetrahydro-2H-pyran-2-yl)pyridin-2-yl]thio}methyl)benzoat

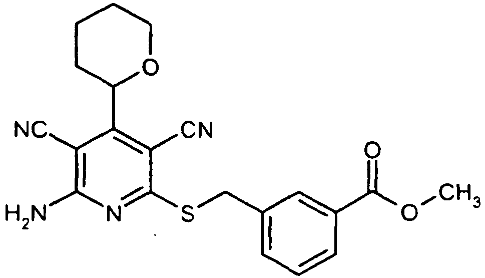

Eine Lösung von 50 mg (0.19 mmol) der Verbindung aus Beispiel 31A und 48 mg (0.21 mmol) 3-(Brommethyl)benzoesäuremethylester in 2 ml trockenem DMF werden mit 48 mg (0.58 mmol) Natriumhydrogencarbonat versetzt und 20 h bei RT gerührt. Der Ansatz wird danach direkt mittels präparativer HPLC gereinigt (Säule: YMC GEL ODS-AQ S-5 / 15 µm; Laufmittelgradient: Acetonitril/Wasser 10:90 → 95:5).
Ausbeute: 44 mg (53% d. Th.)
¹H-NMR (400 MHz, CDCl₃): δ = 8.25-7.77 (br. s, 2H), 8.06 (s, 1H), 7.82 (pseudo-dd, 2H), 7.46 (t, 1H), 4.58-4.49 (m, 1H), 4.53 (s, 2H), 4.02 (dd, 1H), 3.85 (s, 3H), 3.55-3.45 (m, 1H), 1.97-1.85 (br. s, 1H), 1.73-1.51 (m, 5H).
LC-MS (Methode 5): R, = 3.79 min; MS (ESIpos): m/z = 409 [M+H]⁺.

Die in der folgenden Tabelle aufgeführten Verbindungen werden analog zu Beispiel 49 aus den entsprechenden Ausgangsverbindungen hergestellt:

| **Beispiel Nr.** | **Struktur (Ausbeute)** | **LC-MS: R, [min] (Methode); MS (ESI): m/z [M+H]⁺** | **¹H-NMR (DMSO-d6): δ =** |
|---|---|---|---|
| **50** | 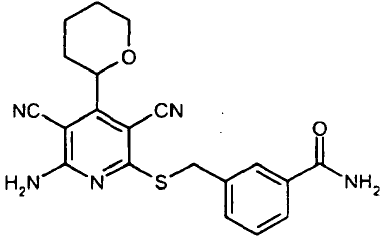 | 3.12 min (5); m/z = 394 | 8.21-7.82 (br. s, 2H), 7.99-7.94 (m, 2H), 7.76 (d, 1H), 7.66 (d, 1H), 7.43-7.35 (m, 2H), 4.57-4.45 (m, 1 H), 4.49 (s, 2H), 4.03 (dd, 1H), 3.56-3.46 (m, 1H), 1.95-1.86(br. s, 1H), 1.74-1.51 (m, 5H). |
| | (29% d. Th.) | | |
| 51 | 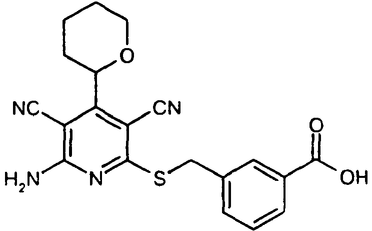 | 3.38 min (5); m/z = 395 | 13.0 (br. s, 1H), 8.30-7.71 (br. s, 2H), 8.02 (s, 1H), 7.79 (dd, 2H), 7.43 (t, 1H), 4.52 (s, 2H), 4.52-4.51 (m, 1H), 4.02 (d, 1H), 3.54-3.46 (m, 1H), 1.97-1.86 (br. s, 1H), 1.73-1.50 (m, 5H). |
| | (44% d. Th.) | | |

### Beispiel 52

### 2-({[2-(4-Chlorphenyl)-1,3-thiazol-4-yl]methyl}sulfanyl)-6-methyl-4-(tetrahydro-2H-pyran-2-yl)-pyridin-3,5-dicarbonitril

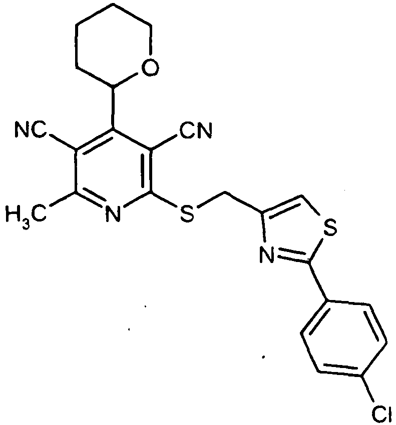

Analog zur Herstellung der Verbindung in Beispiel 14 werden 50 mg (0.14 mmol) der Verbindung aus Beispiel 41A, 40 mg (0.16 mmol) 4-(Chlormethyl)-2-(4-chlorphenyl)-1,3-thiazol und 45 mg (0.54 mmol) Natriumhydrogencarbonat in 2.8 ml trockenem DMF umgesetzt.
Ausbeute: 32 mg (50% d. Th.)
¹H-NMR (400 MHz, DMSO-d₆): δ = 7.94 (d, 2H), 7.73 (s, 1H), 7.57 (d, 2H), 4.73 (s, 2H), 4.71-4.69 (m, 1H), 4.07 (dd, 1H), 3.62-3.53 (m, 1H), 2.78 (s, 3H), 2.00-1.89 (br. s, 1H), 1.82-1.72 (m, 1H), 1.71-1.53 (m, 4H).
LC-MS (Methode 4): Rₜ = 3.36 min; MS (ESIpos): m/z = 467 [M+H]⁺.

### Beispiel 53

### rac-Ethyl-4-[4-({[3,5-dicyano-6-methyl-4-(tetrahydro-2H-pyran-2-yl)pyridin-2-yl]sulfanyl}-methyl)-1,3-thiazol-2-yl]benzoat

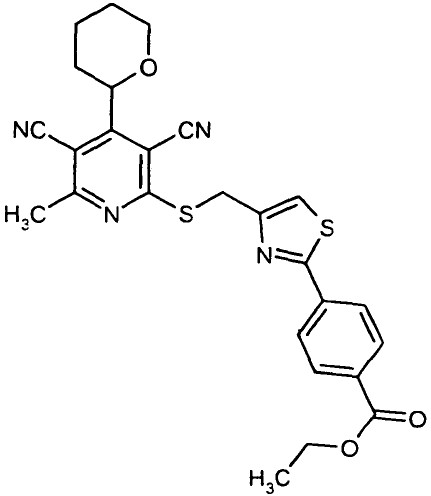

Die Titelverbindung wird analog zu Beispiel 52 aus den entsprechenden Edukten erhalten.
Ausbeute: 192 mg (37% d. Th.)
¹H-NMR (400 MHz, DMSO-d_{b}): δ = 8.06 (d, 4H), 7.81 (s, 1H), 4.75 (s, 2H), 4.70 (d, 1H), 4.33 (q, 2H), 4.07 (dd, 1H), 3.62-3.53 (m, 1H), 2.79 (s, 3H), 1.99-1.89 (m, 1H), 1.82-1.73 (m, 1H), 1.70-1.55 (m, 4H), 1.34 (t, 3H).
LC-MS (Methode 4): Rₜ = 3.32 min; MS (ESIpos): m/z = 505 [M+H]⁺.

### Beispiel 54 und Beispiel 55

### ent-Ethyl-4-[4-({[3,5-dicyano-6-methyl-4-(tetrahydro-2H-pyran-2-yl)pyridin-2-yl]sulfanyl}-methyl)-1,3-thiazol-2-yl]benzoat (Enantiomer 1 und Enantiomer 2)

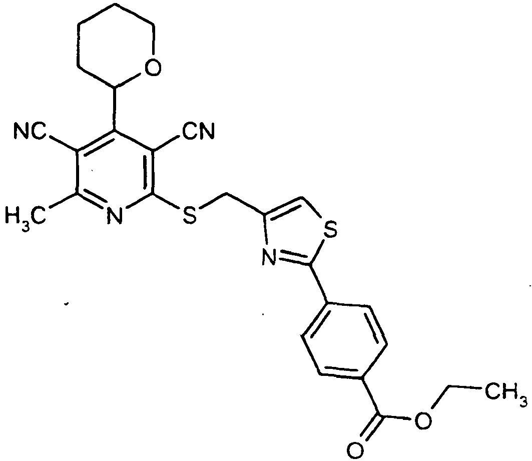

190 mg der Verbindung aus Beispiel 53 werden bei ca. 30°C in 4 ml Methanol und 10 ml TBME gelöst und mittels präparativer HPLC an chiraler Phase (Methode 16) in die Enantiomere getrennt:

### Beispiel 54 (Enantiomer 1):

Ausbeute: 90 mg
HPLC (Methode 17): Rₜ = 5.44 min; ee >99%
optische Drehung: +0.073° (c = 0.50 g / 100 ml, Chloroform).

### Beispiel 55 (Enantiomer 2):

Ausbeute: 82 mg
HPLC (Methode 17): Rₜ = 5.83 min; ee >98%.

### Beispiel 56

### (+)-4-[4-({(3,5-Dicyano-6-methyl-4-(tetrahydro-2H-pyran-2-yl)pyridin-2-yl]sulfanyl}methyl)-1,3-thiazol-2-yl]benzoesäure

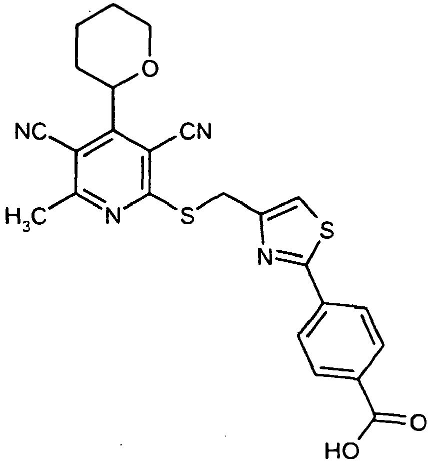

Die Titelverbindung wird analog zu Beispiel 22 aus der Verbindung in Beispiel 54 erhalten.
Ausbeute: 17 mg (19% d. Th.)
¹H-NMR (400 MHz, DMSO-d₆): δ = 13.22-13.13 (br. s, 1H), 8.04 (s, 4H), 7.71 (s, 1H), 4.76 (s, 2H), 4.74-4.68 (m, 1H), 4.06 (dd, 1H), 3.62-3.53 (m, 1H), 2.79 (s, 3H), 1.98-1.89 (m, 1H), 1.82-1.72 (m, 1H), 1.72-1.55 (m, 4H).
LC-MS (Methode 4): Rₜ = 2.84 min; MS (ESIpos): m/z = 477 [M+H]⁺
optische Drehung: +0.009° (c = 0.17 g / 100 ml, Methanol).

### Beispiel 57

### (-)-4-[4-({[3,5-Dicyano-6-methyl-4-(tetrahydro-2H-pyran-2-yl)pyridin-2-yl]sulfanyl}methyl)-1,3-thiazol-2-yl]benzoesäure

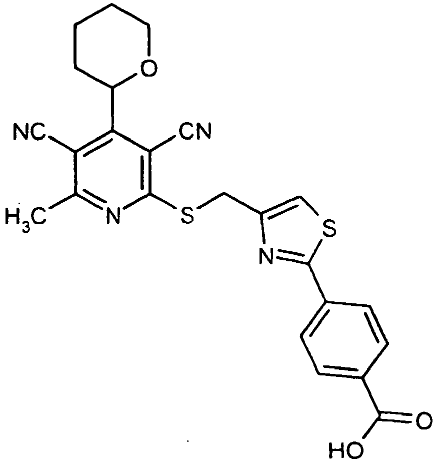

Die Titelverbindung wird analog zu Beispiel 22 aus der Verbindung in Beispiel 55 erhalten.
Ausbeute: 11 mg (12% d. Th.)
¹H-NMR (400 MHz, DMSO-d₆): δ = 13.22-13.11 (br. s, 1H), 8.04 (s, 4H), 7.70 (s, 1H), 4.76 (s, 2H), 4.74-4.68 (m, 1H), 4.07 (dd, 1H), 3.62-3.52 (m, 1H), 2.79 (s, 3H), 1.98-1.89 (m, 1H), 1.82-1.72 (m, 1H), 1.71-1.54 (m, 4H).
LC-MS (Methode 22): Rₜ = 2.39 min; MS (ESIpos): m/z = 477 [M+H]⁺.

### Beispiel 58

### 2-Amino-6-({[2-(4-chlorphenyl)-1,3-oxazol-4-yl]methyl}sulfanyl)-4-(tetrahydro-2H-pyran-3-yl)-pyridin-3,5-dicarbonitril

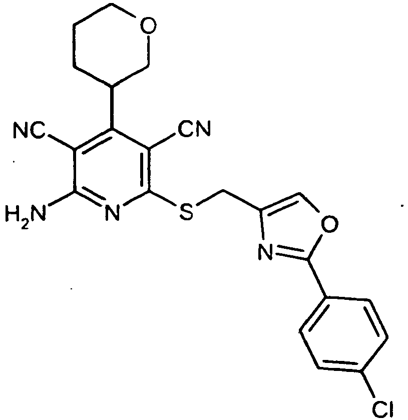

Die Titelverbindung wird analog zu Beispiel 14 aus 200 mg (0.67 mmol) der Verbindung in Beispiel 32A und 197 mg (0.74 mmol) der Verbindung in Beispiel 42A erhalten.
Ausbeute: 324 mg (95% d. Th.)
¹H-NMR (400 MHz, DMSO-d₆): δ = 8.33 (s, 1H), 8.28-7.88 (br. s, 2H), 7.97 (d, 2H), 7.61 (d, 2H), 4.48 (s, 2H), 3.95-3.88 (m, 1H), 3.83 (s, 2H), 3.82-3.78 (m, 1H), 3.19-3.08 (m, 1H), 2.30-2.17 (m, 1H), 1.93-1.86 (m, 1H), 1.77-1.68 (m, 1H), 1.68-1.55 (m, 1H).
LC-MS (Methode 4): Rₜ = 2.87 min; MS (ESIpos): m/z = 452 [M+H]⁺.

### Beispiel 59

### rac-Ethyl-4-[4-({[6-amino-3,5-dicyano-4-(tetrahydro-2H-pyran-3-yl)pyridin-2-yl]sulfanyl}methyl)-1,3-thiazol-2-yl]benzoat

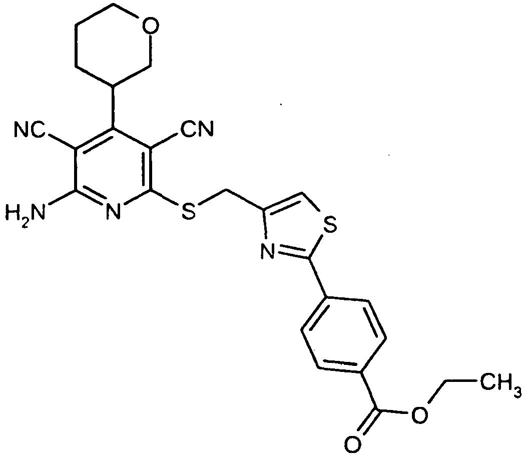

Die Titelverbindung wird analog zu Beispiel 14 aus 282 mg (0.76 mmol) der Verbindung in Beispiel 32A und 290 mg (0.83 mmol) Ethyl-4-[4-(chlormethyl)-1,3-thiazol-2-yl]benzoat erhalten.
Ausbeute: 181 mg (47% d. Th.)
¹H-NMR (400 MHz, DMSO-d₆): δ = 8.33-7.89 (br. s, 2H), 8.08 (s, 4H), 7.96 (s, 1H), 4.61 (s, 2H), 4.36 (q, 2H), 3.96-3.87 (m, 1H), 3.83 (s, 2H), 3.83-3.79 (m, 1H), 3.19-3.08 (m, 1H), 2.29-2.18 (m, 1H), 1.93-1.85 (m, 1H), 1.76-1.69 (m, 1H), 1.69-1.57 (m, 1H), 1.35 (t, 3H).
LC-MS (Methode 4): Rₜ = 2.87 min; MS (ESIpos): m/z = 452 [M+H]⁺.

### Beispiel 60 und Beispiel 61

### ent-Ethyl-4-[4-({[6-amino-3,5-dicyano-4-(tetrahydro-2H-pyran-3-yl)pyridin-2-yl]sulfanyl}methyl)-1,3-thiazol-2-yl]benzoat (Enantiomer 1 und Enantiomer 2)

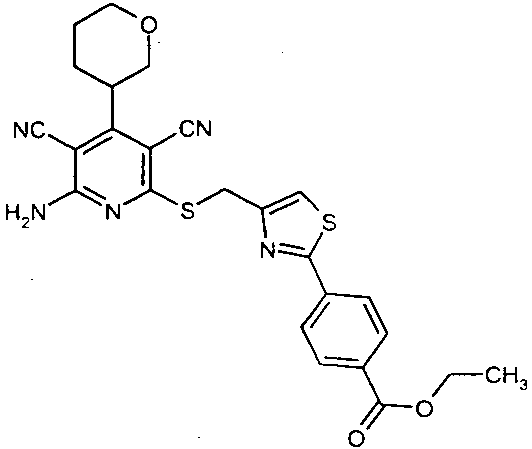

180 mg der Verbindung aus Beispiel 59 werden bei ca. 30°C in 5 ml Methanol und 20 ml TBME gelöst und mittels präparativer HPLC an chiraler Phase (Methode 18) in die Enantiomere getrennt:

### Beispiel 60 (Enantiomer 1):

Ausbeute: 59 mg
HPLC (Methode 19): Rₜ = 9.11 min; ee >99%
optische Drehung: +0.057° (c = 0.455 g / 100 ml, Chloroform).

### Beispiel 61 (Enantiomer 2):

Ausbeute: 77 mg
HPLC (Methoden 19): Rₜ = 10.29 min; ee >99%.

### Beispiel 62

### (+)-4-[4-({[6-Amino-3,5-dicyano-4-(tetrahydro-2H-pyran-3-yl)pyridin-2-yl]sulfanyl}methyl)-1,3-thiazol-2-yl]benzoesäure

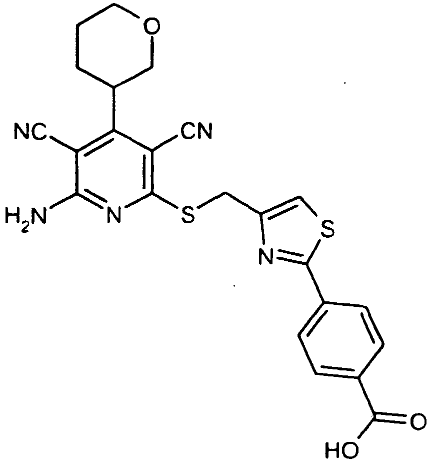

Die Titelverbindung wird analog zu Beispiel 22 aus der Verbindung in Beispiel 60 erhalten.
Ausbeute: 18 mg (48% d. Th.)
¹H-NMR (400 MHz, DMSO-d₆): δ = 13.23-13.10 (br. s, 1H), 8.35-7.83 (br. s, 2H), 8.05 (s, 4H), 7.96 (s, 1H), 4.61 (s, 2H), 3.91 (dd, 1H), 3.84-3.78 (m, 2H), 3.19-3.08 (m, 1H), 2.30-2.18 (m, 1H), 1.93-1.85 (m, 1H), 1.77-1.69 (m, 1H), 1.69-1.56 (m, 1H).
LC-MS (Methode 4): R, = 2.41 min; MS (ESIpos): m/z = 478 [M+H]⁺.

### Beispiel 63

### (-)-4-[4-({[6-Amino-3,5-dicyano-4-(tetrahydro-2H-pyran-3-yl)pyridin-2-yl]sulfanyl}methyl)-1,3-thiazol-2-yl]benzoesäure

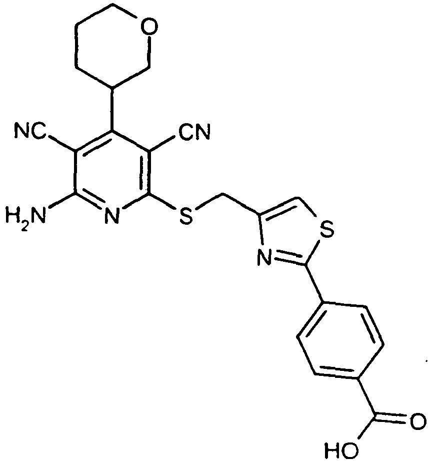

Die Titelverbindung wird analog zu Beispiel 22 aus der Verbindung in Beispiel 61 erhalten.
Ausbeute: 30 mg (53% d. Th.)
¹H-NMR (400 MHz, DMSO-d₆): δ = 13.22-13.13 (br. s, 1H), 8.29-7.88 (br. s, 2H), 8.04 (s, 4H), 7.95 (s, 1H), 4.60 (s, 2H), 3.92 (dd, 1H), 3.83-3.78 (m, 2H), 3.19-3.09 (m, 1H), 2.31-2.18 (m, 1 H), 1.95-1.85 (m, 1H), 1.77-1.68 (m, 1H), 1.68-1.57 (m, 1H).
LC-MS (Methode 4): Rₜ = 2.42 min; MS (ESIpos): m/z = 478 [M+H]⁺
optische Drehung: -0.050° (c = 0.495 g / 100 ml, Methanol/Dichlormethan 1:1).

### Beispiel 64

### rac-4-[4-({[6-Amino-3,5-dicyano-4-(tetrahydro-2H-pyran-3-yl)pyridin-2-yl]sulfanyl}methyl)-1,3-thiazol-2-yl]benzoesäure

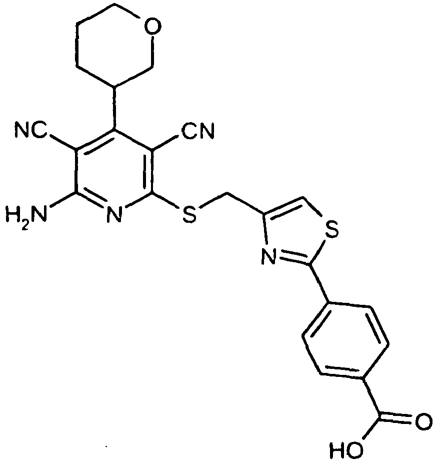

Die Titelverbindung wird analog zu Beispiel 22 aus 69 mg (0.14 mmol) der Verbindung aus Beispiel 59 erhalten.
Ausbeute: 51 mg (78% d. Th.)
LC-MS (Methode 5): Rₜ = 3.37 min; MS (ESIpos): m/z = 478 [M+H]⁺.

### Beispiel 65

### rac-4-[4-({[3,5-Dicyano-4-(tetrahydro-2H-pyran-3-yl)pyridin-2-yl]sulfanyl}methyl)-1,3-thiazol-2-yl]benzoesäure

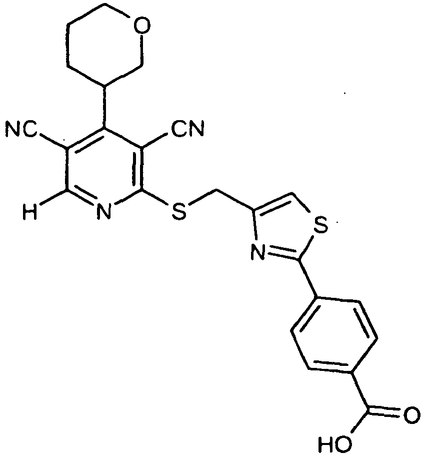

51 mg (0.11 mmol) der Verbindung aus Beispiel 64 werden in 2.6 ml trockenem THF vorgelegt und mit 84 mg (0.72 mmol) Isopentylnitrit sowie 1.43 mg (0.01 mmol) Kupfer(II)chlorid versetzt. Das Reaktionsgemisch wird 10 h bei RT gerührt. Der Ansatz wird danach mit 4 ml 1 N Salzsäure versetzt und die wässrige Phase zweimal mit je 10 ml Ethylacetat extrahiert. Die vereinigten organischen Phasen werden einmal mit 5 ml gesättigter wässriger Natriumhydrogencarbonat-Lösung und einmal mit 5 ml gesättigter wässriger Natriumchlorid-Lösung gewaschen und über Magnesiumsulfat getrocknet. Nach Entfernen des Lösungsmittels am Rotationsverdampfer wird der Rückstand mittels präparativer HPLC gereinigt (Säule: YMC GEL ODS-AQ S-5, 15 µm; Laufmittelgradient: Acetonitril/Wasser 10:90 → 95:5, mit 0.3% Salzsäure). Man erhält einen weißen Feststoff.
Ausbeute: 7 mg (14% d. Th.)
¹H-NMR (400 MHz, DMSO-d₆): δ = 13.18 (s, 1 H), 9.10 (s, 1H), 8.04 (s, 4H), 7.80 (s, 1 H), 4.78 (s, 2H), 3.99-3.91 (m, 2H), 3.82 (t, 1H), 3.42-3.31 (m, 2H), 2.34-2.20 (m, 1H), 2.05-1.9 (m, 1H), 1.81-1.60 (m, 2H).
LC-MS (Methode 7): R, = 3.45 min; MS (ESIpos): m/z = 463 [M+H]⁺.

### Beispiel 66

### rac-Methyl-N-[6-({[2-(4-chlorphenyl)-1,3-oxazol-4-yl]methyl} sulfanyl)-3,5-dicyano-4-(tetrahydro-2H-pyran-3-yl)pyridin-2-yl]-N-methylglycinat

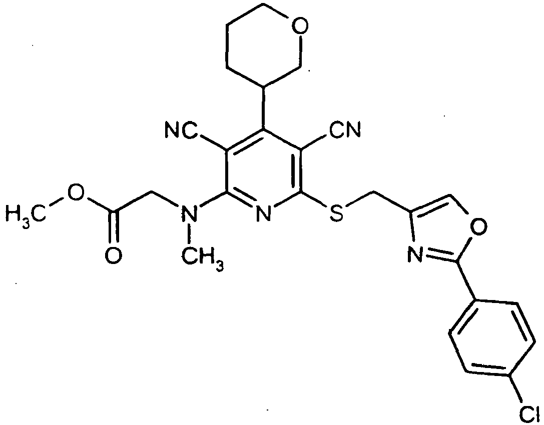

239 mg (0.42 mmol) der Verbindung aus Beispiel 43A werden in 6 ml trockenem THF gelöst und nacheinander mit 116 mg (0.83 mmol) Sarcosinmethylester-Hydrochlorid sowie 126 mg (1.25 mmol) Triethylamin versetzt. Das Reaktionsgemisch wird 10 h bei RT gerührt. Das Lösungsmittel wird danach am Rotationsverdampfer entfernt und der Rückstand mittels präparativer HPLC gereinigt (Säule: YMC GEL ODS-AQ S-5, 15 µm; Laufmittelgradient: Acetonitril/Wasser 10:90 → 95:5).
Ausbeute: 123 mg (55% d. Th.)
¹H-NMR (400 MHz, DMSO-d₆): δ = 8.16 (s, 1H), 7.97 (d, 2H), 7.61 (d, 2H), 4.56 (s, 2H), 4.36 (s, 2H), 3.97-3.81 (m, 3H), 3.63 (s, 3H), 3.42 (s, 3H), 3.30 (s, 2H), 2.38-2.25 (m, 1H), 1.98-1.89 (m, 1H), 1.79-1.71 (m, 1H), 1.71-1.58 (m, 1H).
LC-MS (Methode 2): R, = 2.47 min; MS (ESIpos): m/z = 538 [M+H]⁺.

### Beispiel 67

### rac-3-({ [6-Amino-3,5-dicyano-4-(tetrahydro-2H-pyran-2-yl)pyridin-2-yl]sulfanyl}methyl)benzamid

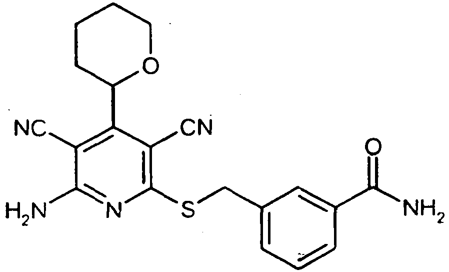

Analog zur Herstellung der Verbindung in Beispiel 14 werden 50 mg (0.14 mmol) der Verbindung aus Beispiel 31A, 36 mg (0.21 mmol) 3-(Chlormethyl)benzamid und 48 mg (0.58 mmol) Natriumhydrogencarbonat in 2.0 ml trockenem DMF umgesetzt.
Ausbeute: 23 mg (29% d. Th.)
¹H-NMR (400 MHz, DMSO-d₆): δ = 8.20-7.82 (br. s, 2H), 7.99-7.93 (m, 2H), 7.76 (d, 1H), 7.65 (d, 1H), 7.43-7.34 (m, 2H), 4.57-4.45 (m, 1H), 4.49 (s, 2H), 4.02 (dd, 1H), 3.56-3.45 (m, 1H), 1.95-1.85 (br. s, 1H), 1.73-1.50 (m, 5H).
LC-MS (Methode 5): Rₜ = 3.12 min; MS (ESIpos): m/z = 394 [M+H]⁺.

### Beispiel 68

### rac-2-Amino-6-{[(2-amino-1,3-thiazol-4-yl)methyl]sulfanyl}-4-(tetrahydro-2H-pyran-2-yl)pyridin-3,5-dicarbonitril

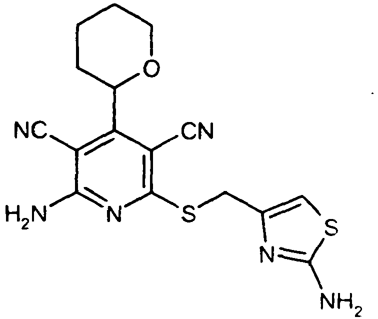

Analog zur Herstellung der Verbindung in Beispiel 14 werden 50 mg (0.14 mmol) der Verbindung aus Beispiel 31 A, 39 mg (0.21 mmol) 4-(Chlormethyl)-1,3-thiazol-2-amin und 48 mg (0.58 mmol) Natriumhydrogencarbonat in 2.0 ml trockenem DMF umgesetzt.
Ausbeute: 35 mg (49% d. Th.)
¹H-NMR (400 MHz, DMSO-d₆): δ = 8.20-7.79 (br. s, 2H), 7.08-6.93 (br. s, 2H), 6.60 (s, 1H), 4.59-4.49 (m, 1H), 4.27 (s, 2H), 4.02 (dd; 1H), 2.55-2.46 (m, 1H), 1.96.1.85 (m, 1H), 1.74-1.65 (m, 2H), 1.65-1.51 (m, 3H).
LC-MS (Methode 5): R, = 2.31 min; MS (ESIpos): m/z = 373 [M+H]⁺.

### Beispiel 69

### Methyl-3-{[(6-amino-3,5-dicyano-4-cyclohexylpyridin-2-yl)sulfanyl]methyl}benzoat

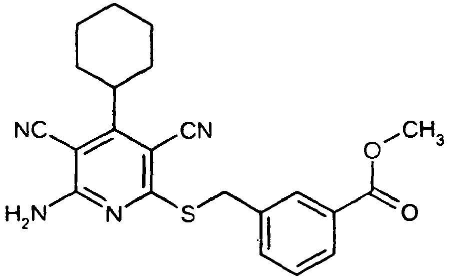

Analog zur Herstellung der Verbindung in Beispiel 29 werden 140 mg (0.54 mmol) der Verbindung aus Beispiel 33A, 137 mg (0.60 mmol) Methyl-3-(brommethyl)benzoat und 182 mg (2.17 mmol) Natriumhydrogencarbonat in 2.0 ml trockenem DMF umgesetzt.
Ausbeute: 30 mg (14% d. Th.)
¹H-NMR (400 MHz, DMSO-d₆): δ = 8.23-7.78 (br. s, 2H), 8.07 (s, 1H), 7.83 (d, 2H), 7.47 (t, 1H), 4.53 (s, 2H), 3.87 (s, 3H), 2.93-2.82 (m, 1H), 2.02-1.89 (m, 2H), 1.89-1.80 (m, 2H), 1.76-1.65 (m, 3H), 1.39-1.13 (m, 3H).
LC-MS (Methode 7): R, = 4.05 min; MS (ESIpos): m/z = 407 [M+H]⁺.

Die in der folgenden Tabelle aufgeführten Verbindungen werden analog zu Beispiel 69 aus den entsprechenden Ausgangsverbindungen hergestellt:

| **Beispiel Nr.** | **Struktur (Ausbeute)** | **LC-MS: R*ₜ*, [min] (Methode); MS (ESI): m/z [M+H]⁺** | **¹H-NMR (DMSO-d₆): δ =** |
|---|---|---|---|
| **70** | 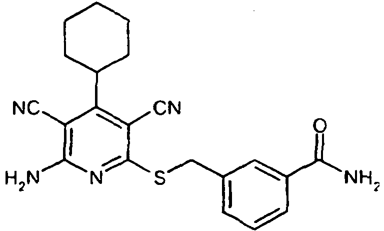 | 3.41 min (7); m/z = 392 | 8.18-7.82 (br. s, 2H), 7.98-7.91 (m, 1H), 7.75 (d, 1H), 7.66 (d, 1H), 7.44-7.33 (m, 2H), 4.48 (s, 2H), 3.47-3.40 (m, 1H), 2.92-2.82 (m, 1H), 2.02-1.88 (m, 2H), 1.87-1.78 (m, 2H), 1.75-1.65 (m, 3H), 1.38-1.13 (m, 3H). |
| | (16% d. Th.) | | |
| **71** | 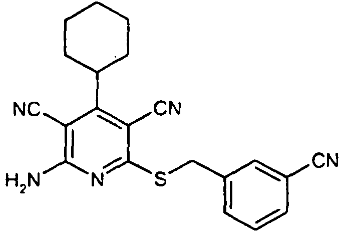 | 4.10 min (5); m/z = 373 | 8.46-7.63 (br. s, 2H), 8.06 (s, 1H), 7.86 (d, 1 H), 7.71 (d, 1 H), 7.51 (t, 1H), 4.45 (s, 2H), 2.91-2.81 (m, 1H), 1.99-1.77 (m, 4H), 1.76-1.63 (m, 3H), 1.37-1.12 (m, 3H). |
| | (26% d. Th.) | | |
| **72** | 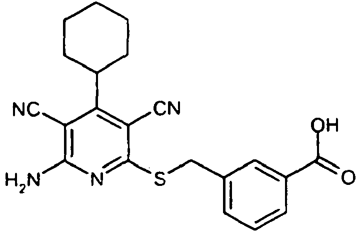 | 2.69 min (4); m/z = 393 | 13.15-12.82 (br. s, 1H), 8.17-7.71 (br. s, 2H), 8.02 (s, 1H), 7.81 (d, 1H), 7.75 (d, 1H), 7.41 (t, 1H), 4.52 (s, 2H), 2.92-2.81 (m, 1H), 2.01-1.88 (m, 2H), 1.88-1.78 (m, 2H), 1.76-1.65 (m, 3H), 1.37-1.13 (m, 3H). |
| | (16% d. Th.) | | |
| **73** | 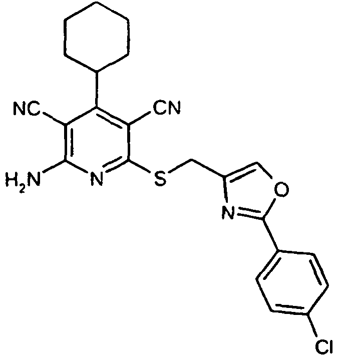 | 2.86 min (22); m/z = 450 | 8.33 (s, 1H), 8.28-7.72 (br. s, 2H), 7.97 (d, 2H), 7.60 (d, 2H), 4.47 (s, 2H), 2.94-2.83 (m, 1 H), 2.01-1.89 (m, 2H), 1.89-1.79 (m, 2H), 1.77-1.66 (m, 3H), 1.38-1.12 (m, 3H). |
| | (68% d. Th.) | | |
| **74** | 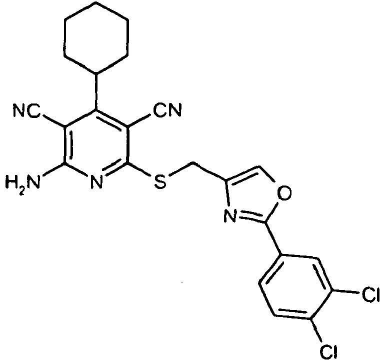 | 3.40 min (4); m/z = 484 | 8.48 (s, 1H), 8.24-7.86 (br. s, 2H), 8.10 (s, 1H), 7.91 (d, 1H), 7.79 (d, 1H), 4.37 (s, 2H), 2.92-2.82 (m, 1H), 2.01-1.87 (m, 2H), 1.87-1.79 (m, 2H), 1.75-1.63 (m, 3H), 1.37-1.13 (m, 3H). |
| | (31 % d. Th.) | | |
| **75** | 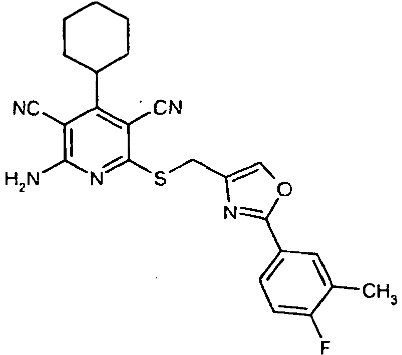 | 3.23 min (4); m/z = 448 | 8.29 (s, 1H), 8.21-7.85 (br. s, 2H), 7.90 (d, 1H), 7.83-7.76 (m, 1H), 7.29 (t, 1H), 4.35 (s, 2H), 2.92-2.82 (m, 1H), 2.30 (s, 3H), 2.00-1.88 (m, 2H), 1.88-1.79 (m, 2H), 1.75-1.65 (m, 3H), 1.37-1.15 (m, 3H). |
| | (20% d. Th.) | | |
| **76** | 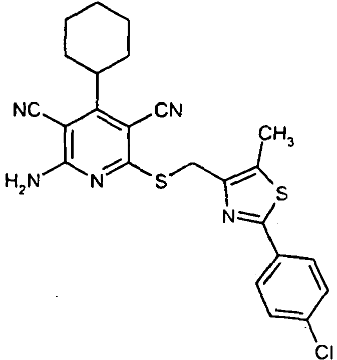 | 3.44 min (4); m/z = 481 | 8.10-7.83 (br. s, 2H), 7.87 (d, 2H), 7.55 (d, 2H), 4.63 (s, 2H), 2.95-2.85 (m, 1H), 2.55 (s, 3H), 2.03-1.90 (m, 2H), 1.89-1.81 (m, 2H), 1.77-1.68 (m, 3H), 1.39-1.13 (m, 3H). |
| | (39% d. Th.) | | |
| **77** | 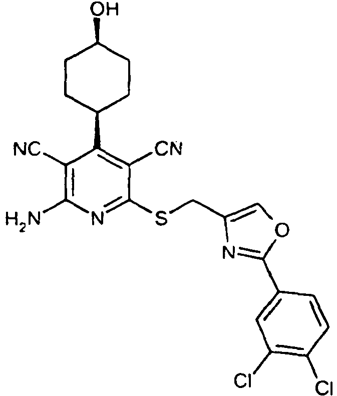 | 2.80 min (4); m/z = 500 | 8.39 (s, 1H), 8.25-7.86 (br. s, 2H), 8.10 (s, 1H), 7.91 (d, 1H), 7.79 (d, 1H), 4.70 (d, 1H), 4.37 (s, 2H), 3.47-3.37 (m, 1H), 2.84-2.73 (m, 1H), 2.07-1.92 (m, 4H), 1.76-1.66 (m, 2H), 1.27-1.15 (m, 2H). |
| | (80% d. Th.) | | |
| **78** | 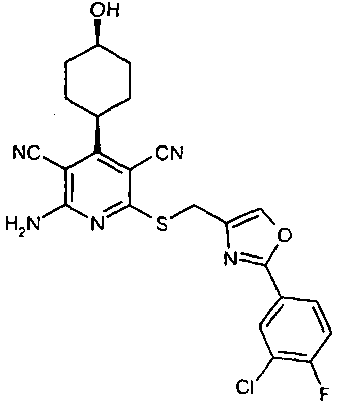 | 2.65 min (4); m/z = 484 | 8.36 (s, 1H), 8.21-7.84 (br. s, 2H), 8.08 (d, 1H), 7.98-7.93 (m, 1H), 7.5 9 (t, 1H), 4.69 (d, 1H), 4.37 (s, 2H), 3.47-3.37 (m, 1H), 2.84-2.75 (m, 1H), 2.07-1.92 (m, 4H), 1.76-1.67 (m, 2H), 1.28-1.15 (m, 2H). |
| | (71 % d. Th.) | | |
| **79** | 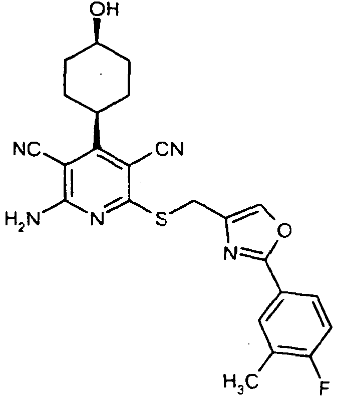 | 2.58 min (4); m/z = 464 | 8.30 (s, 1H), 8.19-7.75 (br. s, 2H), 7.91 (d, 1H), 7.83-7.77 (m, 1H), 7.29 (t, 1 H), 4.70 (d, 1 H), 4.36 (s, 2H), 3.47-3.36 (m, 1H), 2.84-2.75 (m, 1H), 2.31 (s, 3H), 2.08-1.92 (m, 4H), 1.75-1.67 (m, 2H), 1.28-1.15 (m, 2H). |
| | (75% d. Th.) | | |

### Beispiel 80

### rac-2-Amino-4-(tetrahydro-2H-pyran-2-yl)-6-{[3-(1H-tetrazol-5-yl)benzyl)sulfanyl}pyridin-3,5-di-carbonitril

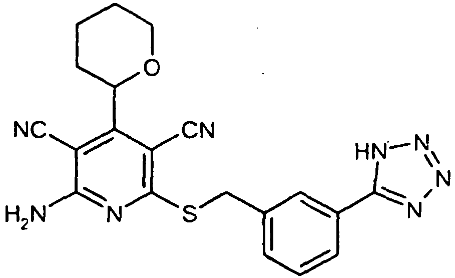

55 mg (0.16 mmol) der Verbindung aus Beispiel 31 A, 65 mg (0.18 mmol) der Verbindung aus Beispiel 50A und 41 mg (0.49 mmol) Natriumhydrogencarbonat werden in 1.7 ml trockenem DMF suspendiert und 10 h bei RT gerührt. Der Ansatz wird danach auf 2 ml Wasser gegossen und durch Zugabe von wenig 1 N Salzsäure auf pH 4 gebracht. Es fällt ein brauner Niederschlag aus, der abfiltriert und mittels präparativer HPLC weiter gereinigt wird (Säule: YMC GEL ODS-AQ S-5, 15 µm; Laufmittelgradient: Acetonitril/Wasser 10:90 → 95:5).
Ausbeute: 5 mg (7% d. Th.)
¹H-NMR (400 MHz, DMSO-d_{b}): δ = 16.96-16.76 (br. s, 1H), 8.18-7.86 (br. s, 2H), 8.14 (s, 1H), 7.92 (d, 1 H), 7.75 (d, 1 H), 7.54 (t, 1H), 4.56 (s, 2H), 4.56-4.49 (m, 1H), 4.01 (d, 1H), 3.58-3.47 (m, 1 H), 1.96-1.87 (m, 1 H), 1.75-1.51 (m, 5H).
LC-MS (Methode 4): Rₜ = 2.50 min; MS (ESIpos): m/z = 419 [M+H]⁺.

### B. Bewertung der pharmakologischen und physiologischen Wirksamkeit

Die pharmakologische und physiologische Wirkung der erfindungsgemäßen Verbindungen kann in folgenden Assays gezeigt werden:

### B-1. Indirekte Bestimmung des Adenosin-Agonismus über Genexpression

Zellen der permanenten Linie CHO (Chinese Hamster Ovary) werden stabil mit der cDNA für die Adenosin-Rezeptor-Subtypen A1, A2a und A2b transfiziert. Die Adenosin-A1-Rezeptoren sind über Gᵢ-Proteine und die Adenosin-A2a- und A2b-Rezeptoren über Gₛ-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration und Medium-Zusammensetzung. Zur pharmakologischen Charakterisierung der Zellen und zum Roboter-gestützten Substanz-Screening wird das folgende Testprotokoll verwendet:

Die Stammkulturen werden in DMEM/F12-Medium mit 10% FCS (fötales Kälberserum) bei 37°C unter 5% CO₂ gezüchtet und jeweils nach 2-3 Tagen 1:10 gesplittet. Testkulturen werden mit 2000 Zellen pro Napf in 384-well-Platten ausgesät und ca. 48 Stunden bei 37°C angezogen. Dann wird das Medium durch eine physiologische Kochsalzlösung (130 mM Natriumchlorid, 5 ml Kaliumchlorid, 2 mM Calciumchlorid, 20 mM HEPES, 1 mM Magnesiumchlorid-Hexahydrat, 5 mM Natriumhydrogencarbonat, pH 7.4) ersetzt. Die in DMSO gelösten zu testenden Substanzen werden in einer Verdünnungsreihe von 5 x 10⁻¹¹ M bis 3 x 10⁻⁶ M (Endkonzentration) zu den Testkulturen pipettiert (maximale DMSO-Endkonzentration im Testansatz: 0.5%). 10 Minuten später wird Forskolin zu den A1-Zellen zugegeben und anschließend werden alle Kulturen für vier Stunden bei 37°C inkubiert. Danach wird zu den Testkulturen 35 µl einer Lösung, bestehend zu 50% aus Lyse-Reagenz (30 mM Dinatriumhydrogenphosphat, 10% Glycerin, 3% TritonX100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) zugegeben, ca. 1 Minute geschüttelt und die Luciferase-Aktivität mit einem Kamerasystem gemessen. Bestimmt werden die EC₅₀-Werte, d.h. die Konzentrationen, bei denen bei der Al-Zelle 50% der Luciferase-Antwort inhibiert bzw. bei den A2b- und A2a-Zellen 50% der maximalen Stimulierbarkeit mit der entsprechenden Substanz erreicht sind. Als Referenzverbindung dient in diesen Experimenten die Adenosin-analoge Verbindung NECA (5-N-Ethylcarboxamido-adenosin), die mit hoher Affinität an alle Adenosin-Rezeptor-Subtypen bindet und eine agonistische Wirkung besitzt [Klotz, K.N., Hessling, J., Hegler, J., Owman, C., Kull, B., Fredholm, B.B., Lohse, M.J., "Comparative pharmacology of human adenosine receptor subtypes - characterization of stably transfected receptors in CHO cells", Naunyn Schmiedebergs Arch. Pharmacol. 357, 1-9 (1998)].

In der folgenden Tabelle 1 sind die EC₅₀-Werte repräsentativer Ausrührungsbeispiele für die Rezeptorstimulation an Adenosin A1-, A2a- und A2b-Rezeptor-Subtypen aufgerührt:

**Tabelle 1**

| **Beispiel Nr.** | **EC₅₀ Al [nM] (1 µM Forskolin)** | **EC₅₀ A2a [nM]** | **EC₅₀ A2b [nM]** |
|---|---|---|---|
| **3** | 0.8 | 1260 | 558 |
| **4** | 2.9 | 1100 | 320 |
| **9** | 8.9 | >3000 | 791 |
| **15** | 10 | 380 | 670 |
| **23** | 0.4 | 620 | 75 |
| **29** | 3.8 | >3000 | 345 |
| **35** | 68 | 1470 | >3000 |
| **51** | 6.8 | >3000 | >3000 |
| **56** | 0.8 | >3000 | 51 |
| **58** | 11 | >3000 | 950 |
| **62** | 0.06 | >3000 | >3000 |
| **65** | 9.5 | >3000 | 860 |
| **70** | 0.08 | 224 | 22 |
| **76** | 106 | >3000 | >3000 |
| **78** | 17 | >3000 | >3000 |

### B-2. Untersuchung an isolierten Gefäßen

Aus narkotisierten Ratten wird die Arteria caudalis präpariert und in eine konventionelle Apparatur zur Messung isolierter Gefäße eingespannt. Die Gefäße werden in einem Wärmebad perfundiert und mit Phenylephrin kontrahiert. Das Maß der Kontraktion wird über einen Kontraktionsmesser ermittelt. Zu den vorkontrahierten Gefäßen werden Testsubstanzen gegeben und die Abnahme der Kontraktion der Gefäße gemessen. Eine Abnahme der Kontraktion entspricht einer Dilatation der Gefäße. Als EC₅₀-Wert einer Testsubstanz bzgl. ihrer relaxierenden Eigenschaften wird die Konzentration angegeben, bei der die Kontraktion der Gefäße um 50% verringert ist.

### B-3 Blutdruck- und Herzfrequenz-Messungen an wachen Ratten

Wachen SHR (spontaneously hypertensive rats)-Ratten, die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral verabreicht. Anschließend werden über 24 Stunden Blutdruck und Herzfrequenz und deren Veränderungen aufgezeichnet.

### B-4. Blutdruck- und Herzfrequenz-Messungen an wachen Krallenaffen

Wachen Krallenaffen, die einen internen Sender tragen, der dauerhaft sowohl Blutdruck als auch Herzfrequenz messen kann (telemetrische Erfassung von hämodynamischen Parametern), werden Testsubstanzen in verschiedenen Konzentrationen oral verabreicht. Anschließend werden über 6-24 Stunden Blutdruck und Herzfrequenz und deren Veränderungen aufgezeichnet.

### B-5. Bestimmung der Löslichkeit

### Benötigte Reagenzien:

- PBS-Puffer pH 7.4: 90.00 g NaCl p.a. (z.B. Fa. Merck, Art.-Nr. 1.06404.1000), 13.61 g KH₂PO₄ p.a. (z.B. Fa. Merck, Art.-Nr. 1.04873.1000) und 83.35 g 1 N NaOH (z.B. Fa. Bernd Kraft GmbH, Art.-Nr. 01030.4000) in einen 1 Liter-Messkolben einwiegen, mit Wasser auffüllen und ca. 1 Stunde rühren;
- Acetatpuffer pH 4.6: 5.4 g Natriumacetat x 3 H₂O p.a. (z.B. Fa. Merck, Art.-Nr. 1.06267.0500) in einen 100 ml-Messkolben einwiegen, in 50 ml Wasser lösen, mit 2.4 g Eisessig versetzen, auf 100 ml mit Wasser auffüllen, pH-Wert überprüfen und falls notwendig auf pH 4.6 einstellen;
- Dimethylsulfoxid (z.B. Fa. Baker, Art.-Nr. 7157.2500);
- destilliertes Wasser.

### Herstellung der Kalibrierlösungen:

*Herstellung der Ausgangslösung für Kalibrierlösungen (Stammlösung):* In ein 2 ml Eppendorf-Safe-Lock Tube (Fa. Eppendorf, Art.-Nr. 0030 120.094) werden ca. 0.5 mg der Testsubstanz genau eingewogen, zu einer Konzentration von 600 µg/ml mit DMSO versetzt (z.B. 0.5 mg Substanz + 833 µl DMSO) und bis zur vollständigen Lösung mittels eines Vortexers geschüttelt.

*Kalibrierlösung 1 (20 µg*/*ml):* 34.4 µl der Stammlösung werden mit 1000 µl DMSO versetzt und homogenisiert.

*Kalibrierlösung 2 (2.5 µg*/*ml):* 100 µl der Kalibrierlösung 1 werden mit 700 µl DMSO versetzt und homogenisiert.

### Herstellung der Probenlösungen:

*Probenlösung für Löslichkeit bis 10 g*/*l in PBS-Puffer pH 7.4:* In ein 2 ml Eppendorf-Safe-Lock Tube (Fa. Eppendorf, Art.-Nr. 0030 120.094) werden ca. 5 mg der Testsubstanz genau eingewogen und zu einer Konzentration von 5 g/l mit PBS-Puffer pH 7.4 versetzt (z.B. 5 mg Substanz + 500 µl PBS-Puffer pH 7.4).

*Probenlösung für Löslichkeit bis 10 g*/*l in Acetatpuffer pH 4.6:* In ein 2 ml Eppendorf-Safe-Lock Tube (Fa. Eppendorf, Art.-Nr. 0030 120.094) werden ca. 5 mg der Testsubstanz genau eingewogen und zu einer Konzentration von 5 g/l mit Acetatpuffer pH 4.6 versetzt (z.B. 5 mg Substanz + 500 µl Acetatpuffer pH 4.6).

*Probenlösung für Löslichkeit bis 10 g*/*l in Wasser:* In ein 2 ml Eppendorf-Safe-Lock Tube (Fa. Eppendorf, Art.-Nr. 0030 120.094) werden ca. 5 mg der Testsubstanz genau eingewogen und zu einer Konzentration von 5 g/l mit Wasser versetzt (z.B. 5 mg Substanz + 500 µl Wasser).

### Durchführung:

Die so hergestellten Probenlösungen werden 24 Stunden bei 1400 rpm mittels eines temperierbaren Schüttlers (z.B. Fa. Eppendorf Thermomixer comfort Art.-Nr. 5355 000.411 mit Wechselblock Art.-Nr. 5362.000.019) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und 1:5, 1:100 und 1:1000 mit dem jeweils verwendeten Lösungsmittel (Wasser, PBS-Puffer 7.4 oder Acetatpuffer pH 4.6) verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/l ausgedrückt. Analysensequenz: 1) Kalibrierlösung 2.5 mg/ml; 2) Kalibrierlösung 20 µg/ml; 3) Probenlösung 1:5; 4) Probenlösung 1:100; 5) Probenlösung 1:1000.

### HPLC-Methode für Säuren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C18, 50 mm x 2 mm, 5 p; Temperatur: 40°C; Eluent A: Wasser/Phosphorsäure pH 2; Eluent B: Acetonitril; Flussrate: 0.7 ml/min; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311 A), Autosampler CTC HTS PAL, Degaser (G1322A) und Säulenthermostat (G1316A); Säule: VDSoptilab Kromasil 100 C18, 60 mm x 2.1 mm, 3.5 µ; Temperatur: 30°C; Eluent A: Wasser + 5 ml Perchlorsäure/l; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

### B-6. Bestimmung pharmakokinetischer Kenngrößen nach intravenöser und oraler Gabe

Die zu untersuchende Substanz wird Tieren (z.B. Maus, Ratte, Hund) intravenös als Lösung appliziert, die orale Applikation erfolgt als Lösung oder Suspension über eine Schlundsonde. Nach Substanzgabe wird den Tieren zu festgelegten Zeitpunkten Blut entnommen. Dieses wird heparinisiert, anschließend wird daraus durch Zentrifugation Plasma gewonnen. Die Substanz wird im Plasma über LC/MS-MS analytisch quantifiziert. Aus den so ermittelten Plasmakonzentration-Zeit-Verläufen werden die pharmakokinetischen Kenngrößen wie AUC, Cₘₐₓ, T_{1/2} (Halbwertszeit) und CL (Clearance) mittels eines validierten pharmakokinetischen Rechenprogramms berechnet.

### C. Ausführunpsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösuns:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 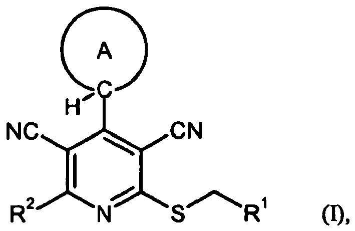 in welcher
der Ring A für Cyclopentyl, Cyclohexyl, Cyclopent-2-en-1-yl, Cyclopent-3-en-1-yl, Cyclohex-2-en-1-yl oder Cyclohex-3-en-1-yl, für einen 5- oder 6-gliedrigen, C-verknüpften, gesättigten Heterocyclus, der ein Ringglied aus der Reihe N-R³ oder O enthält, oder für einen C-verknüpften, gesättigten Heterocyclus der Formel 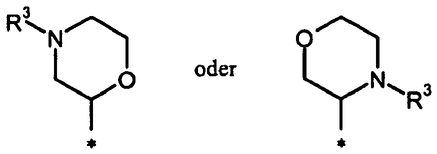 steht, worin
Cyclopentyl, Cyclohexyl, Cyclopentenyl sowie Cyclohexenyl ein- oder zweifach, gleich oder verschieden, mit einem Rest ausgewählt aus der Reihe (C₁-C₄)-Alkyl, Hydroxy, (C₁-C₄)-Alkoxy, Amino, Mono-(C₁-C₄)-alkylamino und Di-(C₁-C₄)-alkyl-amino substituiert sein können, wobei die genannten (C₁-C₄)-Alkyl- und (C₁-C₄)-Alkoxy-Reste ihrerseits ein- oder zweifach, gleich oder verschieden, mit Hydroxy, (C₁-C₄)-Alkoxy und/oder (C₃-C₅)-Cycloalkyl substituiert sein können,
* die Verknüpfungsstelle mit dem Pyridin-Ring bedeutet
und
R³ Wasserstoff, (C₁-C₄)-Alkyl, welches ein- oder zweifach, gleich oder verschieden, mit Hydroxy, (C₁-C₄)-Alkoxy, (C₁-C₄)-Acyloxy und/oder (C₃-C₅)-Cycloalkyl substituiert sein kann, oder (C₁-C₄)-Acyl, welches mit Hydroxy oder (C₁-C₄)-Alkoxy substituiert sein kann, bedeutet,
R¹ für Phenyl oder 5- oder 6-gliedriges Heteroaryl mit bis zu drei Ring-Heteroatomen aus der Reihe N, O und/oder S steht, welche jeweils
(*i*) ein- oder zweifach, gleich oder verschieden, mit einem Rest ausgewählt aus der Reihe Fluor, Chlor, Cyano, (C₁-C₄)-Alkyl, Amino, Mono-(C₁-C₄)-alkylamino, Di-(C₁-C₄)-alkylaminno, (C₁-C₄)-Alkoxycarbonyl, Carboxyl und Carbamoyl
und/oder
(*ii*) mit Morpholino, *N'*-(C₁-C₄)-Alkylpiperazino oder einer Gruppe der Formel -L-R⁴, worin
L eine Bindung oder NH bedeutet
und
R⁴ Phenyl oder 5- oder 6-gliedriges Heteroaryl mit bis zu drei Ring-Heteroatomen aus der Reihe N, O und/oder S bedeutet, welche jeweils ein- bis dreifach, gleich oder verschieden, mit einem Rest ausgewählt aus der Reihe Fluor, Chlor, Cyano, (C₁-C₄)-Alkyl, Trifluormethyl, (C₁-C₄)-Alkoxy, Trifluormethoxy, (C₁-C₄)-Alkoxycarbonyl und Carboxyl substituiert sein können,
substituiert sind,
oder
R¹ für *N*-Oxidopyridyl steht,
und
R² für Wasserstoff oder für (C₁-C₄)-Alkoxy, das bis zu dreifach mit Fluor substituiert sein kann, steht
oder
R² für eine Gruppe der Formel -NR⁵R⁶ steht, worin
R⁵ Wasserstoff oder (C₁-C₄)-Alkyl, das mit Hydroxy, (C₁-C₄)-Alkoxy, Amino, Mono-(C₁-C₄)-alkylamino, Di-(C₁-C₄)-alkylamino, Carboxyl, (C₁-C₄)-Alkoxycarbonyl oder einem 5- oder 6-gliedrigen gesättigten Heterocyclus substituiert sein kann, bedeutet,
wobei der genannte Heterocyclus ein oder zwei Ring-Heteroatome aus der Reihe N und/oder O enthält und seinerseits ein- oder zweifach, gleich oder verschieden, mit Methyl, Ethyl, Hydroxy, Methoxy und/oder Ethoxy substituiert sein kann,
R⁶ Wasserstoff oder Methyl bedeutet
oder
R⁵ und R⁶ zusammen mit dem Stickstoffatom, an das sie gebunden sind, einen 5- oder 6-gliedrigen gesättigten Heterocyclus bilden, der ein weiteres Ring-Heteroatom aus der Reihe N oder O enthalten und ein- oder zweifach, gleich oder verschieden, mit Methyl, Ethyl, Hydroxy, Methoxy und/oder Ethoxy substituiert sein kann,
sowie ihre Salze, Solvate und Solvate der Salze.

2. Verbindung der Formel (I) nach Anspruch 1 in welcher
der Ring A für eine Gruppe der Formel 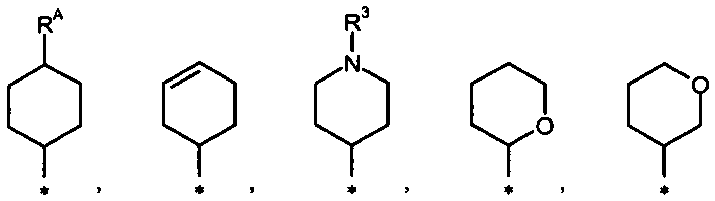 oder 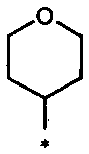 steht, worin
* die Verknüpfungsstelle mit dem Pyridin-Ring, R^{A} Wasserstoff, Hydroxy, Methoxy, Ethoxy oder 2-Hydroxyethoxy
und
R³ Methyl, Ethyl, 2-Hydroxyethyl, 2-Acetoxyethyl, 3-Hydroxypropyl, 3-Acetoxypropyl oder Hydroxyacetyl
bedeuten,
R¹ für Phenyl, Oxazolyl, Thiazolyl oder Pyridyl steht, welche jeweils
(i) ein- oder zweifach, gleich oder verschieden, mit einem Rest ausgewählt aus der Reihe Fluor, Chlor, Methyl, Amino, Methoxycarbonyl, Ethoxycarbonyl, Carboxyl und Carbamoyl
oder
(*ii*) mit einer Gruppe der Formel -L-R⁴, worin
L eine Bindung oder NH bedeutet
und
R⁴ Phenyl oder Pyridyl bedeutet, welche jeweils ein- oder zweifach, gleich oder verschieden, mit einem Rest ausgewählt aus der Reihe Fluor, Chlor, Cyano, Methyl, Methoxy und Carboxyl substituiert sein können,
substituiert sind,
und
R² für Wasserstoff, Methoxy oder eine Gruppe der Formel -NR⁵R⁶ steht, worin
R⁵ Wasserstoff oder (C₁-C₄)-Alkyl, das mit Hydroxy, Amino, Methylamino, Ethylamino, Dimethylamino, Diethylamino oder einem Heterocyclus der Formel 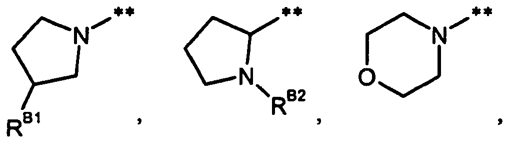 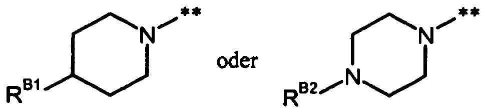 substituiert sein kann, bedeutet,
R⁶ Wasserstoff bedeutet
oder
R⁵ und R⁶ zusammen mit dem Stickstoffatom, an das sie gebunden sind, eine Gruppe der Formel 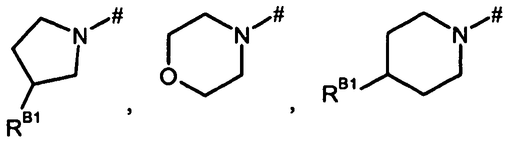 oder 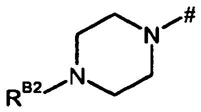 bilden, worin jeweils
** die Verknüpfungsstelle mit dem (C₁-C₄)-Alkylrest,
# die Verknüpfungsstelle mit dem Pyridin-Ring, R^{B1} Wasserstoff oder Hydroxy
und
R^{B2} Wasserstoff oder Methyl
bedeuten,
sowie ihre Salze, Solvate und Solvate der Salze.

3. Verfahren zur Herstellung von Verbindungen der Formel (I), wie in den Ansprüchen 1 bis 2 definiert, in welcher R² für NH₂ steht, **dadurch gekennzeichnet, dass** man eine Verbindung der Formel (II) 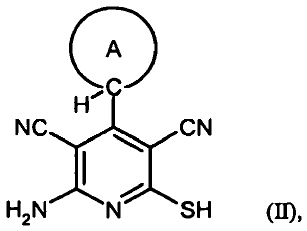 in welcher der Ring A die in den Ansprüchen 1 bis 2 angegebene Bedeutung hat,
in einem inerten Lösungsmittel in Gegenwart einer Base mit einer Verbindung der Formel (III) 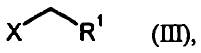 in welcher R' die in den Ansprüchen 1 bis 2 angegebene Bedeutung hat und
X für eine geeignete Abgangsgruppe wie Halogen, Mesylat, Tosylat oder Triflat steht,
zu einer Verbindung der Formel (I-A) 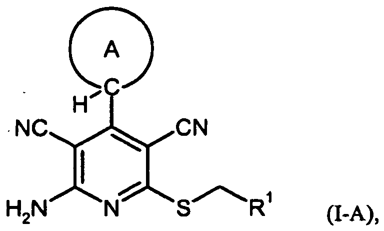 in welcher R¹ und der Ring A die oben angegebenen Bedeutungen haben,
umsetzt
und die Verbindungen der Formel (I-A) gegebenenfalls mit den entsprechenden (i) Lösungsmitteln und/oder (ii) Basen oder Säuren in ihre Solvate, Salze und/oder Solvate der Salze überführt.

4. Verfahren zur Herstellung von Verbindungen der Formel (I), wie in den Ansprüchen 1 bis 2 definiert, in welcher R² für die Gruppe -NR⁵R⁶ steht, worin mindestens einer der beiden Reste R⁵ und R⁶ nicht Wasserstoff bedeutet, **dadurch gekennzeichnet, dass** man Verbindungen der Formel (I-A) 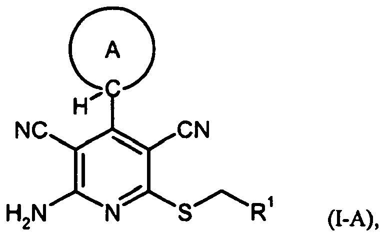 in welcher R¹ und der Ring A die in den Ansprüchen 1 bis 2 angegebenen Bedeutungen haben,
zunächst mit Kupfer(II)chlorid und Isoamylnitrit in einem geeigneten Lösungsmittel in Verbindungen der Formel (VI) 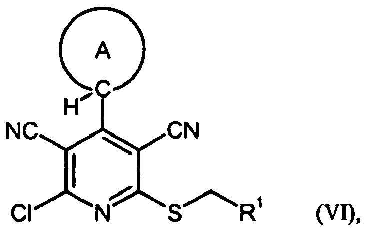 in welcher R¹ und der Ring A die oben angegebenen Bedeutungen haben,
überführt und diese anschließend mit einer Verbindung der Formel (VII) 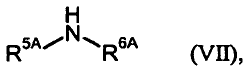 in welcher
R^{5A} die in den Ansprüchen 1 bis 2 angegebene Bedeutung von R⁵ hat,
R^{6A} die in den Ansprüchen 1 bis 2 angegebene Bedeutung von R⁶ hat,
jedoch mindestens einer der beiden Reste R^{5A} und R^{6A} nicht für Wasserstoff steht,
zu Verbindungen der Formel (I-B) 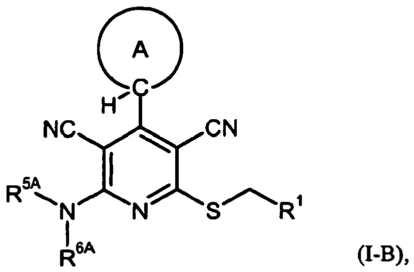 in welcher R¹, R^{5A}, R^{6A} und der Ring A jeweils die oben angegebenen Bedeutungen haben,
umsetzt
und die Verbindungen der Formel (I-B) gegebenenfalls mit den entsprechenden (*i*) Lösungsmitteln und/oder (*ii*) Basen oder Säuren in ihre Solvate, Salze und/oder Solvate der Salze überführt.

5. Verbindung der Formel (I), wie in einem der Ansprüche 1 bis 2 definiert, zur Behandlung und/oder Prophylaxe von Krankheiten.

6. Verwendung einer Verbindung der Formel (I), wie in einem der Ansprüche 1 bis 2 definiert, zur Herstellung eines Arzneimittels zur Behandlung und/oder Prophylaxe von Hypertonie, koronarer Herzerkrankung, akutem Koronarsyndrom, Angina pectoris, Herzinsuffizienz, Myokardinfarkt und Vorhofflimmern.

7. Verwendung einer Verbindung der Formel (I), wie in einem der Ansprüche 1 bis 2 definiert, zur Herstellung eines Arzneimittels zur Behandlung und/oder Prophylaxe von Diabetes, Metabolischem Syndrom und Dyslipidämien.

8. Arzneimittel enthaltend eine Verbindung der Formel (I), wie in einem der Ansprüche 1 bis 2 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 2 definiert, in Kombination mit einem oder mehreren weiteren Wirkstoffen ausgewählt aus der Gruppe bestehend aus den Fettstoffwechsel verändernden Wirkstoffen, Antidiabetika, blutdrucksenkenden Wirkstoffen und antithrombotisch wirkenden Mitteln.

10. Arzneimittel nach Anspruch 8 oder 9 zur Verwendung in der Behandlung und/oder Prophylaxe von Hypertonie, koronarer Herzerkrankung, akutem Koronarsyndrom, Angina pectoris, Herzinsuffizienz, Myokardinfarkt und Vorhofflimmem.

11. Arzneimittel nach Anspruch 8 oder 9 zur Verwendung in der Behandlung und/oder Prophylaxe von Diabetes, Metabolischem Syndrom und Dyslipidämien.

## Claims

1. Compound of the formula (I) 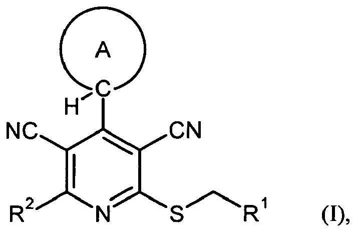 in which
ring A represents cyclopentyl, cyclohexyl, cyclopent-2-en-1-yl, cyclopent-3-en-1-yl, cyclohex-2-en-1-yl or cyclohex-3-en-1-yl, represents a 5- or 6-membered saturated heterocycle which is attached via carbon und which contains a ring member from the group consisting of N-R³ and O, or represents a saturated heterocycle of the formula 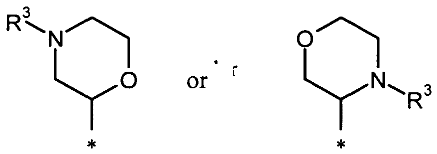 which is attached via carbon and in which
cyclopentyl, cyclohexyl, cyclopentenyl and cyclohexenyl may be mono- or disubstituted by identical or different radicals selected from the group consisting of (C₁-C₄)-alkyl, hydroxyl, (C₁-C₄)-alkoxy, amino, mono-(C₁-C₄)-alkylamino and di-(C₁-C₄)-alkylamino, where the (C₁-C₄)-alkyl- and (C₁-C₄)-alkoxy radicals mentioned for their part may be mono- or disubstituted by identical or different radicals from the group consisting of hydroxyl, (C₁-C₄)-alkoxy and (C₃-C₅)-cycloalkyl,
* denotes the point of attachment to the pyridine ring
and
R³ represents hydrogen, (C₁-C₄)-alkyl which may be mono- or disubstituted by identical or different radicals from the group consisting of hydroxyl, (C₁-C₄)-alkoxy, (C₁-C₄)-acyloxy and (C₃-C₅)-cycloalkyl, or (C₁-C₄)-acyl which may be substituted by hydroxyl or (C₁-C₄)-alkoxy,
R¹ represents phenyl or 5- or 6-membered heteroaryl having up to three ring heteroatoms from the group consisting of N, O and S, each of which radicals is
(*i*) mono- or disubstituted by identical or different radicals selected from the group consisting of fluorine, chlorine, cyano, (C₁-C₄)-alkyl, amino, mono-(C₁-C₄)-alkylamino, di-(C₁-C₄)-alkylamino, (C₁-C₄)-alkoxycarbonyl, carboxyl and carbamoyl
and/or
(*ii*) substituted by morpholino, *N'*-(C₁-C₄)-alkylpiperazino or a group of the formula -L-R⁴ in which
L represents a bond or NH
and
R⁴ represents phenyl or 5- or 6-membered heteroaryl having up to three ring heteroatoms from the group consisting of N, O and S, each of which radicals may be mono- to trisubstituted by identical of different radicals selected from the group consisting of fluorine, chlorine, cyano, (C₁-C₄)-alkyl, trifluoromethyl, (C₁-C₄)-alkoxy, trifluoromethoxy, (C₁-C₄)-alkoxycarbonyl and carboxyl,
or
R¹ represents N-oxidopyridyl,
and
R² represents hydrogen or represents (C₁-C₄)-alkoxy which may be substituted up to three times by fluorine
or
R² represents a group of the formula -NR⁵R⁶ in which
R⁵ represents hydrogen or (C₁-C₄)-alkyl which may be substituted by hydroxyl, (C₁-C₄)-alkoxy, amino, mono-(C₁-C₄)-alkylamino, di-(C₁-C₄)-alkylamino, carboxyl, (C₁-C₄)-alkoxycarbonyl or a 5- or 6-membered saturated heterocycle,
where the heterocycle mentioned contains one or two ring heteroatoms from the group consisting of N and O and for its part may be mono- or disubstituted by identical or different radicals from the group consisting of methyl, ethyl, hydroxyl, methoxy and ethoxy,
R⁶ represents hydrogen or methyl
or
R⁵ and R⁶ together with the nitrogen atom, to which they are attached form a 5- or 6-membered saturated heterocycle which may contain a further ring heteroatom from the group consisting of N or O and may be mono- or disubstituted by identical or different radicals from the group consisting of methyl, ethyl, hydroxyl, methoxy and ethoxy,
and its salts, solvates and solvates of the salts.

2. Compound of the formula (I) according to Claim I in which
ring A represents a group of the formula 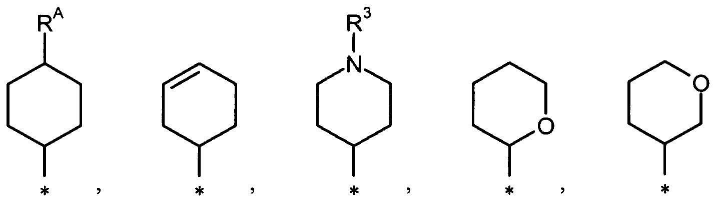 or 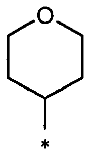 in which
* denotes the point of attachment to the pyridine ring,
R^{A} represents hydrogen, hydroxyl, methoxy, ethoxy or 2-hydroxyethoxy
and
R³ represents methyl, ethyl, 2-hydroxyethyl, 2-acetoxyethyl, 3-hydroxypropyl, 3-acetoxypropyl or hydroxyacetyl,
R¹ represents phenyl, oxazolyl, thiazolyl or pyridyl, each of which radicals is
(i) mono- or disubstituted by identical or different radicals selected from the group consisting of fluorine, chlorine, methyl, amino, methoxycarbonyl, ethoxycarbonyl, carboxyl and carbamoyl
or
(*ii*) substituted by a group of the formula -L-R⁴ in which
L represents a bond or NH
and
R⁴ represents phenyl or pyridyl, each of which radicals may be mono- or disubstituted by identical or different radicals selected from the group consisting of fluorine, chlorine, cyano, methyl, methoxy and carboxyl,
and
R² represents hydrogen, methoxy or a group of the formula -NR⁵R⁶ in which
R⁵ represents hydrogen or (C₁-C₄)-alkyl which may be substituted by hydroxyl, amino, methylamino, ethylamino, dimethylamino, diethylamino or a heterocycle of the formula 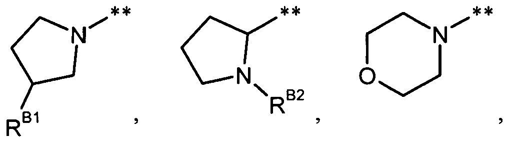 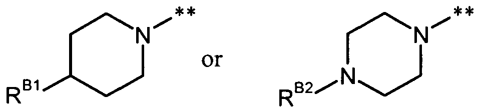
R⁶ represents hydrogen
or
R⁵ and R⁶ together with the nitrogen atom to which they are attached form a group of the formula 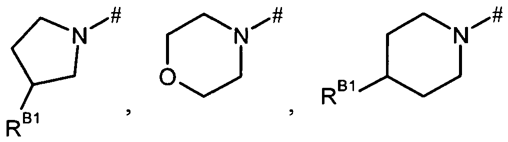 or 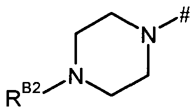 in which in each case
** denotes the point of attachment to the (C₁-C₄)-alkyl radical,
# denotes the point of attachment to the pyridine ring,
R^{B1} represents hydrogen or hydroxyl
and
R^{B2} represents hydrogen or methyl,
and its salts, solvates and solvates of the salts.

3. Process for preparing compounds of the formula (I) as defined in Claims 1 and 2 in which R² represents NH₂, **characterized in that** a compound of the formula (II) 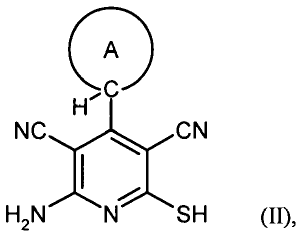 in which ring A has the meaning given in Claims 1 and 2,
is reacted in an inert solvent in the presence of a base with a compound of the formula (III) 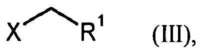 in which R¹ has the meaning given in Claims 1 and 2 and
X represents a suitable leaving group, such as halogen, mesylate, tosylate or triflate,
to give a compound of the formula (I-A) 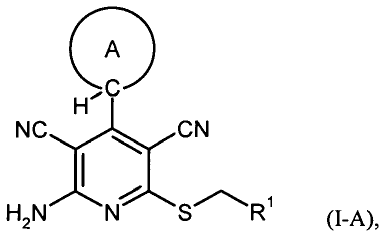 in which R¹ and ring A have the meanings given above,
and the compounds of the formula (I-A) are, if appropriate, converted with the appropriate (*i*) solvents and/or (*ii*) bases or acids into their solvates, salts and/or solvates of the salts.

4. Process for preparing compounds of the formula (I) as defined in Claims 1 and 2 in which R² represents the group -NR⁵R⁶ and in which at least one of the two radicals R⁵ and R⁶ is not hydrogen, **characterized in that** compounds of the formula (I-A) 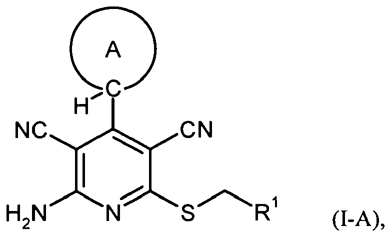 in which R¹ and ring A have the meanings given in Claims 1 and 2,
are initially converted with copper(II) chloride and isoamyl nitrite in a suitable solvent into compounds of the formula (VI) 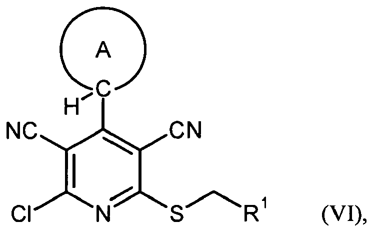 in which R¹ and the ring A have the meanings given above,
and these are then reacted with a compound of the formula (VII) 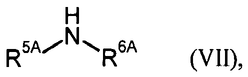 in which
R^{5A} has the meaning of R⁵ given in Claims 1 and 2,
R^{6A} has the meaning of R⁶ given in Claims 1 and 2,
but at least one of the two radicals R^{5A} and R^{6A} does not represent hydrogen, to give compounds of the formula (I-B) 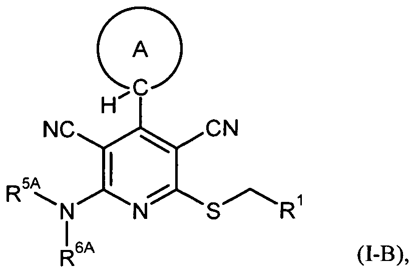 in which R¹, R^{5A}, R^{6A} and ring A each have the meanings given above,
and the compounds of the formula (I-B) are, if appropriate, converted with the appropriate (i) solvents and/or (*ii*) bases or acids into their solvates, salts and/or solvates of the salts.

5. Compound of the formula (I) as defined in either of Claims 1 and 2 for the treatment and/or prophylaxis of diseases.

6. Use of a compound of the formula (I) as defined in either of Claims 1 and 2 for preparing a medicament for the treatment and/or prophylaxis of hypertension, coronary heart disease, acute coronary syndrome, angina pectoris, heart failure, myocardial infarction and atrial fibrillation.

7. Use of a compound of the formula (I) as defined in either of Claims 1 and 2 for preparing a medicament for the treatment and/or prophylaxis of diabetes, metabolic syndrome and dyslipidemias.

8. Medicament comprising a compound of the formula (I) as defined in either of Claims 1 and 2 in combination with an inert nontoxic pharmaceutically suitable auxiliary.

9. Medicament comprising a compound of the formula (I) as defined in either of Claims 1 and 2 in combination with one or more further active compounds selected from the group consisting of lipid metabolism-modifying active compounds, antidiabetics, antihypertensive drugs and antithrombotic drugs.

10. Medicament according to Claim 8 or 9 for use in the treatment and/or prophylaxis of hypertension, coronary heart disease, acute coronary syndrome, angina pectoris, heart failure, myocardial infarction and atrial fibrillation.

11. Medicament according to Claim 8 or 9 for use in the treatment and/or prophylaxis of diabetes, metabolic syndrome and dyslipidemias.

## Revendications

1. Composé de formule (I) 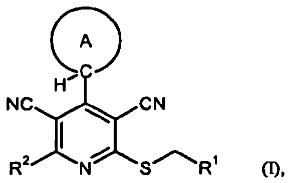 dans laquelle
le cycle A représente cyclopentyle, cyclohexyle, cyclopent-2-én-1-yle, cyclopent-3-én-1-yle, cyclohex-2-én-1-yle ou cyclohex-3-én-1-yle, un hétérocycle de 5 ou 6 chaînons, lié par C, saturé, qui contient un élément de cycle de la série N-R³ ou O, ou hétérocycle lié par C, saturé de formule 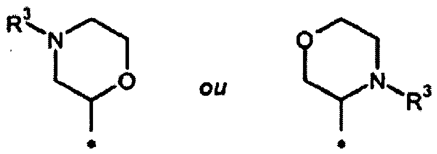 où
cyclopentyle, cyclohexyle, cyclopentényle ainsi que cyclohexényle peuvent être monosubstitués ou disubstitués, de manière identique ou différente, par un radical choisi dans la série (C₁-C₄)-alkyle, hydroxy, (C₁-C₄) -alcoxy, amino, mono-(C₁-C₄)-alkylamino et di-(C₁-C₄)-alkylamino, les radicaux (C₁-C₄)-alkyle et (C₁-C₄)-alcoxy mentionnés pouvant à leur tour être monosubstitués ou polysubstitués,
de manière identique ou différente, par hydroxy, (C₁-C₄) -alcoxy et/ou (C₃-C₅)-cycloalkyle,
* signifie le site de liaison avec le cycle pyridine et
R³ signifie hydrogène, (C₁-C₄)-alkyle, qui peut être monosubstitué ou disubstitué, de manière identique ou différente, par hydroxy, (C₁-C₄)-alcoxy, (C₁-C₄) -acyloxy et/ou (C₃-C₅)-cycloalkyle, ou (C₁-C₄)-acyle, qui peut être substitué par hydroxy ou (C₁-C₄)-alcoxy,
R¹ représente phényle ou hétéroaryle de 5 ou 6 chaînons, comprenant jusqu'à trois hétéroatomes de cycle de la série N, O et/ou S, qui sont à chaque fois
(i) monosubstitués ou disubstitués, de manière identique ou différente, par un radical choisi dans la série fluor, chlore, cyano, (C₁-C₄)-alkyle, amino, mono-(C₁-C₄)-alkylamino, di-(C₁-C₄)-alkylamino, (C₁-C₄)-alcoxycarbonyle, carboxyle et carbamoyle et/ou
(ii) substitués par morpholino, N'-(C₁-C₄)-alkylpipérazino ou un groupe de formule -L-R⁴, dans laquelle
L signifie une liaison ou NH et
R⁴ signifie phényle ou hétéroaryle de 5 ou 6 chaînons comprenant jusqu'à trois hétéroatomes de cycle de la série N, O et/ou S, qui peuvent être à chaque fois monosubstitués à trisubstitués, de manière identique ou différente par un radical choisi dans la série fluor, chlore, cyano, (C₁-C₄)-alkyle, trifluorométhyle, (C₁-C₄)-alcoxy, trifluorométhoxy, (C₁-C₄)-alcoxycarbonyle et carboxyle,
ou
R¹ représente N-oxydopyridyle, et
R² représente hydrogène ou (C₁-C₄)-alcoxy, qui peut être jusqu'à trisubstitué par fluor, ou
R² représente un groupe de formule -NR⁵R⁶, où
R⁵ signifie hydrogène ou (C₁-C₄)-alkyle, qui peut être substitué par hydroxy, (C₁-C₄)-alcoxy, amino, mono-(C₁-C₄)-alkylamino, di-(C₁-C₄)-alkylamino, carboxyle, (C₁-C₄)-alcoxycarbonyle ou un hétérocycle de 5 ou 6 chaînons, saturé, l'hétérocycle mentionné contenant un ou deux hétéroatomes de cycle de la série N et/ou O et pouvant à son tour être monosubstitué ou disubstitué, de manière identique ou différente, par méthyle, éthyle, hydroxy, méthoxy et/ou éthoxy,
R⁶ signifie hydrogène ou méthyle ou
R⁵ et R⁶ forment, ensemble avec l'atome d'azote auquel ils sont liés, un hétérocycle de 5 ou 6 chaînons, saturé, qui peut contenir un autre hétéroatome de cycle de la série N ou O et qui peut être monosubstitué ou
disubstitué, de manière identique ou différente, par méthyle, éthyle, hydroxy, méthoxy et/ou éthoxy,
ainsi que ses sels, solvates et les solvates des sels.

2. Composé de formule (I) selon la revendication 1, dans lequel le cycle A représente un groupe de formule 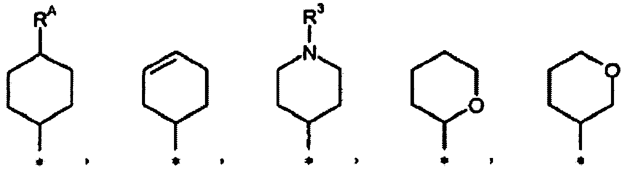 ou 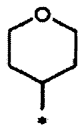 où
* signifie le site de liaison avec le cycle pyridine,
R^{A} signifie hydrogène, hydroxy, méthoxy, éthoxy ou 2-hydroxyéthoxy et
R³ signifie méthyle, éthyle, 2-hydroxyéthyle, 2-acétoxyéthyle, 3-hydroxypropyle, 3-acétoxypropyle ou hydroxyacétyle
R¹ représente phényle, oxazolyle, thiazolyle ou pyridyle, qui sont à chaque fois
(i) monosubstitués ou disubstitués, de manière identique ou différente, par un radical choisi dans la série fluor, chlore, méthyle, amino, méthoxycarbonyle, éthoxycarbonyle, carboxyle et carbamoyle ou
(ii) substitués par un groupe de formule -L-R⁴, où L signifie une liaison ou NH et
R⁴ signifie phényle ou pyridyle, qui peuvent être à chaque fois monosubstitués ou disubstitués, de manière identique ou différente par un radical choisi dans la série fluor, chlore, cyano, méthyle, méthoxy et carboxyle,
et
R² représente hydrogène, méthoxy ou un groupe de formule -NR⁵R⁶, dans laquelle
R⁵ signifie hydrogène ou (C₁-C₄)-alkyle, qui peut être substitué par hydroxy, amino, méthylamino, éthylamino, diméthylamino, diéthylamino ou un hétérocycle de formule 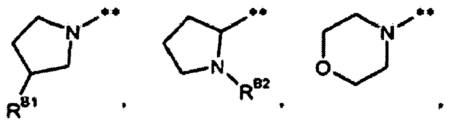 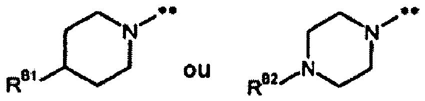 R⁶ signifie hydrogène, ou
R⁵ et R⁶ forment ensemble avec l'atome d'azote auquel ils sont liés un groupe de formule 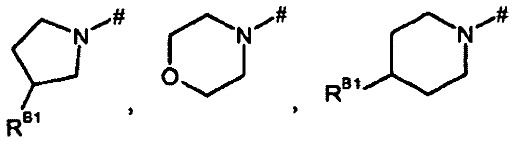 ou 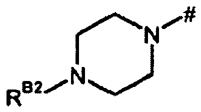 où, à chaque fois
** représente le site de liaison avec le radical (C₁-C₄)-alkyle,
# signifie le site de liaison avec le cycle pyridine,
R^{B1} signifie hydrogène ou hydroxy et
R^{B2} signifie hydrogène ou méthyle
ainsi que ses sels, solvates et les solvates des sels.

3. Procédé pour la préparation de composés de formule (I), tels que définis dans les revendications 1 à 2, où R² représente NH₂, **caractérisé en ce qu'**on transforme un composé de formule (II) 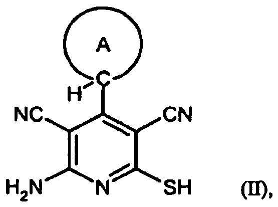 dans laquelle le cycle A présente la signification indiquée dans les revendications 1 à 2,
dans un solvant inerte en présence d'une base avec un composé de formule (III) 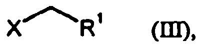 dans laquelle R¹ présente la signification indiquée dans les revendications 1 à 2 et
X représente un groupe partant approprié tel qu'halogène, mésylate, tosylate ou triflate,
en un composé de formule (I-A) 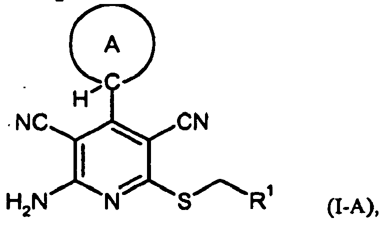 dans laquelle R¹ et le cycle A présentent les significations indiquées ci-dessus,
et on transforme les composés de formule (I-A) le cas échéant avec (i) les solvants et/ou (ii) les bases ou acides correspondants en leurs solvates, sels et/ou solvates des sels.

4. Procédé pour la préparation de composés de formule (I), tels que définis dans les revendications 1 à 2, où R² représente le groupe -NR⁵R⁶, dans laquelle au moins un des deux radicaux R⁵ et R⁶ ne signifie pas hydrogène, **caractérisé en ce qu'**on transforme des composés de formule (I-A) 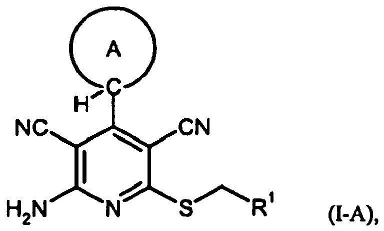 dans laquelle R¹ et le cycle A présentent les significations indiquées dans les revendications 1 à 2, d'abord avec du chlorure de cuivre (II) et du nitrite d'isoamyle dans un solvant approprié en composés de formule (VI) 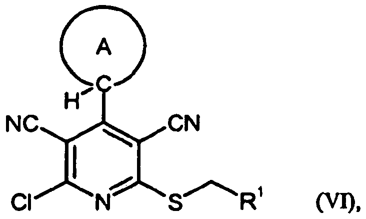 dans laquelle R¹ et le cycle A présentent les significations indiquées ci-dessus,
et on transforme ceux-ci ensuite avec un composé de formule (VII) 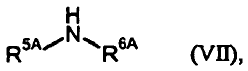 dans laquelle
R^{5A} présente la signification de R⁵ indiquée dans les revendications 1 à 2 et
R^{6A} présente la signification de R⁶ indiquée dans les revendications 1 à 2 et
cependant au moins un des radicaux R^{5A} et R^{6A} ne représente pas hydrogène, en composés de formule (I-B) 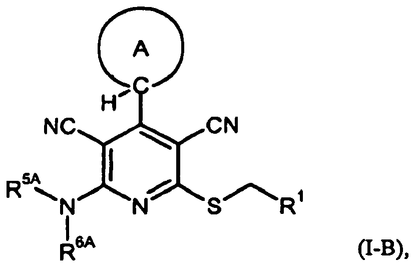 dans laquelle R¹, R^{5A}, R^{6A} et le cycle A présentent à chaque fois les significations indiquées ci-dessus,
et on transforme les composés de formule (I-B) le cas échéant avec (i) les solvants et/ou (ii) les bases ou acides correspondants en leurs solvates, sels et/ou solvates des sels.

5. Composé de formule (I), tel que défini dans l'une quelconque des revendications 1 à 2, destiné au traitement et/ou à la prophylaxie de maladies.

6. Utilisation d'un composé de formule (I), tel que défini dans l'une quelconque des revendications 1 à 2, pour la préparation d'un médicament destiné au traitement et/ou à la prophylaxie de l'hypertonie, de maladies coronariennes, du syndrome coronarien aigu, de l'angine de poitrine, de l'insuffisance cardiaque, de l'infarctus du myocarde et de la fibrillation auriculaire.

7. Utilisation d'un composé de formule (I), tel que défini dans l'une quelconque des revendications 1 à 2, pour la préparation d'un médicament destiné au traitement et/ou à la prophylaxie du diabète, du syndrome métabolique et des dyslipidémies.

8. Médicament contenant un composé de formule (I), tel que défini dans l'une quelconque des revendications 1 à 2, en combinaison avec un adjuvant inerte, non toxique, pharmaceutiquement approprié.

9. Médicament, contenant un composé de formule (I), tel que défini dans l'une quelconque des revendications 1 à 2, en combinaison avec une ou plusieurs autres substances actives choisies dans le groupe constitué par les substances actives modifiant le métabolisme des graisses, les antidiabétiques, les substances actives abaissant la tension artérielle et les agents à action antithrombotique.

10. Médicament selon la revendication 8 ou 9 destiné à une utilisation dans le traitement et/ou la prophylaxie de l'hypertonie, de maladies coronariennes, du syndrome coronarien aigu, de l'angine de poitrine, de l'insuffisance cardiaque, de l'infarctus du myocarde et de la fibrillation auriculaire.

11. Médicament selon la revendication 8 ou 9 destiné à une utilisation dans le traitement et/ou la prophylaxie du diabète, du syndrome métabolique et des dyslipidémies.
